# EUROPÄISCHE PATENTSCHRIFT

(11) **EP 2 456 782 B1**
(45) Veröffentlichungstag und Bekanntmachung des Hinweises auf die Patenterteilung: **19.08.2015**
(21) Anmeldenummer: 10731477.5
(22) Anmeldetag: 07.07.2010
(51) Int. Cl.: C07J 7/00

(54) **17-HYDROXY-17-PENTAFLUORETHYL-ESTRA-4,9(10)-DIEN-11-BENZYLIDEN-DERIVATE, VERFAHREN ZU DEREN HERSTELLUNG UND DEREN VERWENDUNG ZUR BEHANDLUNG VON KRANKHEITEN**
17-HYDROXY-17-PENTAFLUORETHYL-ESTRA-4,9(10)-DIEN-11-BENZYLIDENE DERIVATIVES, METHODS FOR THE PRODUCTION THEREOF AND USE THEREOF FOR TREATING DISEASES
DÉRIVÉS 17-HYDROXY-17-PENTAFLUOROÉTHYL-ESTRA-4,9(10)-DIÈNE-11-BENZYLIDÈNE, PROCÉDÉS DE PRÉPARATION ET UTILISATION POUR LE TRAITEMENT DE MALADIES

(30) Priorität: 20.07.2009 DE 102009034367
(43) Veröffentlichungstag der Anmeldung: 30.05.2012
(73) Patentinhaber: Bayer Intellectual Property GmbH, 40789 Monheim (DE)
(72) Erfinder: KLAR, Ulrich, 13503 Berlin (DE); SCHWEDE, Wolfgang, 16548 Glienicke (DE); MÖLLER, Carsten, 10115 Berlin (DE); ROTGERI, Andrea, 13503 Berlin (DE); BONE, Wilhelm, 13467 Berlin (DE)
(74) Vertreter: BIP Patents
(86) Internationale Anmeldenummer: PCT/EP2010/004150
(87) Internationale Veröffentlichungsnummer: WO 2011/009532

(56) Entgegenhaltungen:
- WO-A1-03/093292

## Beschreibung

Die Erfindung betrifft den in den Ansprüchen beschriebenen Gegenstand, d.h. neue 17-Hydroxy-13-methyl-17-pentafluorethyl-11-phenyl-dodecahydrocyclopenta[a]phenanthren-3-on-Derivate mit Progesteron antagonisierender Wirkung, Verfahren zu deren Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten.

Verbindungen mit antagonistischer Wirkung am Progesteronrezeptor (kompetitive Progesteronrezeptorantagonisten) sind erstmals 1982 bekannt geworden (RU 486; EP 0 057 115) und seither zahlreich beschrieben worden.

Progesteronrezeptorantagonisten mit fluorierter 17α-Seitenkette wurden von U. Fuhrmann et al. beschrieben und sind im J. Med. Chem. 43, 5010 - 5016 (2000) bzw. in der WO 98/34947 veroffentlicht. Eine besonders aktive Verbindung, die dort offenbart wird, ist das 11-ß-(4-Acetylphenyl)-20,20,21,21,21,-pentafluor-17-hydroxy-nor-17α-pregna-4,9,-dien-3-on. Diese Substanz zeigt jedoch im Vergleich zu den strukturell ähnlichen Verbindungen der vorliegenden Erfindung eine relative kurze invivo Halbwertszeit und darüber hinaus eine schlechte Wasserlöslichkeit. WO 03/093292 betrifft 12-Hydroxy-13-methyl-17-pentafluorethyl-1-phenyl-dodecahydrocyclopenta[a]phenanthren-3-on Derivate zur Behandlung von postmenopausalen gynäkologischen Erkrankungen.

Aufgabe der vorliegenden Erfindung ist es, hoch potente kompetitive Progesteronrezeptorantagonisten zur Verfügung zu stellen und damit alternative Behandlungsmöglichkeiten gynäkologischer Erkrankungen zu schaffen.

Es wurde gefunden, dass die erfindungsgemäßen Verbindungen der allgemeinen Formel (I) besonders geeignet sind, um diese Aufgabe zu lösen.

Die vorliegende Erfindung betrifft 17-Hydroxy-17-pentafluorethyl-estra-4,9(10)-dien-11-benzyliden-Derivate der allgemeinen Formel (I) 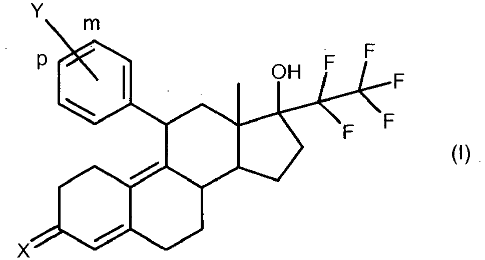 worin
- Y: für eine -CR¹=CHR² oder eine -CR¹=NR³ Gruppe steht, die jeweils in der meta (m) oder para (p) Position am Phenylring angeknüpft ist,
worin
- R¹: Wasserstoff oder ein C₁-C₁₀-Alkylrest,
- R²: für Wasserstoff, einen C₁-C₁₀-Alkyl, C₂-C₁₀-Alkenyl, Aryl, C₇-C₂₀-Aralkyl, C₁-C₁₀-Acyl, CO₂R⁴, CN, CH=CH-CO₂R⁴, CH₂CH(CO₂R⁴)₂, CH₂CH(CN)₂ oder einen CH₂NHCONHR⁵ Rest steht und
- R³: für einen C₁-C₁₀-Alkyl, O-Alkyl, O-(CH₂)ₙ-COOR⁴, O-CH₂-Aryl, Aryl, C₇-C₂₀-Aralkyl oder einen CH₂CO₂R⁴ -Rest steht, worin
- R⁴: für Wasserstoff oder eine C₁-C₁₀-Alkylgruppe und
- R⁵: für eine C₁-C₁₀-Alkyl-, C₂-C₁₀-Alkenyl-, Aryl-, C₇-C₂₀-Aralkyl -, (CH₂)ₙCO₂R⁴ - Gruppe mit n = 1 oder 2 und R⁴ in der angegebenen Bedeutung oder für eine 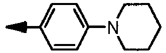 Gruppe steht und
- X: Sauerstoff, eine NOR⁶ oder eine NNHSO₂R⁶ Gruppe bedeutet,
worin
- R⁶: für Wasserstoff, einen C₁-C₁₀-Alkyl, Aryl oder C₇-C₂₀-Aralkyl-Rest steht.

Sofern R⁵ für 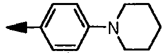 steht, kennzeichnet der Pfeil die Stelle an der die Bindung an die Aminogruppe des benachbarten Restes -CH₂NHCONHR⁵ erfolgt.

Gemäß einer bevorzugten Ausführungsform der Erfindung steht X für Sauerstoff.

Erfindungsgemäß bevorzugt sind weiterhin die Verbindungen der Formel (I) bei denen der Substituent (Rest) Y in para Position an den Phenylring gebunden ist.

Im Rahmen der vorliegenden Erfindung haben die Substituenten, soweit nicht anders spezifiziert, die folgende Bedeutung:

Unter Alkyl in R¹, R², R³, R⁴, R⁵ und R⁶ sowie in anderen Fällen sind gerad- oder verzweigtkettige Alkylgruppen mit der angegebenen Anzahl an Kohlenstoffatomen oder gegebenenfalls 1-10 Kohlenstoffatomen zu verstehen, wie beispielsweise Methyl, Ethyl, Propyl, Isopropyl, Butyl, Isobutyl, tert.-Butyl, Pentyl, Isopentyl, Neopentyl, Hexyl, Heptyl, Octyl, Nonyl oder Decyl. Die Alkylgruppen R¹, R², R³, R⁴, R⁵ und R⁶ können ferner perfluoriert oder durch 1-5 Halogenatome, Hydroxygruppen, C₁-C₄-Alkoxygruppen, C₆-C₁₂-Arylgruppen (die wiederum durch 1-3 Halogenatome substituiert sein können) substituiert sein. Insbesondere kann Alkyl daher auch für Hydroxymethylen (HO-CH₂), Hydroxyethylen (HO-C₂H₄), Hydroxypropylen (HO-C₃H₆) und Hydroxybutylen (HO-C₄H₈) sowie deren Isomere stehen.

Unter Alkenyl in R⁴ und R⁵ sind gerad- oder verzweigtkettige Alkenylgruppen mit 2-10 Kohlenstoffatomen zu verstehen, wie beispielsweise Vinyl, Propenyl, Butenyl, Pentenyl, Isobutenyl oder Isopentenyl.

Die Alkenylgruppen R⁴ und R⁵ können durch 1-5 Halogenatome, Hydroxygruppen, C₁-C₃-Alkoxygruppen oder C₆-C₁₂-Arylgruppen (die wiederum durch 1-3 Halogenatome substituiert sein können) substituiert sein.

Unter Aryl in R², R³, R⁵ und R⁶ sowie in anderen Fällen sind substituierte und unsubstituierte carbocyclische Reste mit einem oder mehreren Heteroatomen zu verstehen, wie zum Beispiel Phenyl, oder Naphthyl, die einfach oder mehrfach mit Halogen, OH, O-Alkyl, S-Alkyl, S(O)Alkyl und SO₂Alkyl, CO₂H, CO₂-Alkyl, NH₂, NO₂, N₃, CN, C₁-C₁₀-Alkyl, C₁-C₁₀-Acyl, C₁-C₁₀-Acyloxy-Gruppen, substituiert sein können. Soweit ansonsten Aryl als Substituent an Alkyl, Alkenyl oder Alkinyl erwähnt wird, handelt es sich insbesondere um Arylgruppen mit 6-12 Ringkohlenstoffatomen.

Unter Aralkyl in R², R³, R⁵ und R⁶ sind Aralkylgruppen zu verstehen, die im Ring bis zu 14 Kohlenstoffatome, bevorzugt 6 bis 10 C-Atome, und in der Alkylkette 1 bis 8, bevorzugt 1 bis 4, Kohlenstoffatome enthalten können. Als Aralkylreste kommen beispielsweise Benzyl, Phenylethyl, Naphthylmethyl, Naphthylethyl, Furylmethyl, Thienylethyl oder Pyridylpropyl in Betracht. Die Ringe können einfach oder mehrfach durch Halogen, OH, O-Alkyl, CO₂H, CO₂-Alkyl NO₂, N₃, CN, C₁-C₂₀-Alkyl, C₁-C₂₀-Acyl, C₁-C₂₀-Acyloxy-Gruppen substituiert sein.

Als Heteroatome kommen Schwefel, Sauerstoff oder Stickstoff in Frage, wobei Stickstoff bevorzugt ist. Als Beispiel sei der Pyridylpropyl-Rest genannt.

Soweit Alkoxy (O-Alkyl) erwähnt wird, handelt es sich um Alkoxygruppen mit 1-4 Kohlenstoffatomen. Alkoxy kann insbesondere Methoxy, Ethoxy und Propoxy sein.

Soweit Acyl (CO-Alkyl) erwähnt wird, handelt es sich um Acylgruppen mit 1-20 Kohlenstoffatomen. Acyl kann insbesondere Formyl, Acetyl, Propionyl und Butyryl sein.

Soweit Acyloxy (O-CO-Alkyl) erwähnt wird, handelt es sich um Acyloxygruppen mit 1-20 Kohlenstoffatomen. Acyloxy kann insbesondere Formyloxy, Acetyloxy, Propionyloxy und Butyryloxy sein.

Halogen bedeutet Fluor, Chlor oder Brom. Unter diesen sind Fluor und Chlor bevorzugt.

Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb auch die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.

Jeder der genannten Substituenten am Steroid-Grundgerüst kann sowohl in einer α- als auch in einer β-Stellung vorliegen. Außerdem können auch die Substituenten am Steroid-Grundgerüst, die eine Doppelbindung enthalten und in denen die Doppelbindung an jedem Atom mindestens einen Substituenten, der nicht Wasserstoff ist, trägt, sowohl E- als auch Z-konfiguriert vorliegen..

Sofern die erfindungsgemäßen Verbindungen in tautomeren Formen vorkommen können, umfasst die vorliegenden Erfindung sämtliche tautomere Formen.

Die erfindungsgemäßen Verbindungen der allgemeinen Formel I können auch als Solvate, Hydrate und Salze vorliegen, wobei auch unterschiedliche Kristallmodifikationen, sowie α-, β- oder y-Cyclodextrinclathrate oder mit Liposomen verkapselten Verbindungen der Formel I umfasst sind.

Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Liegen die Derivate der Formel I als Solvat vor, kann das enthaltene Solvens in einem stöchiometrischen oder auch nicht-stöchiometrischen Verhältnis vorliegen. Bei stöchiometrischen Solvaten, spricht man auch von Hemi-, (Semi-), Mono-, Sesqui-, Di-, Tri-, Tetra-, Penta-, usw. Solvaten.

Hydrate sind eine spezielle Form der Solvate, bei denen die Koordination mit Wasser erfolgt.

Unter den Sovaten bevorzugt sind die Hydrate, d.h. Koordinationsverbindungen mit Wasser.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure oder Benzoesäure.

Physiologisch unbedenkliche Salze der erfindungsgemäßen Verbindungen umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methyl-aminomethan oder 1-Amino-2,3,4-butantriol.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Es wurde gefunden, dass die erfindungsgemäßen Verbindungen der Formel (I) überraschenderweise eine gute, das Progesteron antagonisierende Wirkung, aufweisen, ohne die Nachteile der Verbindungen des Standes der Technik zu haben.

So zeigen die meisten erfindungsgemäßen Verbindungen, eine überraschend hohe metabolische Stabilität in vitro, in Lebermikrosomen von Ratte und Mensch. Gleichzeitig ist es gelungen, die Wasserlöslichkeit für einige Verbindungen deutlich zu verbessern (*siehe Beispiel 60*)

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden.

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/ Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84-tägige Gabe des Progesteronrezeptorantagonisten gefolgt von der 14-tägigen Gabe des Gestagens.

Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika.

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

Zur Behandlung von mit der Menopause assoziierten Beschwerden kommt eine gleichzeitige oder sequentielle Verabreichung der erfindungsemäßen Verbindungen z.B. mit SERMs, SERDs und Östrogenen in Betracht.

SERMs (Selective Estrogen Receptor Modulators) sind erfindungsgemäß solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen.

Selektive Estrogenrezeptordestabilisatoren (SERD) sind Arzneistoffe, die den Estrogenrezeptor vollständig antagonisieren und zu einem Abbau des Rezeptors führen.

Antiöstrogene sind Verbindungen die den Estrogenrezeptor vollständig antagonisieren, beispielsweise Fulvestrant.

Aromataseinhibitoren inhibieren das Enzym Aromatase und somit die Aromatisierung von Aridrogenen in Estrogene. Dazu gehören u.a. Anastrozole, Letrozole, Exemestane, Vorozole, Formestane and Fadrozole. Kinaseinhibitoren hemmen Enzyme, die einen Phosphatrest von ATP auf andere Substrate, dort insbesondere auf Hydroxygruppen, übertragen, z.B. Sorafenib (Nexavar) oder Imatinib (Gleevec).

Angiogenesehemmer, z.B. Avastin, reduzieren bzw. blockieren die Gefäßneubildung und damit die Durchblutung eines Tumors.

Zytostatika, z.B. cis-Platin, Taxol, Taxotere, Sagopilon, Ixabepilon sind natürliche oder synthetische Substanzen, die Tumorzellen in die Apoptose treiben.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die erfindungsgemäßen Verbindungen können systemisch und/oder lokal wirken. Zu diesem Zweck können sie auf geeignete Weise, wie z.B. oral, intrauterinär, intravaginal, parenteral, pulmonal, nasal, sublingual, lingual, buccal, rectal, dermal, transdermal, conjunctival, otisch oder als Implantat bzw. Stent appliziert werden.

Intrauterinär bedeutet dabei insbesondere die Applikation mittels IUS (intrauterine system) oder IUD (intrauterine device). Die intravaginale Applikation kann u.a. mittels IVR (Vaginalring) erfolgen.

Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nichtsilikon- und/oder Silikonpolymere, insbesondere auch siloxanebasierte Elastomere (vergl. WO 01/47490, insbesondere Seite 7, Zeile 19 - Seite 15, Zeile 15), enthalten.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektiöns- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die Wirksamkeit der erfindungsgemäßen Verbindungen als Progesteronrezeptorantagonist wurde in vitro in Transaktivierungstests und in vivo an der Ratte (Terminierung der frühen Schwangerschaft) gezeigt.

Erfindungsgemäß bevorzugt sind die nachfolgenden Verbindungen, deren Synthese in den Beispielen beschrieben ist:
➢ (8S,11R,13S,14S,17S)-17-Hydroxy-13-methyl-17-pentafluorethyl-11-(4-vinylphenyl)-1,2,6,7,8,11,12,13,14,15,16,17-dodecahydrocyclopenta[a]phenanthren-3-on (Beispiel 1)
➢ (8S,11R,13S,14S,17S)-17-Hydroxy-11-[4-((E)-3-hydroxy-propenyl)-phenyl]-13-methyl-17-pentafluorethyl-1,2,6,7,8,11,12,13,14,15,16,17-dodecahydrocyclopenta[*a*]phenanthren-3-on (Beispiel 2)
➢ (8S,11R,13S,14S,17S)-17-Hydroxy-11-{4-[(E)-3-(4-methoxy-phenyl)-propenyl]phenyl}-13-methyl-17-pentafluorethyl-1,2,6,7,8,11,12,13,14,15,16,17-dodecahydro-cyclopenta[*a*]phenanthren-3-on (Beispiel 3)
➢ 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-malonsäuredimethylester (Beispiel 4)
➢ 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-malonsäure (Beispiel 5)
➢ (8S,11R,13S,14S,17S)-17-Hydroxy-13-methyl-17-pentafluorethyl-11-[4-((E)-2-pyridin-2-yl-vinyl)-phenyl]-1,2,6,7,8,11,12,13,14,15,16,17-dodecahydrocyclopenta[a]phenanthren-3-on (Beispiel 6)
➢ (8S,11R,13S,14S,17S)-17-Hydroxy-13-methyl-17-pentafluorethyl-11-[4-((E)-2-pyridin-3-yl-vinyl)-phenyl]-1,2,6,7,8,11,12,13,14,15,16,17-dodecahydrocyclopenta[*a*]phenanthren-3-on (Beispiel 7)
➢ (8S,11R,13S,14S,17S)-17-Hydroxy-13-methyl-17-pentafluorethyl-11-[4-((E)-2-pyridin-4-yl-vinyl)-phenyl]-1,2,6,7,8,11,12,13,14,15,16,17-dodecahydrocyclopenta[*a*]phenanthren-3-on (Beispiel 8)
➢ (8S,11R,13S,14S,17S)-17-Hydroxy-13-methyl-11-{4-[(E)-2-(2-methyl-thiazol-4-yl)-vinyl]-phenyl}-17-pentafluorethyl-1,2,6,7,8,11,12,13,14,15,16,17-dodecahydro-cyclopenta[*a*]phenanthren-3-on (Beispiel 9)
➢ (8S,11R,13S,14S,17S)-17-Hydroxy-13-methyl-11-{4-[(E)-2-(2-methyl-benzothiazol-5-yl)-vinyl]-phenyl}-17-pentafluorethyl-1,2,6,7,8,11,12,13,14,15,16,17-dodecahydro-cyclopenta[*a*]phenanthren-3-on (Beispiel 10)
➢ (8S,11R,13S,14S,17S)-17-Hydroxy-11-(4-isopropenyl-phenyl)-13-methyl-17-pentafluorethyl-1,2,6,7,8,11,12,13,14,15,16,17-dodecahydrocyclopenta[*a*]phenanthren-3-on (Beispiel 11)
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylsäureethylester (Beispiel 12)
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylsäure (Beispiel 13)
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylnitril (Beispiel 14)
➢ (2E,4E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-penta-2,4-diensäureethylester (Beispiel 15)
➢ (8S,11R,13S,14S,17S)-17-Hydroxy-13-methyl-11-[4-((E)-3-oxo-but-1-enyl)-phenyl]-17-pentafluorethyl-1,2,6,7,8,11,12,13,14,15,16,17-dodecahydrocyclopenta[*a*]phenanthren-3-on (Beispiel 16)
➢ (2E,4E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-penta-2,4-diensäure (Beispiel 17)
➢ (8S,11R,13S,14S,17S)-11-[((E/Z)-4-Buta-1,3-dienyl)-phenyl]-17-hydroxy-13-methyl-17-pentafluorethyl-1,2,6,7,8,11,12,13,14,15,16,17-dodecahydrocyclopenta[*a*]phenanthren-3-on (Beispiel 18)
➢ (8S,11R,13S,14S,17S)-17-Hydroxy-13-methyl-17-pentafluorethyl-11-(3-vinyl-phenyl)-1,2,6,7,8,11,12,13,14,15,16,17-dodecahydrocyclopenta[a]phenanthren-3-on (Beispiel 19)
➢ (8S,11R,13S,14S,17S)-17-Hydroxy-11-(3-isopropenyl-phenyl)-13-methyl-17-pentafluorethyl-1,2,6,7,8,11,12,13,14,15,16,17-dodecahydrocyclopenta[*a*]phenanthren-3-on (Beispiel 20)
➢ (8S,11R,13S,14S,17S)-17-Hydroxy-13-methyl-17-pentafluorethyl-11-[3-((E)-2-pyridin-2-yl-vinyl)-phenyl]-1,2,6,7,8,11,12,13,14,15,16,17-dodecahydrocyclopenta[*a*]phenanthren-3-on (Beispiel 21)
➢ (8S,11R,13S,14S,17S)-17-Hydroxy-13-methyl-17-pentafluorethyl-11-[3-((E)-2-pyridin-3-yl-vinyl)-phenyl]-1,2,6,7,8,11,12,13,14,15,16,17-dodecahydrocyclopenta[*a*]phenanthren-3-on (Beispiel 22)
➢ (8S,11R,13S,14S,17S)-17-Hydroxy-13-methyl-17-pentafluorethyl-11-[3-((E)-2-pyridin-4-yl-vinyl)-phenyl]-1,2,6,7,8,11,12,13,14,15,16,17-dodecahydrocyclopenta[*a*]phenanthren-3-on (Beispiel 23)
➢ (8S,11R,13S,14S,17S)-17-Hydroxy-13-methyl-11-{3-[(E)-2-(2-methyl-thiazol-4-yl)-vinyl]-phenyl}-17-pentafluorethyl-1,2,6,7,8,11,12,13,14,15,16,17-dodecahydro-cyclopenta[*a*]phenanthren-3-on (Beispiel 24)
➢ (8S,11R,13S,14S,17S)-17-Hydroxy-13-methyl-11-{3-[(E)-2-(2-methyl-benzothiazol-5-yl)-vinyl]-phenyl}-17-pentafluorethyl-1,2,6,7,8,11,12,13_{;}14,15,16,17-dodecahydro-cyclopenta[*a*]phenanthren-3-on (Beispiel 25)
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylsäureethylester (Beispiel 26)
➢ 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-malonitril (Beispiel 27)
➢ 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-vinyl}-benzoesäure (Beispiel 28)
➢ 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure (Beispiel 29)
➢ 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[*a*]phenanthren-11-yl)-phenyl]-vinyl}-benzoesäuremethylester (Beispiel 30)
➢ 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-vinyl}-benzoesäuremethylester (Beispiel 31)
➢ (8S,11R,13S,14S,17S)-17-Hydroxy-11-{4-[(E)-2-(4-methansulfonyl-phenyl)-vinyl]-phenyl}-13-methyl-17-pentafluorethyl-1,2,6,7,8,11,12,13,14,15,16,17-dodecahydro-cyclopenta[*a*]phenanthren-3-on (Beispiel 32)
➢ (8S,11R,13S,14S,17S)-17-Hydroxy-13-methyl-11-{4-[(E)-2-(4-methylsulfanyl-phenyl)-vinyl]-phenyl}-17-pentafluorethyl-1,2,6,7,8,11,12,13,14,15,16,17-dodecahydro-cyclopenta[*a*]phenanthren-3-on (Beispiel 33)
➢ [1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-meth-(E)-ylidenaminooxy]-essigsäure (Beispiel 34)
➢ 4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzaldehyd *O*-benzyl-oxim (Beispiel 35)
➢ 4-((8S,11 R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzaldehyd O-ethyl-oxim (Beispiel 36)
➢ 4-((8S,11 R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzaldehyd *O*-(3,4-dichloro-benzyl)-oxim (Beispiel 37)
➢ 4-((8S,11 R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzaldehyd O-isobutyl-oxim (Beispiel 38)
➢ 1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 39)
➢ 1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzyl]-3-isopropyl-harnstoff (Beispiel 40)
➢ 1-*tert*-Butyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 41)
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-3-phenyl-harnstoff (Beispiel 42)
➢ 1-(4-Cyano-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 43)
➢ 1-(4-Fluor-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 44)
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-3-p-tolyl-harnstoff (Beispiel 45)
➢ 1-Benzyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 46)
➢ 1-tert-Butyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 47)
➢ 1-(3,5-Dimethyl-isoxazol-4-yl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-allyl}-harnstoff (Beispiel 48)
➢ 3-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-propionsäureethylester (Beispiel 49)
➢ 3-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-propionsäure (Beispiel 50)
➢ (3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-essigsäureethylester (Beispiel 51)
➢ (3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-essigsäure (Beispiel 52)
➢ 1-(4-Chlor-phenyl)-'3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 53)
➢ 1-{(E)-3-[4-((8S,11R,13S,14S,17S)-17-Hydroxy-13-methyl-3-oxo-17-pentatluorethyl-2,3,6,7,8,11,12,13,14,15,16,17-dodecahydro-1*H-*cyclopenta[a]phenanthren-11-yl)-phenyl]-allyl}-3-(4-methoxy-phenyl)-harnstoff (Beispiel 54)
➢ 4-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-benzoesäureethylester (Beispiel 55)
➢ 4-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-benzoesäure (Beispiel 56)
➢ 1-Allyl-3-{(E)-3-[4-((8S,11,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff (Beispiel 57)
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-3-(4-piperidin-1-yl-phenyl)-harnstoff (Beispiel 58)

Die Erfindung betrifft weiterhin ein Verfahren zu Herstellung der erfindungsgemäßen Derivate der Formel I. Derartige Derivate lassen sich wie in Schema 1 dargestellt, herstellen, indem man ein Epoxid der allgemeinen Formel II, worin
- X': ein Sauerstoffatom, zwei Alkoxygruppen OR⁷, eine C₂-C₁₀-Alkylen-α,ω-dioxygruppe, die geradkettig oder verzweigt sein kann,
- R⁷: C₁-C₄-Alkyl,
darstellen durch metallorganische Kupplungsreaktionen, vorzugsweise durch Kupfer katalysierte Grignardreaktionen, in eine Verbindung der allgemeinen Formel III, worin und X' die oben genannten Bedeutungen haben und
- R⁸: Wasserstoff,
- R⁹: eine Hydroxylgruppe, oder
- R⁸, R⁹: gemeinsam eine Bindung
darstellen, umsetzt und gegebenenfalls in R¹ vorhandene Funktionalitäten umwandelt und/oder weitere Folgereaktionen zu Verbindungen der allgemeinen Formel I' durchführt und gebenenfalls die Gruppe X in der Bedeutung von Sauerstoff aus der Gruppe X' freisetzt und/oder eine Doppelbindung (R⁸, R⁹ gemeinsam eine Bindung) durch Eliminierung von Wasser (R⁸ = Wasserstoff, R⁹ = Hydroxylgruppe) erzeugt und gegebenenfalls die Carbonylgruppe (X = Sauerstoff) weiter funktionalisiert (X = NOR⁶ oder eine NNHSO₂R⁶ Gruppe).

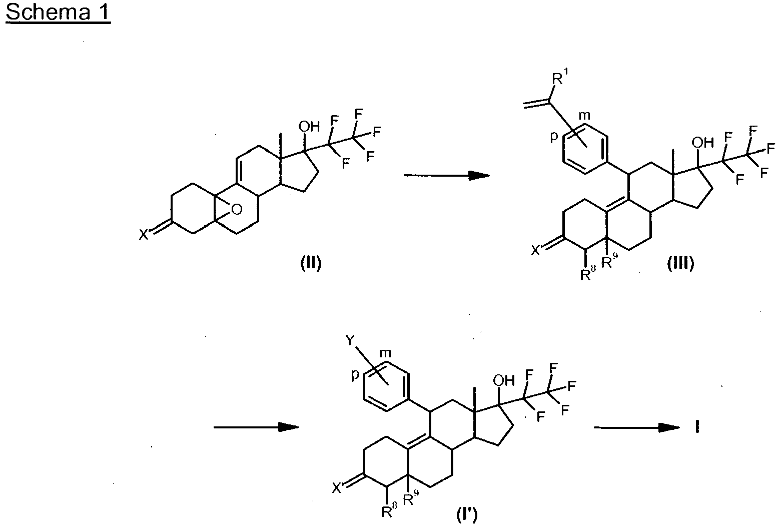

Typische Ausführungen für die Herstellung von Verbindungen der allgemeinen Formel III finden sich in den Beispielen 1 a, 11 a, 19a und 20a.

Stellen R⁸ und R⁹ gemeinsam eine Bindung in der Struktur der allgemeinen Formel I' dar, so steht diese stellvertretend für die drei möglichen Doppelbindungsisomeren I'-A, I'-B und I'-C, die während der beschriebenen Umsetzungen in unterschiedlichsten Verhältnissen zueinander entstehen können.

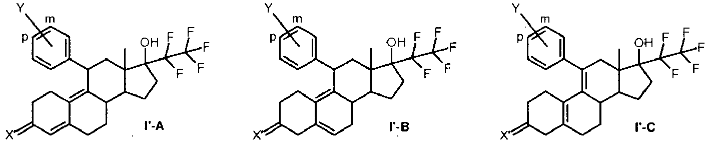

Einige mögliche Umsetzungssequenzen zu Verbindungen der allgemeinen Formel I' sind exemplarisch in Schema 2 näher ausgeführt.

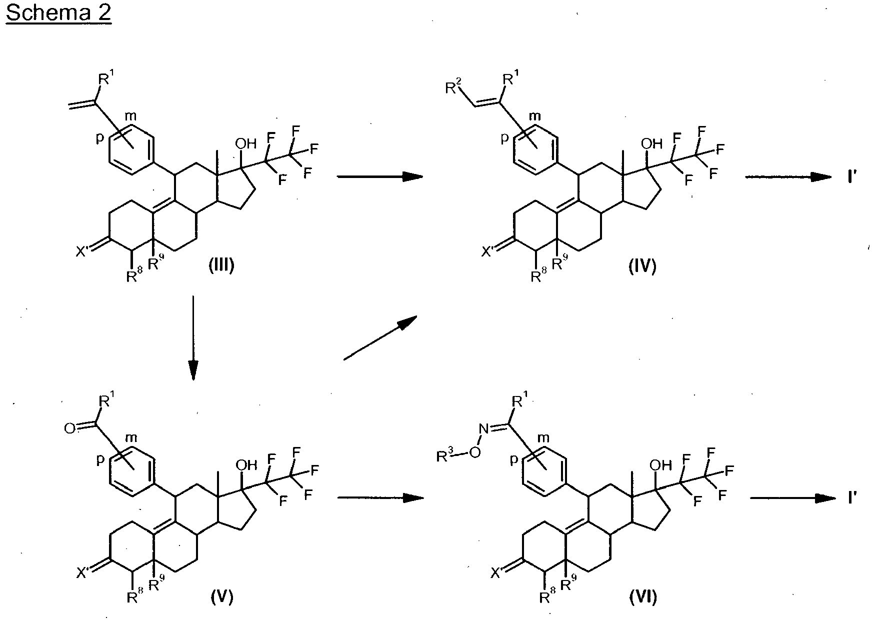

Die Umsetzung von Verbindungen der allgemeinen Formel III zu Verbindungen der allgemeinen Formeln IV erfolgt durch Metall katalysierte Kupplungsreaktionen mit Olefinen nach den, dem Fachmann bekannten Methoden. Als Katalysatoren bevorzugt sind Komplexe mit Ruthenium. Typische Ausführungen finden sich in den Beispielen 2a, 3a, 4a und 27a.

Die Umsetzung von Verbindungen der allgemeinen Formel III zu Verbindungen der allgemeinen Formel V erfolgt durch oxidative Spaltung der Doppelbindung nach den, dem Fachmann bekannten Methoden. Typische Ausführungen finden sich in den Beispielen 21b und 28b.

Die Umsetzung von Verbindungen der allgemeinen Formel V zu Verbindungen der allgemeinen Formeln IV erfolgt in einer Horner-Wittig- oder Wittig-Reaktion nach den, dem Fachmann bekannten Methoden. Typische Ausführungen finden sich in den Beispielen 6a-10a, 12a, 14a-16a, 18a, 21a-26a, 28a, 29, 32a und 33a.

Die Umsetzung von Verbindungen der allgemeinen Formel V zu Verbindungen der allgemeinen Formeln VI erfolgt durch Umsetzung mit Oximen oder Oximethern nach den, dem Fachmann bekannten Methoden. Typische Ausführungen finden sich in den Beispielen 34a-38a.

In R¹, R² oder R³ enthaltene funktionelle Gruppen in Verbindungen der allgemeinen Formeln III, IV, V, VI und I' lassen sich nach den, dem Fachmann bekannten Methoden weiter abwandeln. Genannt sei beispielsweise die Esterspaltung wie diese in den Beispielen 5, 13, 17, 50, 52 und 56 beschrieben ist, die Veresterung von Carbonsäuren wie dies exemplarisch in den Beispielen 30 und 31 beschrieben ist, die Umwandlung einer Hydroxylgruppe in eine Azidgruppe, deren Reduktion zu einer Aminogruppe wie dies exemplarisch in den Beispielen 39b und 39a beschrieben ist, sowie deren weitere funktionelle Transformation beispielsweise zu Harnstoffen wie dies exemplarisch in den Beispielen 39, 40-48, 49a, 51 a, 53, 54,55a, 57 und 58 beschrieben ist.

Soweit durch die beschriebenen Umsetzungen noch nicht geschehen, werden in den Verbindungen der allgemeinen Formeln I', IV, VI, gegebenenfalls vorhandene Ketale in X' gespalten und/oder, falls R⁸ die Bedeutung Wasserstoff und R⁹ die Bedeutung Hydroxyl besitzt, Wasser eliminiert. Typische Ausführung finden sich in den Beispielen 1-4, 6-12, 14-16, 18-28, 32-38, 49, 51 und 55.

Soweit die Herstellung der Ausgangsverbindungen hier nicht beschrieben ist, sind diese dem Fachmann bekannt oder analog zu bekannten Verbindungen oder hier beschriebenen Verfahren herstellbar.

Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die folgenden Beispiele dienen der Erläuterung der Erfindung ohne diese zu beschränken..

### Beispiel 1

### (8S,11R,13S,14S,17S)-17-Hydroxy-13-methyl-17-pentafluorethyl-11-(4-vinylphenyl)-1,2,6,7,8,11,12,13,14,15,16,17-dodecahydro-cyclopenta[a]phenanthren-3-on

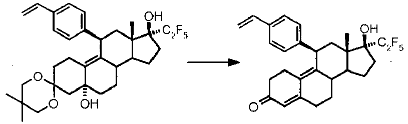

Die Lösung von 5,0g (8,38 mmol) der nach Beispiel 1a dargestellten Verbindung in 250 ml Aceton versetzte man mit 12 ml einer 4N Salzsäure und rührte 30 Minuten bei 23°C. Man goss in eine gesättigte Natriumhydrogencarbonatlösung, extrahierte mehrfach mit Dichlormethan, trocknete die vereinigten organischen Extrakte über Natriumsulfat und reinigte den nach Filtration und Lösungsmittelabzug erhaltenen Rückstand durch Kristallisation aus Diisopropylether. Isoliert wurden 3,92 g (95%) der Titelverbindung als kristalliner Feststoff.

¹H-NMR (CDCl₃): δ= 0,66 (3H), 1,45-1,60 (2H), 1,76-2,68 (14H), 2,77 (1H), 4,48 (1H), 5,27 (1H), 5,77 (1H), 5,83 (1H), 6,73 (1H), 7,18 (2H), 7,38 (2H) ppm.

### Beispiel 1 a

### (5R,8S,11R,13S,14S,17S)-11-(4-Ethenylphenyl)-5',5',13-trimethyl-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

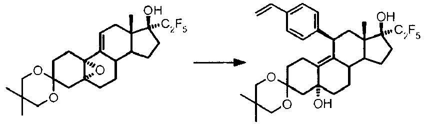

Aus 2,22 g Magnesiumspäne und einer Lösung von 11,95 ml 4-Bromstyrol in 75 ml Tetrahydrofuran stellte man unter leichtem Erwärmen auf 30-50°C und gegebenenfalls unter Zusatz eines Jodkristalls das Grignard-Reagenz her. Man kühlte auf 5°C, versetzte mit 117 mg Küpfer(I)chlorid und tropfte die Lösung von 15 g (30,5 mmol) (5R,8S,10R,13S,14S,17S)-17-Pentafluorethyl-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol in 150 ml Tetrahydrofuran zu. Man rührte noch 1 Stunde bei 23°C, verdünnte mit Essigsäureethylester und goss in eine gesättigte Ammoniumchloridlösung. Die wäßrige Phase extrahierte man noch mehrfach mit Essigsäureethylester, wusch die vereinigten organischen Extrakte mit gesättigter Natriumchloridlösung und trocknete über Natriumsulfat. Den nach Filtration und Lösungsmittelabzug erhaltenen Feststoff kristallisierte man aus Hexan um und isolierte 16,6 g (91%) der Titelverbindung als farblosen Feststoff.

### Beispiel 2

### (8S,11R,13S,14S,17S)-17-Hydroxy-11-[4-((E)-3-hydroxy-propenyl)-phenyl]-13-methyl-17-pentafluorethyl-1,2,6,7,8,11,12,13,14,15,16,17-dodecahydrocyclopenta[a]phenanthren-3-on

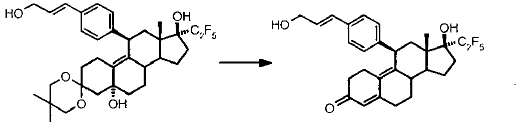

In Analogie zu Beispiel 1 setzte man 7,9 mg (13 µmol) der nach Beispiel 2a dargestellten Verbindung um und isolierte nach Aufarbeitung und Reinigung 4,5 mg (68%) der Titelverbindung als farblosen Schaum.

¹ H-NMR (CDCl₃): δ= 0,65 (3H), 1,42-1,64 (3H), 1,78-1,91 (3H), 2,04-2,69 (11 H), 2,78 (1H), 4,37 (2H), 4,49 (1H), 5,83 (1H), 6,38 (1H), 6,63 (1H), 7,17 (2H), 7,36 (2H) ppm.

### Beispiel 2a

### (5R,8S,11 R,13S,14S,17S)-11-{4-[(1E)-3-Hydroxyprop-1-en-1-yl]phenyl}-5',5',13-trimethyl-17-(pentafluorethyl)-1,2,3,4,6,7,8,11,12,13,14,15,16,17-tetradecahydrospiro[cyclopenta[a]phenanthren-3,2'-[1,3]dioxan]-5,17-diol

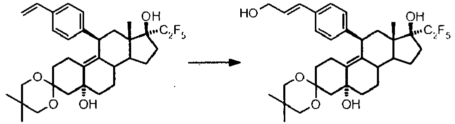

Die Lösung von 1,0 g (1,68 mmol) der nach Beispiel 1 a dargestellten Verbindung in 16,5 ml Dichlormethan versetzte man mit 0,57 ml Allylalkohol, 145 mg [[1,3-bis(2,4,6-trimethylphenyl)-2-imidazolidinyl]methyl]dichloro(phenylmethylen)(tricyclohexylphosphoranyl)-ruthenium und erhitzt 4,5 Stunden unter Rückfluß. Man reinigte durch Chromatographie und isolierte 163 mg (16%) der Titelverbindung als farblosen Schaum.

### Beispiel 3

### (8S,11R,13S,14S,17S)-17-Hydroxy-11-{4-[(E)-3-(4-methoxy-phenyl)-propenyl]-phenyl}-13-methyl-17-pentafluorethyl-1,2,6,7,8,11,12,13,14,15,16,17-dodecahydrocyclopenta[a]phenanthren-3-on

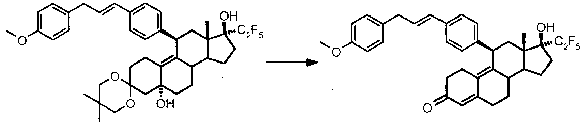

In Analogie zu Beispiel 1 setzte man 15 mg (21 µmol) der nach Beispiel 3a dargestellten Verbindung um und isolierte nach Aufarbeitung und Reinigung 7,3 mg (57%) der Titelverbindung als farblosen Schaum.

¹H-NMR (CDCl₃): δ= 0,65 (3H), 1,44-1,60 (2H), 1,76-1,90 (3H), 2,07 (1H), 2,10 (1H), 2,26-2,68 (9H), 2,77 (1 H), 3,52 (2H), 3,84 (3H), 4,46 (1 H), 5,82 (1 H), 6,30-6,47 (2H), 6,89 (2H), 7,13 (2H), 7,20 (2H), 7,31 (2H) ppm.

### Beispiel 3a

### (5R,8S,11R,13S,14S,17S)-11-{4-[(1E)-3-(4-methoxyphenyl)prop-1-en-1-yl]phenyl}-5',5',13-trimethyl-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

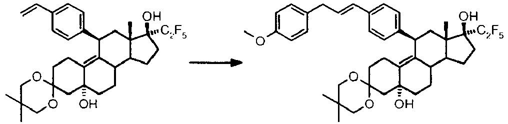

In Analogie zu Beispiel 2a setzte man 50 mg (84 µmol) der nach Beispiel 1a dargestellten Verbindung um und isolierte nach Aufarbeitung und Reinigung 15 mg (25%) der Titelverbindung als farblosen Schaum.

### Beispiel 4

### 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-malonsäuredimethylester

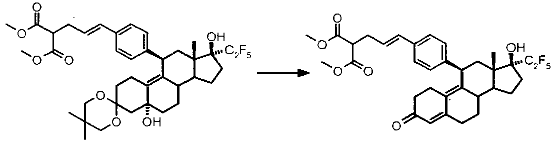

In Analogie zu Beispiel 1 setzte man 51 mg (69 µmol) der nach Beispiel 4a dargestellten Verbindung um und isolierte nach Aufarbeitung und Reinigung 15,2 mg (35%) der Titelverbindung als farblosen Schaum.

¹H-NMR (CDCl₃): δ= 0,60 (3H), 1,40-1,54 (2H), 1,73-1,87 (3H), 2,05 (1H), 2,20 (1H), 2,24-2,63 (9H), 2,72 (1H), 2,79 (2H), 3,51 (1H), 3,74 (6H), 4,41 (1H), 5,78 (1H), 6,10 (1 H), 6,43 (1 H), 7,09 (2H), 7,24 (2H) ppm.

### Beispiel 4a

### Dimethyl-[(2E)-3-{4-[(5R,8S,11R,13S,14S,17S)-5,17-dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}prop-2-en-1-yl]propandioat

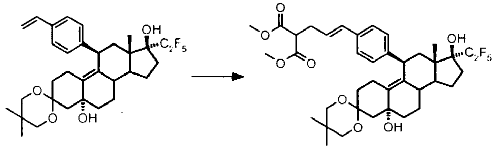

In Analogie zu Beispiel 2a setzte man 200 mg (340 µmol) der nach Beispiel 1a dargestellten Verbindung um und isolierte nach Aufarbeitung und Reinigung 42 mg (20%) der Titelverbindung als farblosen Schaum.

### Beispiel 5

### 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-1-yl)-phenyl]-allyl}-malonsäure

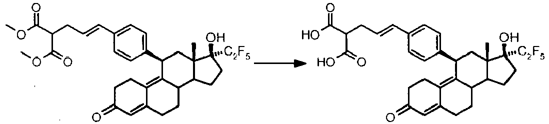

Die Lösung von 323 mg (0,51 mmol) der nach Beispiel 4 dargestellten Verbindung in 5,4 ml Tetrahydrofuran versetzte man mit 2,3 ml einer 5%igen wäßrigen Lithiumhydroxidlösung und rührte 3 Stunden bei 23°C. Man säuerte durch Zugabe einer 1 molaren Salzsäure an, sättigte mit Natriumchlorid, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 146 mg (47%) der Titelverbindung.

¹H-NMR (CD₃OD): δ=0,59 (3H), 1,33-1,55 (2H), 1,68-1,83 (3H), 2,08 (1H), 2,16-2,47 (5H), 2,54-2,84 (7H), 3,39 (1H), 4,48 (1H), 5,73 (1H), 6,17 (1H), 6,44 (1H), 7,15 (2H), 7,26 (2H) ppm.

### Beispiel 6

### (8S,11R,13S,14S,17S)-17-Hydroxy-13-methyl-17-pentafluorethyl-11-[4-((E)-2-pyridin-2-yl-vinyl)-phenyl]-1,2,6,7,8,11,12,13,14,15,16,17-dodecahydrocyclopenta[a]phenanthren-3-on

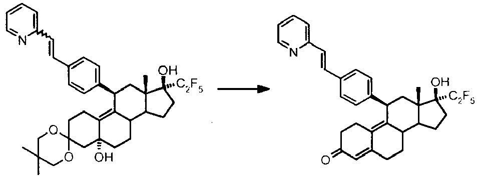

In Analogie zu Beispiel 1 setzte man 67 mg (99 µmol) eines Gemisches der nach Beispiel 6a dargestellten Verbindung A und B um und isolierte nach Aufarbeitung und Reinigung 42 mg (74%) der Titelverbindung als farblosen Schaum.

¹H-NMR (CDCl₃): δ= 0,67 (3H), 1,44-1,62 (2H), 1,79-1,92 (3H), 2,12 (1H), 2,24 (1H), 2,30-2,71 (9H), 2,79 (1H), 4,51 (1H), 5,84 (1H), 7,13-7,26 (4H), 7,43 (1H), 7,54 (2H), 7,62 (1 H), 7,71 (1 H), 8,64 (1H) ppm.

### Beispiel 6a

### (5R,8S,11R,13S,14S,17S)-5',5',13-Trimethyl-17-(pentafluorethyl)-11-{4-[(E)-2-(pyridin-2-yl)ethenyl]phenyl}-1,2,3,4,6,7,8,11,12,13,14,15,16,17-tetradecahydrospiro[cyclopenta[a]phenanthren-3,2'-[1,3]dioxan]-5,17-diol

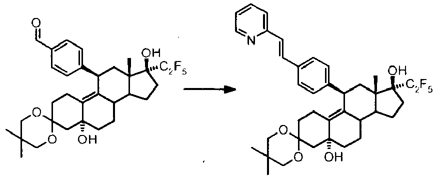

Die Lösung von 345 mg Pyridin-2-ylmethyl-phosphonsäurediethylester in 10 ml Tetrahydrofuran versetzte man bei -10°C mit 0,6 ml einer 1,6 molaren Lösung von n-Butyllithium in Hexan und rührte 1,5 Stunden bei 23°C. Anschließend kühlte man auf - 70°C, tropfte die Lösung von 300 mg (0,5 mmol) der nach Beispiel 28b dargestellten Verbindung in 5 ml Tetrahydrofuran zu, entfernte das Kühlbad und ließ 1,5 Stunden reagieren. Man goss in eine gesättigte Ammoniumchloridlösung, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 302 mg (89%) der Titelverbindung als farbloser Schaum.

### Beispiel 7

### (8S,11R,13S,14S,17S)-17-Hydroxy-13-methyl-17-pentafluorethyl-11-[4-((E)-2-pyridin-3-yl-vinyl)-phenyl]-1,2,6,7,8,11,12,13,14,15,16,17-dodecahydrocyclopenta[a]phenanthren-3-on

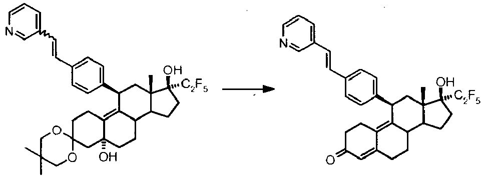

In Analogie zu Beispiel 1 setzte man 100 mg (0,15 mmol) eines Gemisches der nach Beispiel 7a dargestellten Verbindung A und B um und isolierte nach Aufarbeitung und Reinigung 61,4 mg (73%) der Titelverbindung als farblosen Schaum.

¹H-NMR (CDCl₃): δ=0,66 (3H), 1,41-1,48 (2H), 1,75-1,91 (3H), 2,08 (1H), 2,22-2,65 (9H), 2,74 (1H), 3,97 (1H), 4,43 (1H), 5,80 (1 H), 7,04 (1H), 7,09-7,18 (3H), 7,33 (1H), 7,43 (2H), 7,86 (1H), 8,46 (1 H), 8,63 (1H) ppm.

### Beispiel 7a

### (5R,8S,11R,13S,14S,17S)-5',5',13-Trimethyl-17-(pentafluorethyl)-11-{4-[(E/Z)-2-(pyridin-3-yl)ethenyl]phenyl}-1,2,3,4,6,7,8,11,12,13,14,15,16,17-tetradecahydro-spiro[cyclopenta[a]phenanthren-3,2'-[1,3]dioxan]-5,17-diol

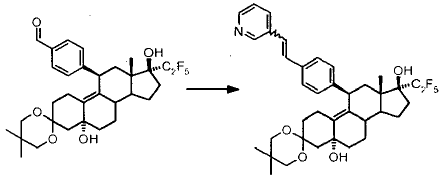

In Analogie zu Beispiel 6a setzte man 250 mg (0,42 mmol) der nach Beispiel 28b dargestellten Verbindung unter Verwendung von Pyridin-3-ylmethyl-phosphonsäurediethylester um und isolierte nach Aufarbeitung und Reinigung 170 mg (60%) eines Gemisches der beiden Titelverbindungen als farblosen Schaum.

### Beispiel 8

### (8S,11R,13S,14S,17S)-17-Hydroxy-13-methyl-17-pentafluorethyl-11-[4-((E)-2-pyridin-4-yl-vinyl)-phenyl]-1,2,6,7,8,11,12,13,14,15,16,17-dodecahydrocyclopenta[a]phenanthren-3-on

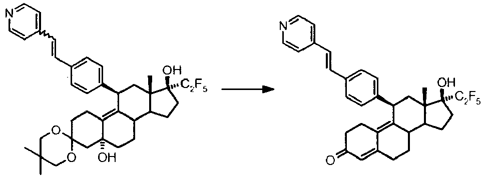

In Analogie zu Beispiel 1 setzte man 100 mg (0,15 mmol) eines Gemisches der nach Beispiel 8a dargestellten Verbindung A und B um und isolierte nach Aufarbeitung und Reinigung 57,2 mg (68%) der Titelverbindung als farblosen Schaum.

¹H-NMR (CDCl₃): δ=0,63 (3H), 1,41-1,58 (2H), 1,74-1,89 (3H), 2,08 (1H), 2,22-2,68 (10H), 2,74 (1H), 4,46 (1H), 5,79 (1H), 6,98 (1H), 7,19 (2H), 7,27 (1H), 7,35 (2H), 7,45 (2H), 8,55 (2H) ppm.

### Beispiel 8a

### (5R,8S,11R,13S,14S,17S)-5',5',13-Trimethyl-17-(pentafluorethyl)-11-{4-[(E/Z)-2-(pyridin-4-yl)ethenyl]phenyl}-1,2,3,4,6,7,8,11,12,13,14,15,16,17-tetradecahydro-spiro[cyclopenta[a]phenanthren-3,2'-[1,3]dioxan]-5,17-diol

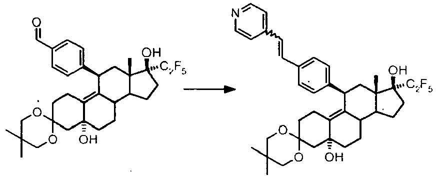

In Analogie zu Beispiel 6a setzte man 200 mg (0,33 mmol) der nach Beispiel 28b dargestellten Verbindung unter Verwendung von Pyridin-4-ylmethyl-phosphonsäurediethylester um und isolierte nach Aufarbeitung und Reinigung 182 mg (81%) eines Gemisches der beiden Titelverbindungen als farblosen Schaum.

### Beispiel 9

### (8S,11R,13S,14S,17S)-17-Hydroxy-13-methyl-11-{4-[(E)-2-(2-methyl-thiazol-4-yl)-vinyl]-phenyl}-17-pentafluorethyl-1,2,6,7,8,11,12,13,14,15,16,17-dodecahydrocyclopenta[a]phenanthren-3-on

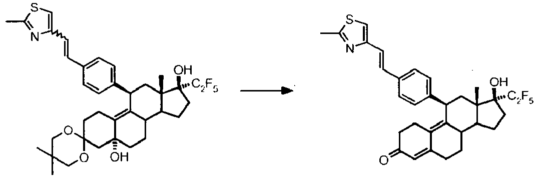

In Analogie zu Beispiel 1 setzte man 200 mg (0,29 mmol) des nach Beispiel 9a dargestellten Isomerengemisches um und isolierte nach Aufarbeitung und Reinigung 129 mg (76%) der Titelverbindung als farblosen Schaum.

¹H-NMR (CDCl₃): δ= 0,63 (3H), 1,41-1,55 (2H), 1,75-1,85 (3H), 2,06 (1H), 2,08 (1H), 2,27-2,65 (9H), 2,74 (1 H), 2,75 (3H), 4,45 (1 H), 5,79 (1 H), 6,99 (1 H), 7,01 (1 H), 7,15 (2H), 7,38 (1H), 7,42 (2H) ppm.

### Beispiel 9a

### (5R,8S,11R,13S,14S,17S)-5',5',13-Trimethyl-11-{4-[(E/Z)-2-(2-methyl-1,3-thiazol-4-yl)ethenyl]phenyl}-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

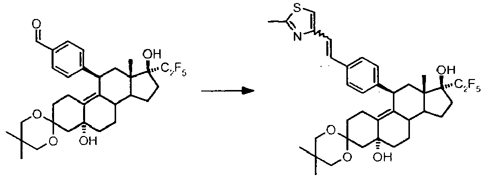

In Analogie zu Beispiel 6a setzte man 500 mg (0,84 mmol) der nach Beispiel 28b dargestellten Verbindung unter Verwendung von (2-Methyl-thiazol-4-ylmethyl)-phosphonsäurediethylester um und isolierte nach Aufarbeitung und Reinigung 479 mg (83%) eines Gemisches der beiden Titelverbindungen als farblosen Schaum.

### Beispiel 10

### (8S,11R,13S,14S,17S)-17-Hydroxy-13-methyl-11-{4-[(E)-2-(2-methyl-benzothiazol-5-yl)-vinyl]-phenyl}-17-pentafluorethyl-1,2,6,7,8,11,12,13,14,15,16,17-dodecahydrocyclopenta[a]phenanthren-3-on

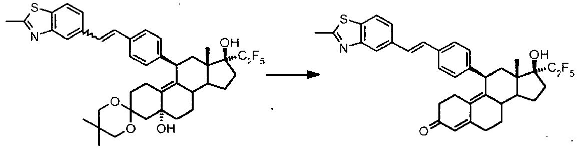

In Analogie zu Beispiel 1 setzte man 84 mg (0,11 mmol) der nach Beispiel 10a dargestellten Verbindung um und isolierte nach Aufarbeitung und Reinigung 44 mg (61%) der Titelverbindung als farblosen Schaum.

¹H-NMR (CDCl₃): δ= 0,64 (3H), 1,41-1,57 (2H), 1,75-1,88 (3H), 2,07 (1H), 2,17 (1H), 2,24-2,67 (9H), 2,75 (1 H), 2,84 (3H), 4,46 (1 H), 5,79 (1 H), 7,09-7,22 (4H), 7,45 (2H), 7,53 (1H), 7,78 (1H), 8,02 (1H) ppm.

### Beispiel 10a

(5R,8S,11R,13S,14S,17S)-5',5',13-Trimethyl-11-{4-[(E)-2-(2-methyl-1,3-benzothiazol-5-yl)ethenyl]phenyl}-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

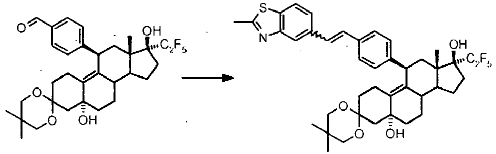

In Analogie zu Beispiel 6a setzte man 196 mg (0,33 mmol) der nach Beispiel 28b dargestellten Verbindung unter Verwendung von (2-Methyl-benzothiazol-5-ylmethyl)-phosphonsäurediethylester um und isolierte nach Aufarbeitung und Reinigung 98 mg (40%) der Titelverbindungen als farblosen Schaum.

### Beispiel 11

### (8S,11R,13S,14S,17S)-17-Hydroxy-11-(4-isopropenyl-phenyl)-13-methyl-17-pentafluorethyl-1,2,6,7,8,11,12,13,14,15,16,17-dodecahydro-cyclopenta[a]phenanthren-3-on

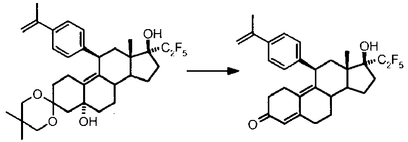

In Analogie zu Beispiel 1 setzte man 21,4 mg (35 µmol) der nach Beispiel 11 a dargestellten Verbindung um und isolierte nach Aufarbeitung und Reinigung 11,1 mg (63%) der Titelverbindung als farblosen Schaum.

¹H-NMR (CDCl₃): δ= 0,62 (3H), 1,37-1,57 (2H), 1,73-1,88 (3H), 2,01-2,65 (11H), 2,13 (3H), 2,74 (1 H), 4,44 (1 H), 5,06 (1 H), 5,37 (1 H), 5,78 (1 H), 7,13 (2H), 7,40 (2H) ppm.

### Beispiel 11a

### (5R,8S,11R,13S,14S,17S)-5',5',13-Trimethyl-17-(pentafluorethyl)-11-[4-(prop-1-en-2-yl)phenyl]-1,2,3,4,6,7,8,11,12,13,14,15,16,17-tetradecahydro-spiro[cyclopenta[a]phenanthren-3,2'-[1,3]dioxan]-5,17-diol

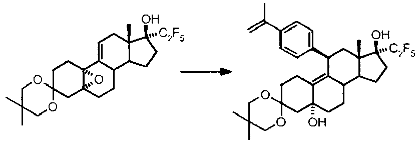

In Analogie zu Beispiel 1a setzte man 5 g (10,2 mmol) (5R,8S,10R,13S,14S,17S)-17-(Pentafluorethyl)-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol mit dem aus 10 g 1-Brom-4-isopropenyl-benzol hergestellten Grignard-Reagenz um und isolierte nach Aufarbeitung und Reinigung 4,01 g (65%) der Titelverbindung als farblosen Schaum.

### Beispiel 12

### (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-acrylsäureethylester

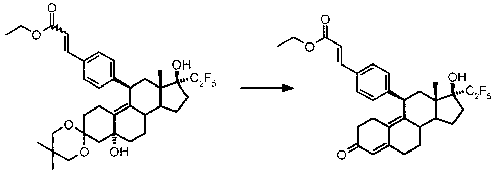

In Analogie zu Beispiel 1 setzte man 50 mg (75 µmol) der nach Beispiel 12a dargestellten Verbindung um und isolierte nach Aufarbeitung und Reinigung 25,2 mg (60%) der Titelverbindung als farblosen Schaum.

¹H-NMR (CDCl₃): δ= 0,60 (3H), 1,33 (3H), 1,42-1,54 (2H), 1,76-1,86 (3H), 2,07 (1H), 2,13 (1H), 2,23-2,64 (9H), 2,73 (1H), 4,26 (2H), 4,46 (1H), 5,79 (1H), 6,40 (1H), 7,21 (2H), 7,45 (2H), 7,64 (1H) ppm.

### Beispiel 12a

Ethyl-(2E)-3-{4-[(5R,8S,11R,13S,14S,17S)-5,17-dihydroxy-5',5',13-trimethyl-17-(pentafluorethyl)-1,2,4,5,6,7,8,11,12,13,14,15,16,17-tetradecahydro-spiro[cyclopenta[a]phenanthren-3,2'-[1,3]dioxan]-11-yl]phenyl}prop-2-enoat

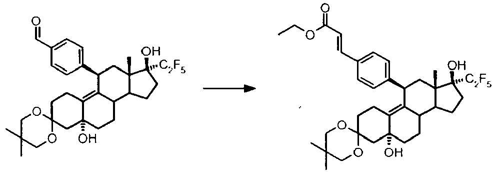

In Analogie zu Beispiel 6a setzte man 250 mg (0,42 mmol) der nach Beispiel 28b dargestellten Verbindung unter Verwendung von (Diethoxy-phosphoryl)-essigsäureethylester um und isolierte nach Aufarbeitung und Reinigung 230 mg (82%) der Titelverbindung als farblosen Schaum.

### Beispiel 13

### (E)-3-[4-((8S,11 R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-acrylsäure

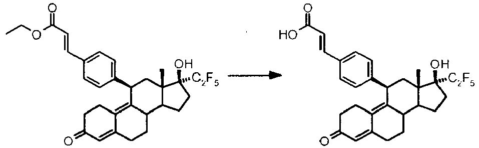

In Analogie zu Beispiel 5 setzte man 20 mg (35 µmol) der nach Beispiel 12 dargestellten Verbindung um und isolierte nach Aufarbeitung und Reinigung 5,2 mg (28%) der Titelverbindung als farblosen Schaum.

¹H-NMR (CD₃OD): δ= 0,59 (3H), 1,37-1,55 (2H), 1,71-1,82 (3H), 2,10 (1H), 2,18-2,46 (5H), 2,56-2,73 (4H), 2,81 (1 H), 4,54 (1 H), 5,74 (1 H), 6,44 (1 H), 7,27 (2H), 7,46-7,52 (3H) ppm.

### Beispiel 14

### (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-acrylnitril

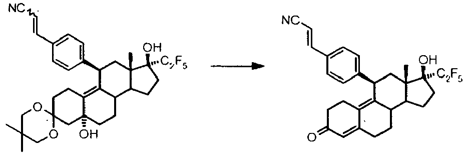

In Analogie zu Beispiel 1 setzte man 50 mg (80 µmol) der nach Beispiel 14a dargestellten Verbindung um und isolierte nach Aufarbeitung und Reinigung 21,2 mg (51%) der Titelverbindung als farblosen Schaum.

¹H-NMR (CDCl₃): δ= 0,58 (3H), 1,40-1,56 (2H), 1,74-1,87 (3H), 2,03-2,13 (2H), 2,18-2,65 (9H), 2,72 (1 H), 4,47 (1 H), 5,80 (1 H), 5,84 (1 H), 7,24 (2H), 7,36 (1 H), 7,38 (2H) ppm.

### Beispiel 14a

### (2E/Z)-3-{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}prop-2-enonitril

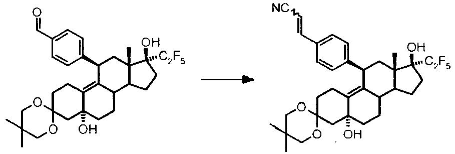

In Analogie zu Beispiel 6a setzte man 1,0 g (1,67 mmol) der nach Beispiel 28b dargestellten Verbindung unter Verwendung von Cyanomethylphosphonsäurediethylester um und isolierte nach Aufarbeitung und Reinigung 961 mg (93%) der Titelverbindung als farblosen Schaum.

### Beispiel 15

### (2E,4E)-5-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-penta-2,4-diensäureethylester

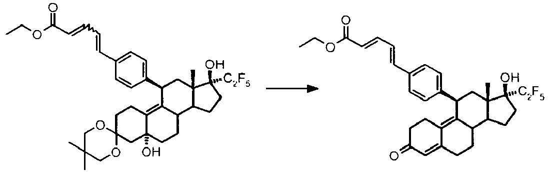

In Analogie zu Beispiel 1 setzte man 50 mg (72 µmol) eines Gemisches der nach Beispiel 15a dargestellten Verbindungen um und isolierte nach Aufarbeitung und Reinigung 27,7 mg (65%) der Titelverbindung als gelben Schaum.

¹H-NMR (CDCl₃): δ= 0,61 (3H), 1,31 (3H), 1,40-1,56 (2H), 1,73-1,87 (3H), 2,07 (1H), 2,11 (1H), 2,21-2,65 (9H), 2,73 (1H), 4,22 (2H), 4,45 (1H), 5,79 (1H), 5,98 (1H), 6,77-6,91 (2H), 7,17 (2H), 7,38 (2H), 7,44 (1H) ppm.

### Beispiel 15a

### Ethyl-(2E/Z,4E/Z)-5-{4-[(5R,8S,11R,13S,14S,17S)-5,17-dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}penta-2,4-dienoat

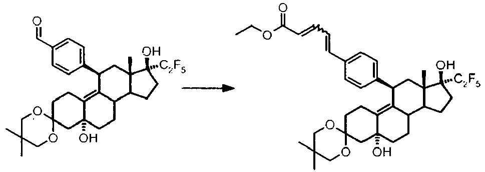

In Analogie zu Beispiel 6a setzte man 180 mg (0,3 mmol) der nach Beispiel 28b dargestellten Verbindung unter Verwendung von (E)-4-(Diethoxy-phosphoryl)-but-2-ensäureethylester um und isolierte nach Aufarbeitung und Reinigung 170 mg (81%) der Titelverbindung als farblosen Schaum.

### Beispiele 16

### (8S,11R,13S,14S,17S)-17-Hydroxy-13-methyl-11-[4-((E)-3-oxo-but-1-enyl)-phenyl]-17-pentafluorethyl-1,2,6,7,8,11,12,13,14,15,16,17-dodecahydrocyclopenta[a]phenanthren-3-on

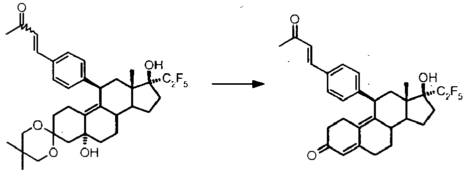

In Analogie zu Beispiel 1 setzte man 33 mg (52 µmol) eines Gemisches der nach Beispiel 16a dargestellten Verbindungen um und isolierte nach Aufarbeitung und Reinigung 6,7 mg (24%) der Titelverbindung als blass gelben Schaum.

¹H-NMR (CDCl₃): δ= 0,60 (3H), 1,41-1,56 (2H), 1,74-1,87 (3H), 2,03-2,15 (2H), 2,21-2,65 (9H), 2,38 (3H), 2,73 (1 H), 4,46 (1 H), 5,80 (1 H), 6,68 (1 H), 7,23 (2H), 7,43-7,51 (3H) ppm.

### Beispiel 16a

### (3E/Z)-4-{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}but-3-en-2-on

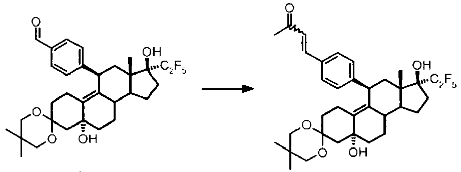

In Analogie zu Beispiel 6a setzte man 180 mg (0,3 mmol) der nach Beispiel 28b dargestellten Verbindung unter Verwendung von (2-Oxopropyl)-phosphonsäuredimethylester um und isolierte nach Aufarbeitung und Reinigung 33 mg (17%) der Titelverbindungen als farblosen Schaum.

### Beispiel 17

### (2E,4E)-5-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-penta-2,4-diensäure

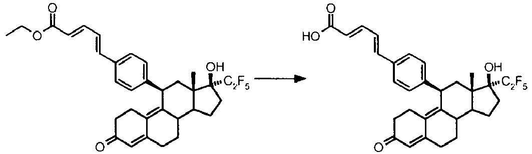

In Analogie zu Beispiel 5 setzte man 20 mg (35 µmol) der nach Beispiel 15 dargestellten Verbindung um und isolierte nach Aufarbeitung und Reinigung 3,1 mg (16%) der Titelverbindung als farblosen Schaum.

¹H-NMR (CD₃OD): δ**=** 0,60 (3H), 1,35-1,59 (2H), 1,69-1,83 (3H), 2,09 (1H), 2,17-2,46 (5H), 2,54-2,74 (4H), 2,80 (1H), 4,51 (1H), 5,73 (1H), 5,99 (1H), 6,78-6,97 (2H), 7,19-7,32 (3H), 7,42 (2H) ppm.

### Beispiel 18

### (8S,11R,13S,14S,17S)-11-[((E/Z)-4-Buta-1,3-dienyl)-phenyl]-17-hydroxy-13-methyl-17-pentafluorethyl-1,2,6,7,8,11,12,13,14,15,16,17-dodecahydrocyclopenta[a]phenanthren-3-on

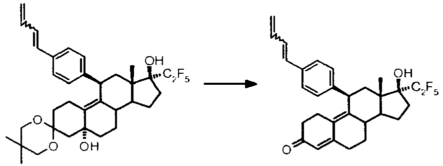

In Analogie zu Beispiel 1 setzte man 39 mg (63 µmol) eines Gemisches der nach Beispiel 18a dargestellten Verbindungen um und isolierte nach Aufarbeitung und Reinigung 12,9 mg (40%) der Titelverbindungen als blass gelben Schaum.

¹H-NMR (CDCl₃): δ= 0,62 (3H), 1,36-1,58 (2H), 1,73-1,88 (3H), 2,01-2,15 (2H), 2,22-2,65 (9H), 2,74 (1H), 4,44 (1H), 5,13-5,43 (2H), 5,79 (1H), 6,19-6,56 (2H), 6,69-6,95 (1 H), 7,08-7,19 (2H), 7,21-7,36 (2H) ppm.

### Beispiel 18a

### (5R,8S,11R,13S,14S,17S)-11-[((E/Z)-4-Buta-1,3-dienyl)-phenyl]-5',5',13-trimethyl-17-pentafluorethyl-1,2,3,4,6,7,8,11,12,13,14,15,16,17-tetradecahydro-spiro[cyclopenta[a]phenanthren-3,2'-[1,3]dioxan]-5,17-diol

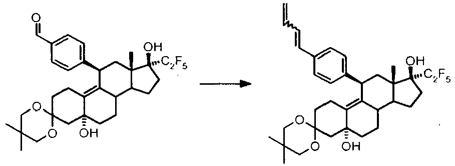

In Analogie zu Beispiel 6a setzte man 180 mg (0,3 mmol) der nach Beispiel 28b dargestellten Verbindung unter Verwendung von

Cyclopropyl(triphenylphosphonium)bromid um und isolierte nach Aufarbeitung und Reinigung 89 mg (48%) der Titelverbindungen als farblosen Schaum.

### Beispiel 19

### (8S,11R,13S,14S,17S)-17-Hydroxy-13-methyl-17-pentafluorethyl-11-(3-vinyl-phenyl)-1,2,6,7,8,11,12,13,14,15,16,17-dodecahydro-cyclopenta[a]phenanthren-3-on

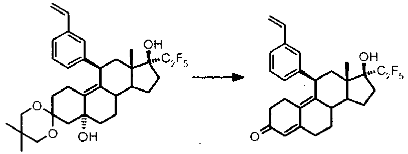

In Analogie zu Beispiel 1 setzte man 300 mg (0,5 mmol) der nach Beispiel 19a dargestellten Verbindung um und isolierte nach Aufarbeitung und Reinigung 165 mg (67%) der Titelverbindung als farblosen Schaum.

¹H-NMR (CDCl₃): δ= 0,62 (3H), 1,41-1,55 (2H), 1,75-1,86 (3H), 2,07 (1H), 2,10 (1H), 2,24-2,65 (9H), 2,74 (1 H), 4,45 (1 H), 5,25 (1 H), 5,71 (1 H), 5,79 (1 H), 6,68 (1 H), 7,05 (1 H), 7,20-7,26 (3H) ppm.

### Beispiel 19a

### (5R,8S,11R,13S,14S,17S)-11-(3-Ethenylphenyl)-5',5',13-trimethyl-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

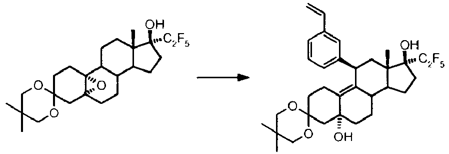

In Analogie zu Beispiel 1a setzte man 15 g (30,5 mmol) (5R,8S,10R,13S,14S,17S)-17-(Pentafluorethyl)-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol mit dem aus 12 ml 3-Bromstyrol hergestellten Grignard-Reagenz um und isolierte nach Aufarbeitung und Reinigung 16,6 g (92%) der Titelverbindung als farblosen Schaum.

### Beispiel 20

### (8S,11R,13S,14S,17S)-17-Hydroxy-11-(3-isopropenyl-phenyl)-13-methyl-17-pentafluorethyl-1,2,6,7,8,11,12,13,14,15,16,17-dodecahydrocyclopenta[a]phenanthren-3-on

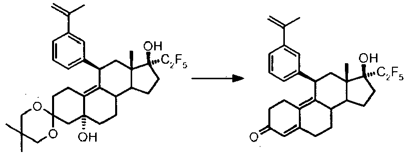

In Analogie zu Beispiel 1 setzte man 250 mg (0,41 mmol) der nach Beispiel 20a dargestellten Verbindung um und isolierte nach Aufarbeitung und Reinigung 164 mg (79%) der Titelverbindung als farblosen Schaum.

¹H-NMR (CDCl₃): δ= 0,62 (3H), 1,41-1,56 (2H), 1,74-1,87 (3H), 2,05 (1H), 2,09 (1H), 2,13 (3H), 2,23-2,65 (9H), 2,75 (1H), 4,46 (1H), 5,08 (1H), 5,32 (1 H), 5,79 (1H), 7,07 (1 H), 7,20-7,31 (3H) ppm.

### Beispiel 20a

### (5R,8S,11R,13S,14S,17S)-11-(3-Isopropenylphenyl)-5',5',13-trimethyl-17-(pentafluorethyl)-1,2,3,4,6,7,8,11,12,13,14,15,16,17-tetradecahydrospiro[cyclopenta[a]phenanthren-3,2'-[1,3]dioxan]-5,17-diol

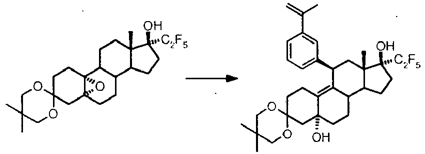

In Analogie zu Beispiel 1a setzte man 5,9 g (12,0 mmol) (5R,8S,10R,13S,14S,17S)-17-(Pentafluorethyl)-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol mit dem aus 11,9 g 1-Brom-3-isopropenyl-benzol hergestellten Grignard-Reagenz um und isolierte nach Aufarbeitung und Reinigung 5,36 g (73%) der Titelverbindung als farblosen Schaum.

### Beispiel 21

### (8S,11R,13S,14S,17S)-17-Hydroxy-13-methyl-17-pentafluorethyl-11-[3-((E)-2-pyridin-2-yl-vinyl)-phenyl]-1,2,6,7,8,11,12,13,14,15,16,17-dodecahydrocyclopenta[a]phenanthren-3-on

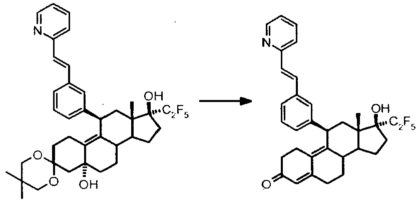

In Analogie zu Beispiel 1 setzte man 70 mg (0,1 mmol) der nach Beispiel 21a dargestellten Verbindung um und isolierte nach Aufarbeitung und Reinigung 45,4 mg (77%) der Titelverbindung als blass gelben Schaum.

¹H-NMR (CDCl₃): δ= 0,63 (3H), 1,42-1,56 (2H), 1,76-1,87 (3H), 2,08 (1H), 2,21 (1H), 2,26-2,67 (9H), 2,75 (1H), 4,47 (1H), 5,80 (1H), 7,10 (1H), 7,14 (1H), 7,17 (1H), 7,29 (1 H), 7,36 (1 H), 7,39 (1 H), 7,43 (1 H), 7,58 (1 H), 7,68 (1 H), 8,60 1(H) ppm.

### Beispiel 21 a

### (5R,8S,11R,13S,14S,17S)-5',5',13-Trimethyl-17-(pentafluorethyl)-11-{3-[(E)-2-(pyridin-2-yl)ethenyl]phenyl}-1,2,3,4,6,7,8,11,12,13,14,15,16,17-tetradecahydrospiro[cyclopenta[a]phenanthren-3,2'-[1,3]dioxan]-5,17-diol

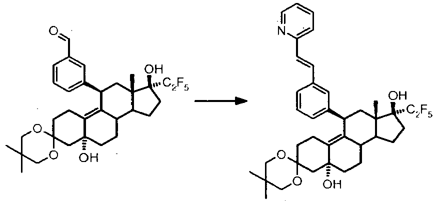

In Analogie zu Beispiel 6a setzte man 200 mg (0,33 mmol) der nach Beispiel 21b dargestellten Verbindung unter Verwendung von Pyridin-2-ylmethylphosphonsäurediethylester um und isolierte nach Aufarbeitung und Reinigung 172 mg (76%) der Titelverbindung als gelben Schaum.

### Beispiel 21 b

### 13-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyd

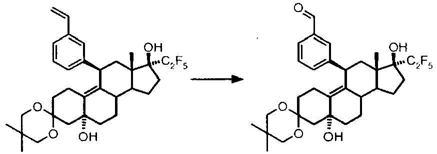

Die Lösung von 5,0 g (8,38 mmol) der nach Beispiel 19a dargestellten Verbindung in 250 ml Tetrahydrofuran versetzte man mit 20 ml Wasser, 1,25 ml Triethylamin, 5 ml einer gesättigten Natriumhydrogencarbonatlösung, 4,1 g Natriumperiodat, 2,7 ml einer 40 mmolaren Lösung von Osmiumtetroxid in tert.-Butanol und rührte bei 23°C. Nach 5 Stunden wurde die Zugabe an Oxidationsmitteln wiederholt und nach weiteren 16 Stunden von festen Anteilen filtriert. Man spülte mit Ethylacetat nach und wusch die vereinigten organischen Phasen mit halbgesättigter Natriumthiosulfatlösung und gesättigter Natriumchloridlösung. Den nach Filtration und Lösungsmittelabzug erhaltenen Rückstand reinigte man durch Kristallisation aus Diisopropylether. Isoliert wurden 3,67 g (73%) der Titelverbindung als farblosen Schaum.

### Beispiel 22

### (8S,11R,13S,14S,17S)-17-Hydroxy-13-methyl-17-pentafluorethyl-11-[3-((E)-2-pyridin-3-yl-vinyl)-phenyl]-1,2,6,7,8,11,12,13,14,15,16,17-dodecahydrocyclopenta[a]phenanthren-3-on

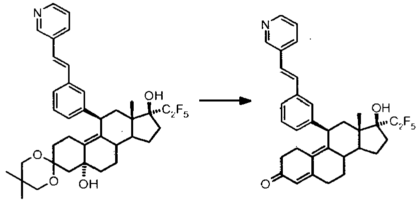

In Analogie zu Beispiel 1 setzte man 60 mg (89 µmol) der nach Beispiel 22a dargestellten Verbindung um und isolierte nach Aufarbeitung und Reinigung 34,6 mg (68%) der Titelverbindung als blass gelben Schaum.

¹H-NMR (CDCl₃): δ= 0,72 (3H), 1,44-1,59 (2H), 1,79-1,91 (3H), 2,10 (1H), 2,24-2,67 (9H), 2,73 (1H), 4,44 (1 H), 4,55 (1H), 5,80 (1H), 6,79 (1H), 6,86 (1H), 7,01 (1H), 7,20 (1 H), 7,24-7,33 (2H), 7,36 (1 H), 7,69 (1 H), 8,49 (1 H), 8,52 (1H) ppm.

### Beispiel 22a

### (5R,8S,11R,13S,14S,17S)-5',5',13-Trimethyl-17-(pentafluorethyl)-11-{3-[(E)-2-(pyridin-3-yl)ethenyl]phenyl}-1,2,3,4,6,7,8,11,12,13,14,15,16,17-tetradecahydrospiro[cyclopenta[a]phenanthren-3,2'-[1,3]dioxan]-5,17-diol

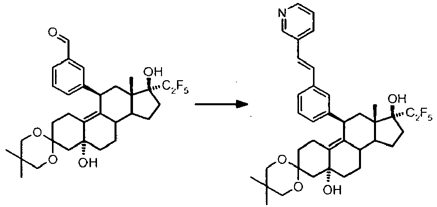

In Analogie zu Beispiel 6a setzte man 120 mg (0,2 mmol) der nach Beispiel 21b dargestellten Verbindung unter Verwendung von Pyridin-3-ylmethylphosphonsäurediethylester um und isolierte nach Aufarbeitung und Reinigung 68 mg (50%) der Titelverbindung als blass gelben Schaum.

### Beispiel 23

### (8S,11R,13S,14S,17S)-17-Hydroxy-13-methyl-17-pentafluorethyl-11-[3-((E)-2-pyridin-4-yl-vinyl)-phenyl]-1,2,6,7,8,11,12,13,14,15,16,17-dodecahydrocyclopenta[a]phenanthren-3-on

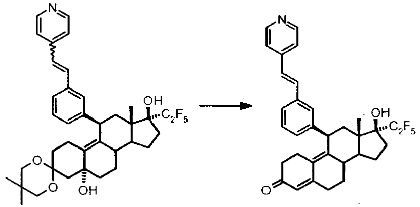

In Analogie zu Beispiel 1 setzte man 70 mg (0,1 mmol) eines Gemisches der nach Beispiel 23a dargestellten Verbindungen um und isolierte nach Aufarbeitung und Reinigung 38,4 mg (65%) der Titelverbindung als blass gelben Schaum.

¹H-NMR (CDCl₃): δ= 0,70 (3H), 1,45-1,57 (2H), 1,78-1,92 (3H), 2,10 (1H), 2,26-2,67 (8H), 2,70-2,80 (2H), 4,48 (1 H), 5,80 (1 H), 6,85 (1 H), 7,03 (2H), 7,16 (1 H), 7,28 (2H), 7,32 (2H), 7,40 (1 H), 8,49 (2H) ppm.

### Beispiel 23a

### (5R,8S,11R,13S,14S,17S)-5',5',13-Trimethyl-17-(pentafluorethyl)-11-{3-[(E/Z)-2-(pyridin-4-yl)ethenyl]phenyl}-1,2,3,4,6,7,8,11,12,13,14,15,16,17-tetradecahydro-spiro[cyclopenta[a]phenanthren-3,2'-[1,3]dioxan]-5,17-diol

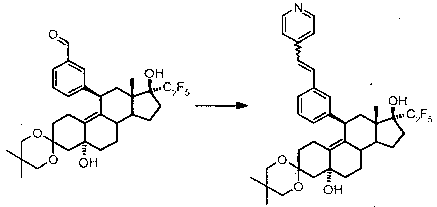

In Analogie zu Beispiel 6a setzte man 120 mg (0,2 mmol) der nach Beispiel 21 b dargestellten Verbindung unter Verwendung von Pyridin-4-ylmethyl-phosphonsäurediethylester um und isolierte nach Aufarbeitung und Reinigung 78 mg (58%) der Titelverbindung als blass gelben Schaum.

### Beispiel 24

### (8S,11R,13S,14S,17S)-17-Hydroxy-13-methyl-11-{3-[(E)-2-(2-methyl-thiazol-4-yl)-vinyl]-phenyl}-17-pentafluorethyl-1,2,6,7,8,11,12,13,14,15,16,17-dodecahydrocyclopenta[a]phenanthren-3-on

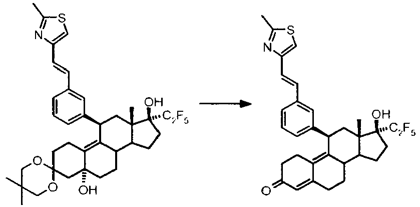

In Analogie zu Beispiel 1 setzte man 62 mg (89 µmol) der nach Beispiel 24a dargestellten Verbindung um und isolierte nach Aufarbeitung und Reinigung 37,6 mg (71%) der Titelverbindung als blass gelben Schaum.

¹H-NMR (CDCl₃): δ= 0,62 (3H), 1,42-1,55 (2H), 1,76-1,87 (3H), 2,06 (1H), 2,09 (1H), 2,25-2,66 (9H), 2,75 (4H), 4,46 (1 H), 5,79 (1 H), 6,99-7,09 (3H), 7,23-7,31 (2H), 7,35-7,41 (2H) ppm.

### Beispiel 24a

### (5R,8S,11R,13S,14S,17S)-5',5',13-Trimethyl-11-{3-[(E)-2-(2-methyl-1,3-thiazol-4-yl)ethenyl]phenyl}-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

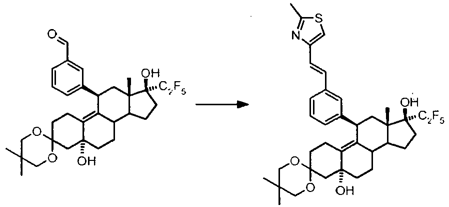

In Analogie zu Beispiel 6a setzte man 120 mg (0,2 mmol) der nach Beispiel 21 b dargestellten Verbindung unter Verwendung von (2-Methyl-thiazol-4-ylmethyl)-phosphonsäurediethylester um und isolierte nach Aufarbeitung und Reinigung 70 mg (50%) der Titelverbindung als blass gelben Schaum.

### Beispiel 25

### (8S,11R,13S,14S,17S)-17-Hydroxy-13-methyl-11-{3-[(E)-2-(2-methyl-benzothiazol-5-yl)-vinyl]-phenyl}-17-pentafluorethyl-1,2,6,7,8,11,12,13,14,15,16,17-dodecahydro-cyclopenta[a]phenanthren-3-on

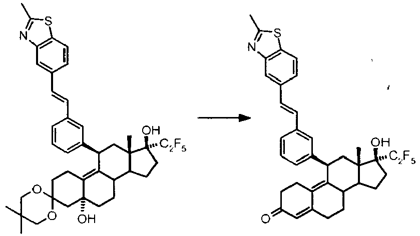

In Analogie zu Beispiel 1 setzte man 51 mg (69 µmol) der nach Beispiel 25a dargestellten Verbindung um und isolierte nach Aufarbeitung und Reinigung 23 mg (52%) der Titelverbindung als blass gelben Schaum.

¹H-NMR (CDCl₃): δ= 0,66 (3H), 1,43-1,57 (2H), 1,76-1,88 (3H), 2,09 (1H), 2,20 (1H), 2,27-2,67 (9H), 2,76 (1H), 2,85 (3H), 4,48 (1H), 5,80 (1H), 7,06 (1H), 7,12 (1H), 7,19 (1H), 7,29 (1H), 7,34 (1H), 7,39 (1H), 7,54 (1H), 7,79 (1H), 8,04 (1H) ppm.

### Beispiel 25a

### (5R,8S,11R,13S,14S,17S)-5',5',13-Trimethyl-11-{3-[(E)-2-(2-methyl-1,3-benzothiazol-5-yl)ethenyl]phenyl}-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

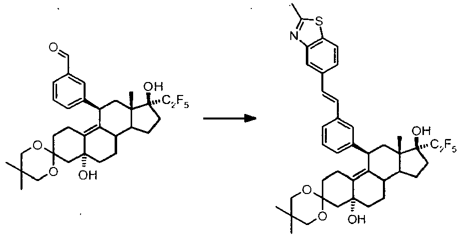

In Analogie zu Beispiel 6a setzte man 120 mg (0,2 mmol) der nach Beispiel 21 b dargestellten Verbindung unter Verwendung von (2-Methyl-benzothiazol-5-ylmethyl)-phosphonsäurediethylester um und isolierte nach Aufarbeitung und Reinigung 70 mg (50%) der Titelverbindung als blass gelben Schaum.

### Beispiel 26

### (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-acrylsäureethylester

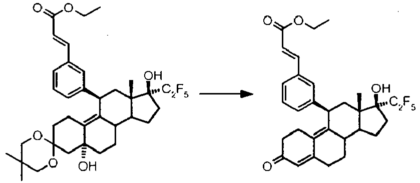

In Analogie zu Beispiel 1 setzte man 163 mg (240 µmol) der nach Beispiel 26a dargestellten Verbindung um und isolierte nach Aufarbeitung und Reinigung 101 mg (73%) der Titelverbindung als farblosen Schaum.

¹H-NMR (CDCl₃): δ= 0,59 (3H), 1,34 (3H), 1,40-1,57 (2H), 1,74-1,87 (3H), 2,08 (1H), 2,16 (1H), 2,20-2,65 (9H), 2,74 (1H), 4,26 (2H), 4,46 (1H), 5,80 (1H), 6,40 (1H), 7,18 (1 H), 7,27-7,39 (3H), 7,64 (1H) ppm.

### Beispiel 26a

### (E)-3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-acrylic acid ethyl ester

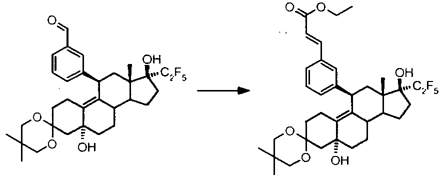

In Analogie zu Beispiel 6a setzte man 200 mg (0,33 mmol) der nach Beispiel 21b dargestellten Verbindung unter Verwendung von (Diethoxy-phosphoryl)-essigsäureethylester um und isolierte nach Aufarbeitung und Reinigung 171 mg (77%) der Titelverbindung als farblosen Schaum.

### Beispiel 27

### 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-malonitril

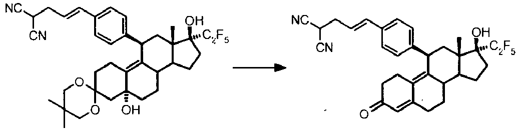

In Analogie zu Beispiel 1 setzte man 27 mg (40 µmol) der nach Beispiel 27a dargestellten Verbindung um und isolierte nach Aufarbeitung und Reinigung 14 mg (59%) der Titelverbindung als farblosen Schaum.

¹H-NMR (CDCl₃): δ=0,61 (3H), 1,42-1,55 (2H), 1,75-1,86 (3H), 2,03-2,11 (2H), 2,22-2,64 (9H), 2,73 (1H), 2,92 (2H), 3,84 (1 H), 4,44 (1H), 5,79 (1H), 6,15 (1H), 6,67 (1H), 7,16 (2H), 7,32 (2H) ppm.

### Beispiel 27a

### 2-{(E)-3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1, 3]dioxan]-11-yl)-phenyl]-allyl}-malonitril

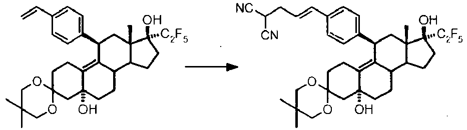

In Analogie zu Beispiel 2a setzte man 500 mg (0,84 mmol) der nach Beispiel 1 a dargestellten Verbindung unter Verwendung von Allylmalonitril um und isolierte nach Aufarbeitung und Reinigung 93 mg (16%) der Titelverbindung als farblosen Schaum.

### Beispiel 28

### 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-vinyl}-benzoesäure

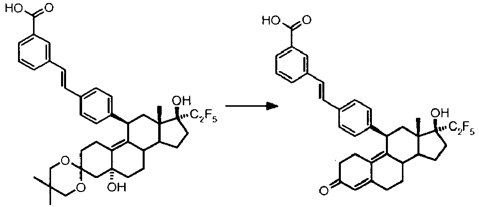

In Analogie zu Beispiel 1 setzte man 1,36 g (1,67 mmol) der nach Beispiel 28a dargestellten Verbindung um und isolierte nach Aufarbeitung und Reinigung 480 mg (47%) der Titelverbindung als farblosen Schaum.

¹H-NMR (CD₃OD): δ= 0,61 (3H), 1,34-1,56 (2H), 1,69-1,83 (3H), 2,07 (1H), 2,18-2,46 (5H), 2,52-2,70 (4H), 2,80 (1H), 4,47 (1H), 5,71 (1H), 6,60 (2H), 7,06-7,30 (6H), 7,76 (1 H), 7,84 (1H) ppm.

### Beispiel 28a

### 3-{(E)-2-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-vinyl}-benzoesäure

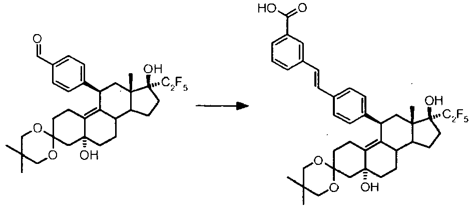

In Analogie zu Beispiel 6a setzte man 500 mg (0,84 mmol) der nach Beispiel 28b dargestellten Verbindung unter Verwendung von 3-(Diethoxy-phosphorylmethyl)-benzoesäure um und isolierte nach Aufarbeitung und Reinigung 48 mg (9%) der Titelverbindung als farblosen Schaum.

### Beispiel 28b

### 4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyde

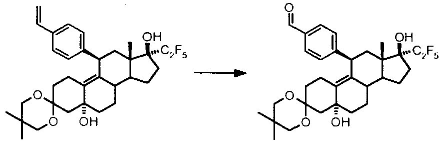

In Analogie zu Beispiel 21 b setzte man 3,99 g (6,69 mmol) der nach Beispiel 1 a dargestellten Verbindung um und isolierte nach Aufarbeitung und Reinigung 3,56 g (89%) der Titelverbindung als farblosen Schaum.

### Beispiel 29

### 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-1-yl)-phenyl]-vinyl}-benzoesäure

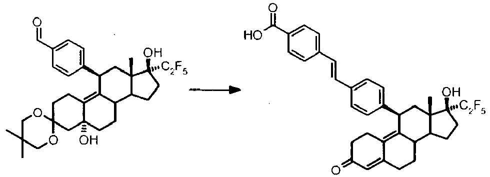

In Analogie zu Beispiel 6a setzte man 500 mg (0,84 mmol) der nach Beispiel 28b dargestellten Verbindung unter Verwendung von 4-(Diethoxy-phosphorylmethyl)-benzoesäure um und isolierte nach Aufarbeitung und Reinigung 105 mg (21%) der Titelverbindung als farblosen Schaum.

¹H-NMR (CDCl₃): δ= 0,63 (3H), 1,22-1,33 (2H), 1,40-1,58 (2H), 1,73-1,88 (3H), 2,08 (1 H), 2,24-2,66 (9H), 2,75 (1 H), 4,46 (1 H), 5,81 (1 H), 7,09 (1 H), 7,16-7,24 (3H), 7,46 (2H), 7,58 (2H), 8,09 (2H) ppm.

### Beispiel 30

### 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-vinyl}-benzoesäuremethylester

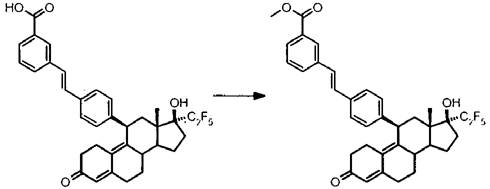

Die Lösung von 24 mg (39 µmol) der nach Beispiel 28 dargestellten Verbindung in 1 ml Tetrahydrofuran versetzte man bei 3°C mit 0,8 ml einer etherischen Lösung von Diazomethan und rührte 30 Minuten. Den nach Lösungsmittelabzug erhaltenen Rückstand reinigte man durch Chromatographie und isolierte 18 mg (75%) der Titelverbindung als farblosen Schaum.

¹H-NMR (CDCl₃): δ= 0,64 (3H), 1,41-1,56 (2H), 1,75-1,87 (3H), 2,07 (1H), 2,26-2,69 (10H), 2,75 (1H), 3,94 (3H), 4,46 (1H), 5,79 (1H), 7,11 (2H), 7,18 (2H), 7,39-7,46 (3H), 7,67 (1H), 7,91 (1 H), 8,18 (1 H) ppm.

### Beispiel 31

### 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-1-yl)-phenyl]-vinyl}-benzoesäuremethylester

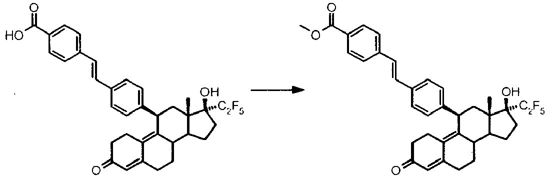

In Analogie zu Beispiel 30 setzte man 13 mg (21 µmol) der nach Beispiel 29 dargestellten Verbindung um und isolierte nach Aufarbeitung und Reinigung 12 mg (90%) der Titelverbindung als farblosen Schaum.

¹H-NMR (CDCl₃): δ= 0,63 (3H), 1,44-1,56 (2H), 1,74-1,87 (3H), 2,07 (1H), 2,17 (1H), 2,25-2,66 (9H), 2,75 (1H), 3,92 (3H), 4,46 (1H), 5,80 (1H), 7,09 (1H), 7,17 (1H), 7,19 (2H), 7,45 (2H), 7,55 (2H), 8,01 (2H) ppm.

### Beispiel 32

### (8S,11R,13S,14S,17S)-17-Hydroxy-11-{4-[(E)-2-(4-methansulfonyl-phenyl)-vinyl]-phenyl}-13-methyl-17-pentafluorethyl-1,2,6,7,8,11,12,13,14,15,16,17-dodecahydro-cyclopenta[a]phenanthren-3-on

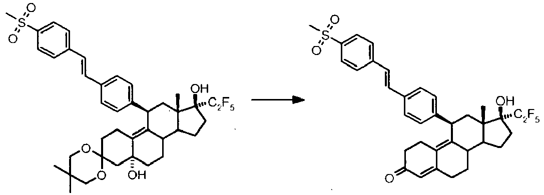

In Analogie zu Beispiel 1 setzte man 25 mg (33 µmol) der nach Beispiel 32a dargestellten Verbindung um und isolierte nach Aufarbeitung und Reinigung 11 mg (52%) der Titelverbindung als farblosen Schaum.

¹H-NMR (CDCl₃): δ= 0,63 (3H), 1,42-1,56 (2H), 1,75-1,87 (3H), 2,08 (1H), 2,21-2,68 (10H), 2,75 (1 H), 3,07 (3H), 4,47 (1 H), 5,80 (1 H), 7,09 (1 H), 7,16-7,24 (3H), 7,45 (2H), 7,66 (2H), 7,90 (2H) ppm.

### Beispiel 32a

### (5R,8S,11R,13S,14S,17S)-11-{4-[(E)-2-(4-Methansulfonyl-phenyl)-vinyl]-phenyl}-5',5',13-trimethyl-17-pentafluorethyl-1,2,3,4,6,7,8,11,12,13,14,15,16,17-tetradecahydrospiro[cyclopenta[a]phenanthren-3,2'-[1,3]dioxan]-5,17-diol

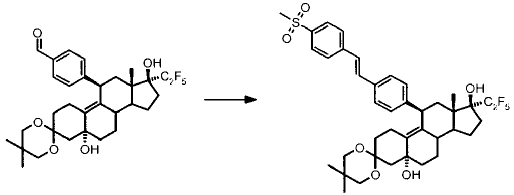

In Analogie zu Beispiel 6a setzte man 200 mg (0,33 mmol) der nach Beispiel 28b dargestellten Verbindung unter Verwendung von (4-Methansulfonyl-benzyl)-phosphonsäurediethylester um und isolierte nach Aufarbeitung und Reinigung 198 mg (79%) der Titelverbindung als farblosen Schaum.

### Beispiel 33

### (8S,11R,13S,14S,17S)-17-Hydroxy-13-methyl-11-{4-[(E)-2-(4-methylsulfanylphenyl)-vinyl]-phenyl}-17-pentafluorethyl-1,2,6,7,8,11,12,13,14,15,16,17-dodecahydro-cyclopenta[a]phenanthren-3-on

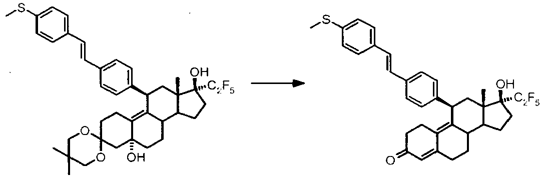

In Analogie zu Beispiel 1 setzte man 25 mg (35 µmol) der nach Beispiel 33a dargestellten Verbindung um und isolierte nach Aufarbeitung und Reinigung 10 mg (48%) der Titelverbindung als farblosen Schaum.

¹H-NMR (CDCl₃): δ= 0,63 (3H), 1,41-1,56 (2H), 1,74-1,87 (3H), 2,07 (1H), 2,10 (1H), 2,25-2,65 (9H), 2,50 (3H), 2,75 (1H), 4,45 (1H), 5,79 (1H), 7,02 (2H), 7,16 (2H), 7,23 (2H), 7,42 (4H) ppm.

### Beispiel 33a

### (5R,8S,11R,13S,14S,17S)-11-{4-[(E)-2-(4-Methylsulfanyl-phenyl)-vinyl]-phenyl}-5',5',13-trimethyl-17-pentafluorethyl-1,2,3,4,6,7,8,11,12,13,14,15,16,17-tetradecahydrospiro[cyclopenta[a]phenanthren-3,2'-[1,3]dioxan]-5,17-diol

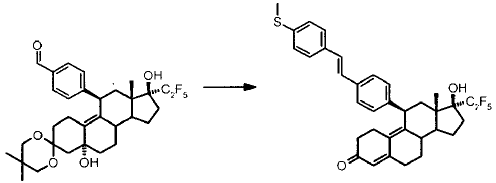

In Analogie zu Beispiel 6a setzte man 200 mg (0,33 mmol) der nach Beispiel 28b dargestellten Verbindung unter Verwendung von (4-Methylsulfanyl-benzyl)-phosphonsäurediethylester um und isolierte nach Aufarbeitung und Reinigung 175 mg (73%) der Titelverbindung als farblosen Schaum.

### Beispiel 34

### [1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-meth-(E)-ylidenaminooxy]-essigsäure

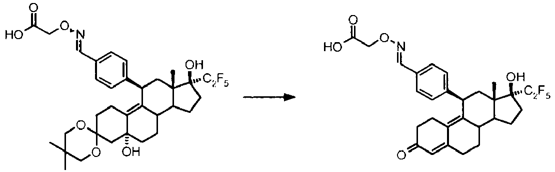

In Analogie zu Beispiel 1 setzte man 59,6 mg (89 µmol) der nach Beispiel 34a dargestellten Verbindung um und isolierte nach Aufarbeitung und Reinigung 39 mg (77%) der Titelverbindung als farblosen Schaum.

¹H-NMR (CD₃OD): δ= 0,59 (3H), 1,35-1,59 (2H), 1,69-1,85 (3H), 2,10 (1H), 2,16-2,48 (5H), 2,55-2,74 (4H), 2,81 (1H), 4,55 (1H), 4,65 (2H), 5,75 (1H), 7,29 (2H), 7,54 (2H), 8,16 (1H) ppm.

### Beispiel 34a

### [1-[4-((5R,8S,11R,13S,14S,17S)-5,17-Dihydroxy-5',5',13-trimethyl-17-pentafluoroethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-meth-(E/Z)-ylidenaminooxy]-essigsäure

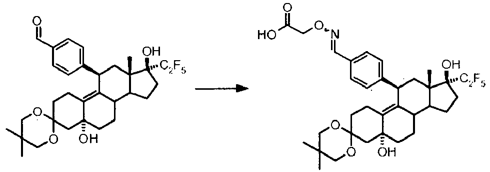

Die Suspension von 31,7 mg Aminooxyessigsäure Hydrochlorid in 2,5 ml Tetrahydrofuran versetzte man bei 23°C mit 230 µl einer 2,5 molaren Lösung von n-Butyllithium in Hexan und nach 20 Minuten mit der Lösung von 100 mg (0,17 mmol) der nach Beispiel 28b dargestellten Verbindung in 1 ml Tetrahydrofuran. Man erwärmte 2,5 Stunden auf 55°C und ließ weitere 16 Stunden bei 23°C reagieren. Man goss in Wasser, säuerte durch Zugabe einer 1 molaren Salzsäure an und extrahierte mehrfach mit Ethylacetat. Die vereinigten organischen Extrakte wusch man mit Wasser, gesättigter Natriumchloridlösung, gesättigter Natriumhydrogencarbonatlösung und trocknete über Natriumsulfat. Den nach Filtration und Lösungsmittelabzug erhaltenen Rückstand reinigte man durch Chromatographie. Isoliert wurden 60 mg (53%) der Titelverbindung als farblosen Schaum.

### Beispiel 35

### 4-((8S,111R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzaldehyd O-benzyl-oxim

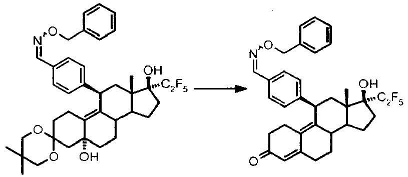

In Analogie zu Beispiel 1 setzte man 86,9 mg (0,12 mmol) der nach Beispiel 35a dargestellten Verbindung um und isolierte nach Aufarbeitung und Reinigung 51 mg (69%) der Titelverbindung als farblosen Schaum.

¹H-NMR (CDCl₃): δ= 0,59 (3H), 1,42-1,54 (2H), 1,74-1,86 (3H), 2,06 (2H), 2,21-2,63 (9H), 2,72 (1H), 4,44 (1H), 5,19 (2H), 5,79 (1H), 8,18 (2H), 7,29-7,44 (5H), 7,50 (2H), 8,10 (1H) ppm.

### Beispiel 35a

### 4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyd O-benzyl-oxim

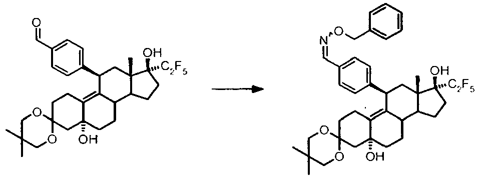

In Analogie zu Beispiel 34a setzte man 100 mg (0,17 mmol) der nach Beispiel 28b dargestellten Verbindung unter Verwendung von *O*-Benzyl-hydroxylamin Hydrochlorid um und isolierte nach Aufarbeitung und Reinigung 87 mg (94%) der Titelverbindung als farblosen Schaum.

### Beispiel 36

### 4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzaldehyd O-ethyl-oxim

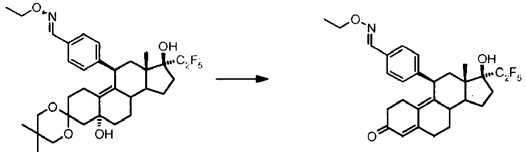

In Analogie zu Beispiel 1 setzte man 40 mg (62 µmol) der nach Beispiel 36a dargestellten Verbindung um und isolierte nach Aufarbeitung und Reinigung 19,7 mg (59%) der Titelverbindung als farblosen Schaum.

¹H-NMR (CDCl₃): δ=0,59 (3H), 1,31 (3H), 1,38-1,58 (2H), 1,71-1,88 (3H), 2,07 (1H), 2,18-2,65 (10H), 2,72 (1H), 4,20 (2H), 4,44 (1H), 5,78 (1H), 7,17 (2H), 7,50 (2H), 8,03 (1H) ppm.

### Beispiel 36a

### 4-((5R,8S,11 R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyd O-ethyl-oxim

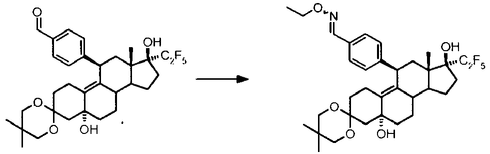

In Analogie zu Beispiel 34a setzte man 100 mg (0,17 mmol) der nach Beispiel 28b dargestellten Verbindung unter Verwendung von *O*-Ethyl-hydroxylamin Hydrochlorid um und isolierte nach Aufarbeitung und Reinigung 40 mg (37%) der Titelverbindung als farblosen Schaum.

### Beispiel 37

### 4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzaldehyd O-(3,4-dichloro-benzyl)-oxim

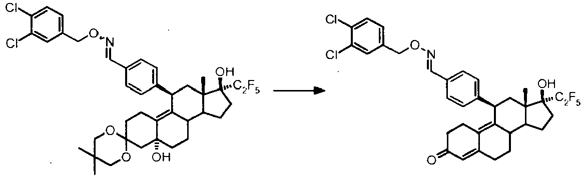

In Analogie zu Beispiel 1 setzte man 105 mg (0,14 mmol) der nach Beispiel 37a dargestellten Verbindung um und isolierte nach Aufarbeitung und Reinigung 50 mg (55%) der Titelverbindung als farblosen Schaum.

¹H-NMR (CDCl₃): δ=0,59 (3H), 1,39-1,55 (2H), 1,74-1,88 (3H), 2,04 (1H), 2,06 (1H), 2,21-2,63 (9H), 2,72 (1H), 4,45 (1H), 5,12 (2H), 5,79 (1H), 7,14-7,25 (3H), 7,43 (1H), 7,45-7,55 (3H), 8,09 (1H) ppm.

### Beispiel 37a

### 4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyd O-(3,4-dichlor-benzyl)-oxim

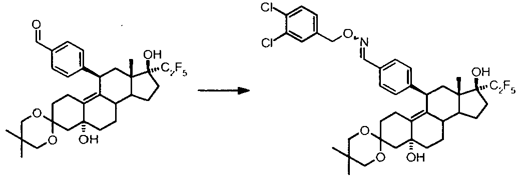

In Analogie zu Beispiel 34a setzte man 100 mg (0,17 mmol) der nach Beispiel 28b dargestellten Verbindung unter Verwendung von *O*-(3,4-Dichlor-benzyl)-hydroxylamin Hydrochlorid um und isolierte nach Aufarbeitung und Reinigung 105 mg (81%) der Titelverbindung als farblosen Schaum.

### Beispiel 38

### 4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzaldehyd O-isobutyl-oxim

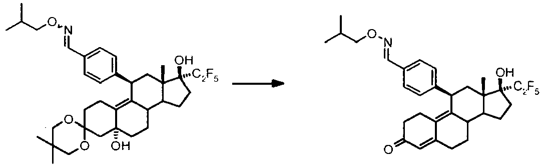

In Analogie zu Beispiel 1 setzte man 58 mg (86 µmol) der nach Beispiel 38a dargestellten Verbindung um und isolierte nach Aufarbeitung und Reinigung 28 mg (58%) der Titelverbindung als farblosen Schaum.

¹H-NMR (CDCl₃): δ= 0,59 (3H), 0,95 (6H), 1,41-1,54 (2H), 1,74-1,86 (3H), 1,99-2,10 (3H), 2,22-2,65 (9H), 2,72 (1H), 3,93 (2H), 4,45 (1H), 5,79 (1H), 7,18 (2H), 7,50 (2H), 8,06 (1H) ppm.

### Beispiel 38a

### 4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-benzaldehyd O-isobutyl-oxim

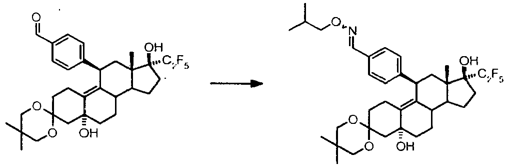

In Analogie zu Beispiel 34a setzte man 100 mg (0,17 mmol) der nach Beispiel 28b dargestellten Verbindung unter Verwendung von *O*-Isobutyl-hydroxylamine Hydrochlorid um und isolierte nach Aufarbeitung und Reinigung 58 mg (52%) der Titelverbindung als farblosen Schaum.

### Beispiel 39

### 1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

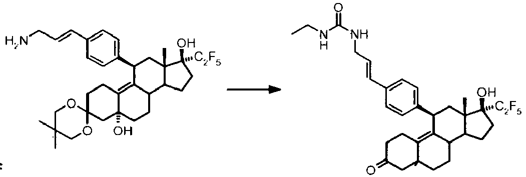

Die Lösung aus 40 mg (53 µmol) der nach Beispiel 39a dargestellten Verbindung in 1 ml Dichlormethan versetzte man mit 5 µl Ethylisocyanat und rührte 1 Stunde bei 23°C. Man engte ein und löste in 1 ml Aceton, versetzte mit 70 µl einer 4 molaren Salzsäure und rührte 1 Stunde. Man versetzte mit gesättigter Natriumhydrogencarbonatlösung, extrahierte mit Dichlormethan und trocknete über Natriumsulfat. Den nach Filtration und Lösungsmittelabzug erhalten Rückstand reinigte man durch Chromatographie. Isoliert wurden 17 mg (54%) der Titelverbindung als farbloser Schaum.

¹H-NMR (CDCl₃): δ= 0,60 (3H), 1,09 (3H), 1,39-1,55 (2H), 1,69-1,89 (3H), 2,05 (1H), 2,19-2,65 (9H), 2,72 (1H), 3,09-3,27 (3H), 3,89 (2H), 4,41 (1H), 4,68 (1H), 4,80 (1H), 5,76 (1H), 6,12 (1H), 6,42 (1H), 7,10 (2H), 7,22 (2H) ppm.

### Beispiel 39a

### (5R,8S,11R,13S,14S,17S)-11-[4-((E)-3-Amino-propenyl)-phenyl]-5',5',13-trimethyl-17-pentafluoroethyl-1,2,3,4,6,7,8,11,12,13,14,15,16,17-tetradecahydro-spiro[cyclopenta[a]phenanthren-3,2'-[1,3]dioxan]-5,17-diol

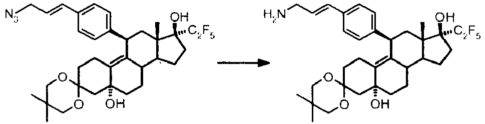

Die Lösung von 778 g (1,19 mmol) der nach Beispiel 39b dargestellten Verbindung in 20 ml Tetrahydrofuran versetzte man mit 3,3 ml Wasser, 0,24 ml Trimethylphosphin und rührte 4 Stunden bei 23°C. Man versetzte mit 2,5 ml einer 25%igen Ammoniaklösung, rührte weitere 16 Stunden bei 23°C und engte ein. Die als Rohprodukt erhaltene Titelverbindung setzte man ohne Reinigung weiter um.

### Beispiel 39b

### (5R,8S,11R,13S,14S,17S)-11-[4-((E)-3-Azido-propenyl)-phenyl]-5',5',13-trimethyl-17-pentafluoroethyl-1,2,3,4,6,7,8,11,12,13,14,15,16,17-tetradecahydro-spiro[cyclopenta[a]phenanthren-3,2'-[1,3]dioxan]-5,17-diol

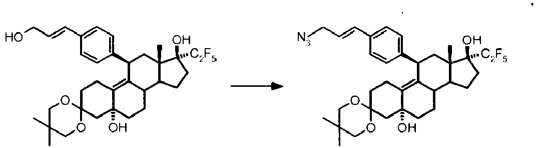

Die Lösung von 800 mg (1,28 mmol) der nach Beispiel 2a dargestellten Verbindung in 20 ml Tetrahydrofuran versetzte man bei 3°C mit 0,38 ml Diphenylphosphorylazid, 0,22 ml 1,8-Diazabicyclo[5.4.0]undec-7-en und ließ 4 Stunden bei 23°C sowie weitere 16 Stunden bei 23°C reagieren. Man versetzte mit Wasser, extrahierte mehrfach mit Ethylacetat, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 785 mg (94%) der Titelverbindung als farbloser Schaum.

### Beispiel 40

### 1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-1-yl)-benzyl]-3-isopropyl-harnstoff

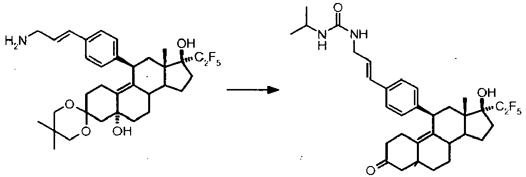

In Analogie zu Beispiel 39 setzte man 40 mg (53 µmol) der nach Beispiel 39a dargestellten Verbindung unter Verwendung von Isopropylisocyanat um und isolierte nach Aufarbeitung und Reinigung 16 mg (50%) der Titelverbindung als farblosen Schaum.

¹H-NMR (CDCl₃): δ= 0,60 (3H), 1,12 (6H), 1,40-1,55 (2H), 1,73-1,88 (3H), 2,05 (1H), 2,21-2,64 (9H), 2,72 (1H), 3,00 (1H), 3,86 (1H), 3,89 (2H), 4,41 (1H), 4,46 (1H), 4,68 (1H), 5,77 (1H), 6,13 (1H), 6,42 (1H), 7,11 (2H), 7,23 (2H) ppm.

### Beispiel 41

### 1-tert-Butyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

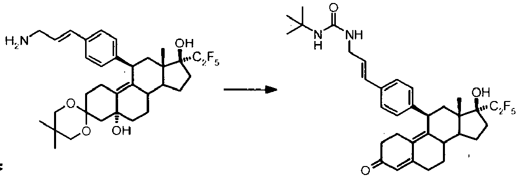

In Analogie zu Beispiel 39 setzte man 40 mg (53 µmol) der nach Beispiel 39a dargestellten Verbindung unter Verwendung von *tert*-Butylisocyanat um und isolierte nach Aufarbeitung und Reinigung 16,3 mg (49%) der Titelverbindung als farblosen Schaum.

¹H-NMR (CDCl₃): δ= 0,60 (3H), 1,32 (9H), 1,40-1,55 (2H), 1,73-1,87 (3H), 2,05 (1H), 2,21-2,63 (9H), 2,65 (1H), 2,73 (1H), 3,88 (2H), 4,41 (1H), 4,42 (1H), 4,47 (1H), 5,77 (1H), 6,15 (1H), 6,44 (1H), 7,10 (2H), 7,24 (2H) ppm.

### Beispiel 42

### 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[alphenanthren-11-yl)-phenyl]-allyl}-3-phenyl-harnstoff

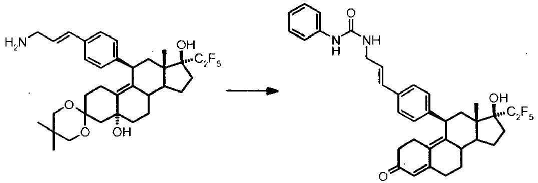

In Analogie zu Beispiel 39 setzte man 40 mg (53 µmol) der nach Beispiel 39a dargestellten Verbindung unter Verwendung von Phenylisocyanat um und isolierte nach Aufarbeitung und Reinigung 10,2 mg (30%) der Titelverbindung als farblosen Schaum.

¹H-NMR (CDCl₃): δ= 0,58 (3H), 1,38-1,53 (2H), 1,72-1,87 (3H), 2,04 (1H), 2,18-2,63 (9H), 2,70 (1H), 2,85 (1H), 3,93 (2H), 4,39 (1H), 5,48 (1H), 5,77 (1H), 6,11 (1H), 6,40 (1H), 6,99-7,11 (3H), 7,12-7,35 (7H) ppm.

### Beispiel 43

### 1-(4-Cyano-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

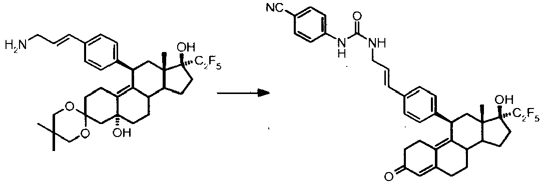

In Analogie zu Beispiel 39 setzte man 40 mg (53 µmol) der nach Beispiel 39a dargestellten Verbindung unter Verwendung von 4-Isocyanato-benzonitril um und isolierte nach Aufarbeitung und Reinigung 19 mg (54%) der Titelverbindung als farblosen Schaum.

¹H-NMR (CDCl₃): δ= 0,63 (3H), 1,40-1,55 (2H), 1,73-1,87 (3H), 2,05 (1H), 2,19-2,65 (9H), 2,72 (1 H), 2,96 (1H), 4,01 (2H), 4,41 (1 H), 5,77 (1 H), 5,90 (1 H), 6,13 (1 H), 6,45 (1H), 7,09 (2H), 7,17 (2H), 7,38 (2H), 7,46 (2H), 8,07 (1H) ppm.

### Beispiel 44

### 1-(4-Fluor-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

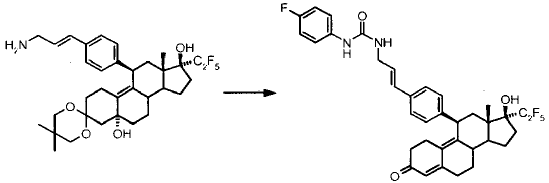

In Analogie zu Beispiel 39 setzte man 40 mg (53 µmol) der nach Beispiel 39a dargestellten Verbindung unter Verwendung von 4-Fluorphenylisocyanat um und isolierte nach Aufarbeitung und Reinigung 21 mg (60%) der Titelverbindung als farblosen Schaum.

¹H-NMR (CDCl₃): δ= 0,58 (3H), 1,38-1,54 (2H), 1,73-1,87 (3H), 2,04 (1H), 2,18-2,62 (9H), 2,70 (1 H), 2,95 (1 H), 3,92 (2H), 4,39 (1H), 5,50 (1 H), 5,76 (1 H), 6,10 (1 H), 6,39 (1 H), 6,88 (2H), 7,06 (2H), 7,15-7,23 (4H), 7,26 (1H) ppm.

### Beispiel 45

### 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-3-p-tolyl-harnstoff

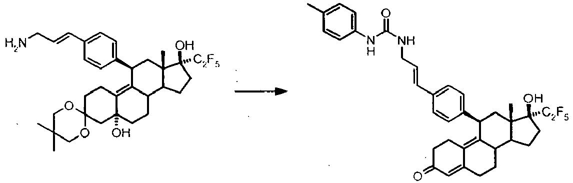

In Analogie zu Beispiel 39 setzte man 40 mg (53 µmol) der nach Beispiel 39a dargestellten Verbindung unter Verwendung von 4-Tolylisocyanat um und isolierte nach Aufarbeitung und Reinigung 20,2 mg (58%) der Titelverbindung als farblosen Schaum.

¹H-NMR (CDCl₃): δ=0,58 (3H), 1,39-1,54 (2H), 1,73-1,87 (3H), 2,05 (1H), 2,18-2,63 (9H), 2,27 (3H), 2,71 (1H), 2,90 (1H), 3,93 (2H), 4,40 (1H), 5,32 (1H), 5,77 (1H), 6,11 (1H), 6,40 (1H), 6,89 (1H), 7,06 (2H), 7,08 (2H), 7,15 (2H), 7,20 (2H) ppm.

### Beispiel 46

### 1-Benzyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

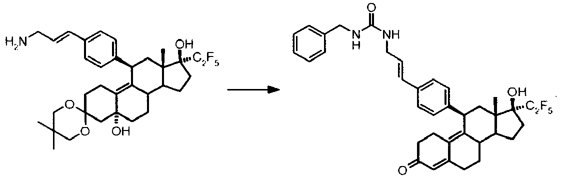

In Analogie zu Beispiel 39 setzte man 40 mg (53 µmol) der nach Beispiel 39a dargestellten Verbindung unter Verwendung von Benzylisocyanat um und isolierte nach Aufarbeitung und Reinigung 18,2 mg (52%) der Titelverbindung als farblosen Schaum.

¹H-NMR (CDCl₃): δ= 0,60 (3H), 1,38-1,54 (2H), 1,73-1,88 (3H), 2,05 (1H), 2,18-2,64 (9H), 2,70 (1H), 2,97 (1H), 3,87 (2H), 4,31 (2H), 4,40 (1H), 4,90 (1H), 5,11 (1H), 5,75 (1H), 6,10 (1H), 6,38 (1H), 7,09 (2H), 7,17-7,32 (7H) ppm.

### Beispiel 47

### 1-tert-Butyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

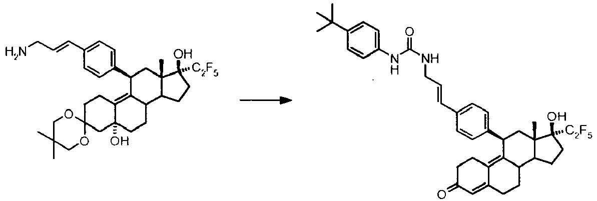

In Analogie zu Beispiel 39 setzte man 40 mg (53 µmol) der nach Beispiel 39a dargestellten Verbindung unter Verwendung von 4-*tert*-Butylphenylisocyanat um und isolierte nach Aufarbeitung und Reinigung 18,6 mg (50%) der Titelverbindung als farblosen Schaum.

¹H-NMR (CDCl₃): δ= 0,58 (3H), 1,27 (9H), 1,39-1,55 (2H), 1,72-1,89 (3H), 2,05 (1H), 2,19-2,65 (9H), 2,71 (1H), 2,80 (1H), 3,95 (2H), 4,40 (1H), 5,32 (1H), 5,77 (1H), 6,13 (1H), 6,41 (1H), 6,85 (1H), 7,08 (2H), 7,17-7,33 (6H) ppm.

### Beispiel 48

### 1-(3,5-Dimethyl-isoxazol-4-yl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

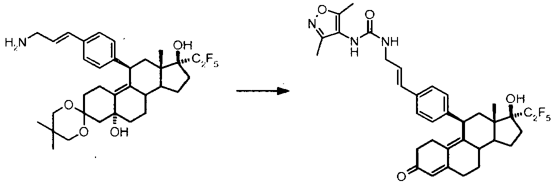

In Analogie zu Beispiel 39 setzte man 40 mg (53 µmol) der nach Beispiel 39a dargestellten Verbindung unter Verwendung von 3,5-Dimethyl-isoxazol-4-yl-isocyanat um und isolierte nach Aufarbeitung und Reinigung 9,1 mg (26%) der Titelverbindung als farblosen Schaum.

¹H-NMR (CDCl₃): δ= 0,59 (3H), 1,40-1,53 (2H), 1,74-1,86 (3H), 2,06 (1H), 2,18 (3H), 2,21-2,62 (9H), 2,33 (3H), 2,65 (1H), 2,72 (1H), 3,97 (2H), 4,42 (1H), 4,84 (1H), 5,77 (1 H), 5,80 (1 H), 6,11 (1 H), 6,42 (1 H), 7,11 (2H), 7,23 (2H) ppm.

### Beispiel 49

### (3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-propionsäureethylester

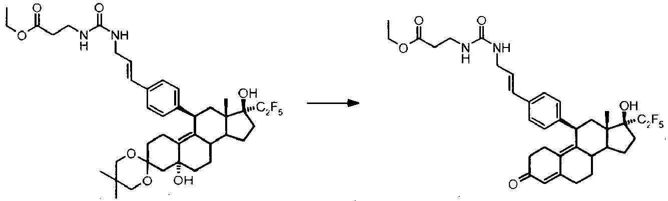

In Analogie zu Beispiel 1 setzte man 40 mg des nach Beispiel 49a dargestellten Rohproduktes um und isolierte nach Aufarbeitung und Reinigung 16 mg (47%) der Titelverbindung als farblosen Schaum.

¹H-NMR (CDCl₃): δ= 0,60 (3H), 1,23 (3H), 1,38-1,56 (2H), 1,72-1,87 (3H), 2,05 (1H), 2,19-2,64 (12H), 2,72 (1H), 3,44 (1H), 3,48 (1H), 3,91 (2H), 4,08 (1H), 4,13 (1H), 4,42 (1 H), 4,64 (1H), 5,06 (1 H), 5,78 (1H), 6,14 (1H), 6,45 (1H), 7,11 (2H), 7,25 (2H) ppm.

### Beispiel 49a

### 3-(3-{(E)-3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-allyl}-ureido)-propionsäureethylester

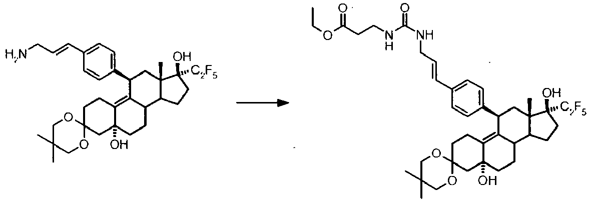

Die Lösung aus 100 mg (0,13 mmol) der nach Beispiel 39a dargestellten Verbindung in 2,5 ml Dichlormethan versetzte man mit 21 µl 3-Isocyanatopropionsäureethylester und rührte 1 Stunde bei 23°C. Man engte ein und isolierte 104 mg der Titelverbindung als Rohprodukt, das man ohne Reinigung weiter umsetzte.

### Beispiel 50

### (3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-propionsäure

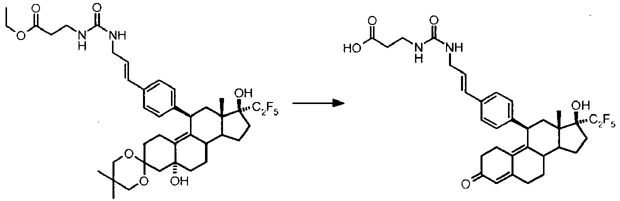

Die Lösung von 64 mg (83 µmol) der nach Beispiel 49a dargestellten Verbindung in 0,8 ml Tetrahydrofuran und 0,8 ml Ethanol versetzte man mit 0,8 ml einer 5%igen wäßrigen Lösung von Lithiumhydroxid und rührte 4 Stunden bei 23°C. Man verdünnte mit Wasser, säuerte durch Zugabe einer 1 molaren Salzsäure an, extrahierte mehrfach mit Dichlormethan, wusch die vereinigten organischen Extrakte mit gesättigter Natriumchloridlösung und trocknete über Natriumsulfat. Den nach Filtration und Lösungsmittelabzug erhaltenen Rückstand löste man in in 1,2 ml Aceton, versetzte mit 90 µl einer 4N Salzsäure und rührte 2 Stunden bei 23°C. Man goss in eine gesättigte Natriumhydrogencarbonatlösung, extrahierte mehrfach mit Dichlormethan, trocknete die vereinigten organischen Extrakte über Natriumsulfat und reinigte den nach Filtration und Lösungsmittelabzug erhaltenen Rückstand durch Chromatographie. Isoliert wurden 16 mg (30%) der Titelverbindung als farbloser Schaum.

¹H-NMR (CD₃OD): δ= 0,60 (3H), 1,35-1,55 (2H), 1,68-1,85 (3H), 2,09 (1H), 2,17-2,48 (7H), 2,54-2,72 (4H), 2,80 (1 H), 3,35 (2H), 3,85 (2H), 4,50 (1 H), 5,74 (1 H), 6,19 (1 H), 6,46 (1H), 7,17 (2H), 7,31 (2H) ppm.

### Beispiel 51

### 3-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-essigsäuresäureethylester

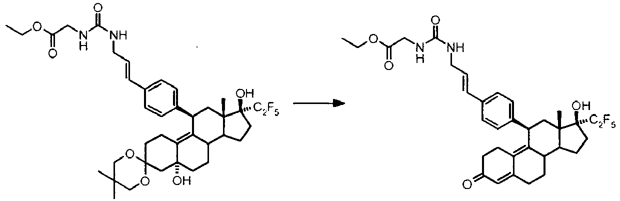

In Analogie zu Beispiel 1 setzte man 40 mg des nach Beispiel 51a dargestellten Rohproduktes um und isolierte nach Aufarbeitung und Reinigung 12,4 mg (36%) der Titelverbindung als farblosen Schaum.

¹H-NMR (CDCl₃): δ=0,59 (3H), 1,25 (3H), 1,38-1,56 (2H), 1,72-1,87 (3H), 2,05 (1H), 2,19-2,64 (10H), 2,72 (1H), 3,93 (2H), 3,99 (2H), 4,18 (2H), 4,41 (1H), 4,90 (1H), 5,13 (1H), 5,77 (1H), 6,14 (1H), 6,46 (1H), 7,10 (2H), 7,25 (2H) ppm.

### Beispiel 51a

### 3-(3-{(E)-3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-allyl}-ureido)-essigsäureethylester

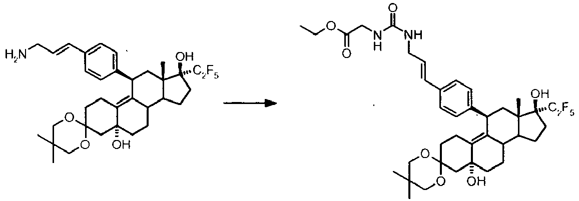

In Analogie zu Beispiel 49a setzte man 100 mg (0,13 mmol) der nach Beispiel 39a dargestellten Verbindung unter Verwendung von Isocyanatoessigsäureethylester um und isolierte nach Aufarbeitung 101 mg der Titelverbindung als Rohprodukt.

### Beispiel 52

### 3-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-essigsäure

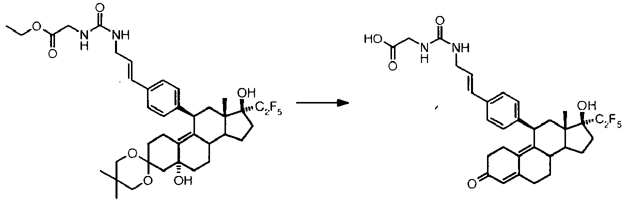

In Analogie zu Beispiel 50 setzte man 60 mg (79 µmol) der nach Beispiel 51a dargestellten Verbindung um und isolierte nach Aufarbeitung und Reinigung 21 mg (42%) der Titelverbindung als farblosen Schaum.

¹H-NMR (CD₃OD):δ= 0,60 (3H), 1,36-1,57 (2H), 1,68-1,84 (3H), 2,09 (1H), 2,17-2,48 (5H), 2,54-2,72 (4H), 2,80 (1H), 3,71 (2H), 3,87 (2H), 4,50 (1 H), 5,74 (1 H), 6,21 (1 H), 6,49 (1 H), 7,17 (2H), 7,31 (2H) ppm.

### Beispiel 53

### 1-(4-Chlor-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

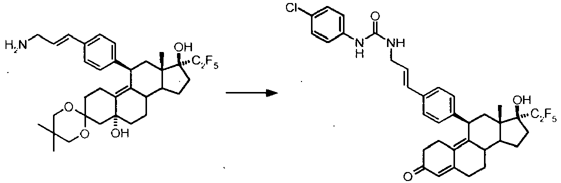

In Analogie zu Beispiel 39 setzte man 40 mg (53 µmol) der nach Beispiel 39a dargestellten Verbindung unter Verwendung von 4-Chlorphenyl-isocyanat um und isolierte nach Aufarbeitung und Reinigung 23,5 mg (66%) der Titelverbindung als farblosen Schaum.

¹H-NMR (CDCl): δ=0,58 (3H), 1,36-1,56 (2H), 1,70-1,90 (3H), 2,04 (1H), 2,15-2,63 (9H), 2,70 (1 H), 3,00 (1 H), 3,91 (2H), 4,39 (1 H), 5,70 (1 H), 5,76 (1 H), 6,07 (1 H), 6,38 (1 H), 7,01-7,25 (8H), 7,53 (1H) ppm.

### Beispiel 54

### 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-3-(4-methoxy-phenyl)-harnstoff

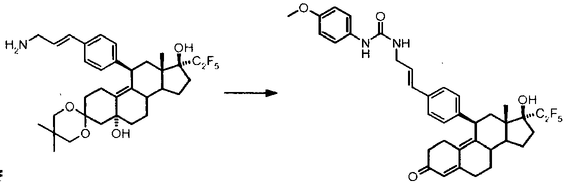

In Analogie zu Beispiel 39 setzte man 40 mg (53 µmol) der nach Beispiel 39a dargestellten Verbindung unter Verwendung von 4-Methoxyphenyl-isocyanat um und isolierte nach Aufarbeitung und Reinigung 22,2 mg (62%) der Titelverbindung als farblosen Schaum.

¹H-NMR (CDCl₃): δ=0,58 (3H), 1,37-1,55 (2H), 1,72-1,88 (3H), 2,04 (1H), 2,17-2,63 (9H), 2,71 (1H), 2,95 (1H), 3,75 (3H), 3,93 (2H), 4,39 (1H), 5,22 (1H), 5,76 (1H), 6,11 (1H), 6,40 (1H), 6,75-6,84 (3H), 7,07 (2H), 7,16 (2H), 7,20 (2H) ppm.

### Beispiel 55

### 4-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-benzoesäureethylester

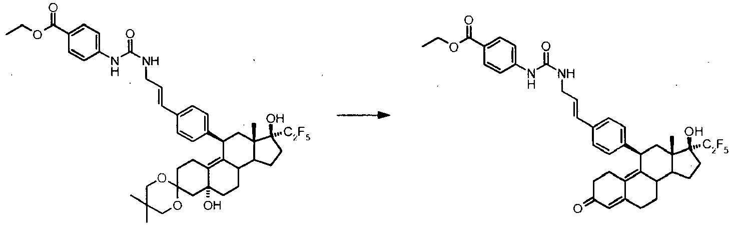

In Analogie zu Beispiel 1 setzte man 41 mg des nach Beispiel 55a dargestellten Rohproduktes um und isolierte nach Aufarbeitung und Reinigung 18,7mg (50%) der Titelverbindung als farblosen Schaum.

¹H-NMR (CDCl₃): δ= 0,61 (3H), 1,35 (3H), 1,39-1,54 (2H), 1,71-1,88 (3H), 2,04 (1H), 2,16-2,64 (9H), 2,70 (1 H), 3,55 (1 H), 3,98 (2H), 4,30 (2H), 4,38 (1 H), 5,77 (1H), 6,11 (1H), 6,15 (1H), 6,43 (1H), 7,04 (2H), 7,11 (2H), 7,39 (2H), 7,82 (2H), 8,09 (1H) ppm.

### Beispiel 55a

### 4-[3-(3-{(E)-3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1H-spiro[cyclopenta[a]phenanthren-3,2'-[1,3]dioxan]-11-yl)-phenyl]-allyl}-ureido)]-benzoesäureethylester

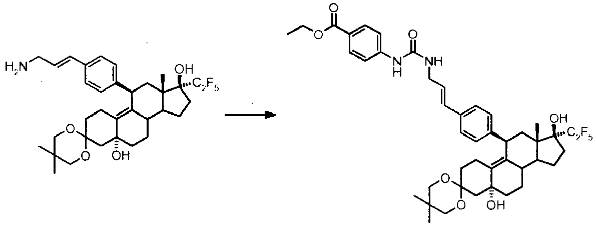

In Analogie zu Beispiel 49a setzte man 120 mg (0,16 mmol) der nach Beispiel 39a dargestellten Verbindung unter Verwendung von 4-Isocyanato-benzoesäureethylester um und isolierte nach Aufarbeitung 121 mg der Titelverbindung als Rohprodukt.

### Beispiel 56

### 4-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-ureido)-benzoesäure

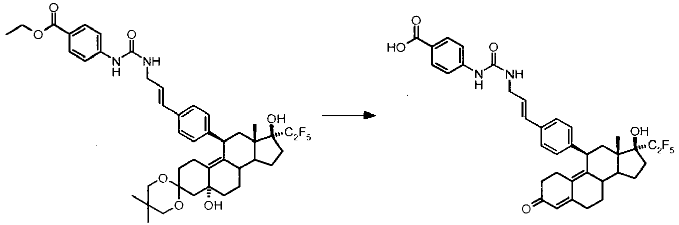

In Analogie zu Beispiel 50 setzte man 80 mg der nach Beispiel 55a dargestellten Verbindung um und isolierte nach Aufarbeitung und Reinigung 35 mg (52%) der Titelverbindung als farblosen Schaum.

¹H-NMR (CD₃OD): δ= 0,58 (3H), 1,35-1,54 (2H), 1,69-1,82 (3H), 2,08 (1H), 2,17-2,46 (5H), 2,55-2,71 (4H), 2,80 (1 H), 3,95 (2H), 4,49 (1 H), 5,73 (1 H), 6,24 (1 H), 6,52 (1 H), 7,17 (2H), 7,32 (2H), 7,42 (2H), 7,87 (2H) ppm.

### Beispiel 57

### 1-Allyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-harnstoff

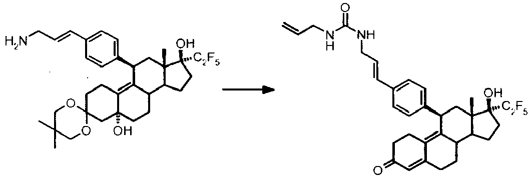

In Analogie zu Beispiel 39 setzte man 20 mg (27 µmol) der nach Beispiel 39a dargestellten Verbindung unter Verwendung von Allylisocyanat um und isolierte nach Aufarbeitung und Reinigung 10 mg (62%) der Titelverbindung als farblosen Schaum.

¹H-NMR (CD₃OD): δ= 0,60 (3H), 1,40-1,55 (2H), 1,74-1,87 (3H), 2,06 (1H), 2,21-2,64 (10H), 2,73 (1 H), 3,80 (2H), 3,94 (2H), 4,42 (1 H), 4,61 (1 H), 4,66 (1 H), 5,11 (1 H), 5,19 (1 H), 5,78 (1 H), 5,84 (1 H), 6,16 (1 H), 6,46 (1 H), 7,11 (2H), 7,25 (2H) ppm.

### Beispiel 58

### 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-allyl}-3-(4-piperidin-1-yl-phenyl)-harnstoff

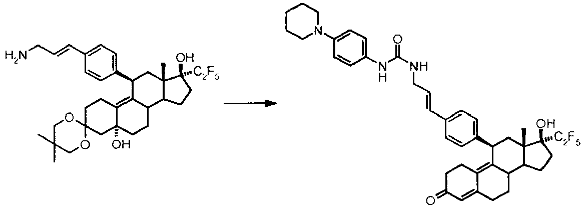

In Analogie zu Beispiel 39 setzte man 40 mg (53 µmol) der nach Beispiel 39a dargestellten Verbindung unter Verwendung von 4-Piperidin-phenylisocyanat um und isolierte nach Aufarbeitung und Reinigung 17 mg (43%) der Titelverbindung als farblosen Schaum.

¹H-NMR (CD₃OD): δ= 0.58 (3H), 1,39-1,61 (4H), 1,63-1,87 (7H), 2,05 (1H), 2,20-2,63 (9H), 2,71 (1H), 2,73 (1H), 3,11 (4H), 3,95 (2H), 4,40 (1H), 4,95 (1H), 5,77 (1H), 6,13 (1H), 6,37 (1H), 6,41 (1 H), 6,87 (2H), 7,09 (2H), 7,12 (2H), 7,23 (2H) ppm.

### Beispiel 59

### Bestimmung der Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MN-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0,01 nmol/l, 0,1 nmol/l, 1 nmol/l, 10 nmol/l, 100 nmol/l und 1 pmol/l) inkubiert, um die agonistische Wirksamkeit zu bestimmen. Als Positivkontrolle der Reportergeninduktion wurden die Zellen mit dem synthetischen Gestagen Promegeston (0,01 nmol/l, 0,1 nmol/l, 1 nmol/l, 10 nmol/l, 100 nmol/l and 1 pmol/l) behandelt. Zur Bestimmung der antagonistischen Aktivität wurden die Zellen mit 0,1 nmol/l Promegeston und zusatzlich mit steigenden Mengen der jeweiligen Testverbindung (0,01 nmol/l, 0,1 nmol/l, 1 nmol/l, 10 nmol/l, 100 nmol/I and 1 pmol/l) behandelt. Die Aktivität des Reportergens LUC (LUC = Luciferase) wurde in den Zellysaten bestimmt und als RLU (relative light units) gemessen. Alle Meßwerte werden angegeben als % Wirksamkeit und als EC₅₀ bzw. IC₅₀ Konzentrationen.

### a) agonistische Aktivitat:

Keine der genannten Verbindungen zeigt eine agonistische Aktivitat.

### b) antagonistische Aktivitat:

Alle genannten Verbindungen zeigen eine 100%ige antagonistische Wirksamkeit.

Die antagonistische Wirkstärke der Verbindungen ist in Tabelle 1 zusammengefasst. Erfindungsgemäß bevorzugt sind Verbindungen deren PR-A oder PR-B Wert ≤ 0,2 nM ist.

**Tabelle 1**

| Bsp. | PR-A IC₅₀ [nM] | PR-B IC₅₀ [nM] | Bsp. | PR-A IC₅₀ [nM] | PR-B IC₅₀ [nM] | Bsp. | PR-A IC₅₀ [nM] | PR-B IC₅₀ [nM] |
|---|---|---|---|---|---|---|---|---|
| 1 | 0,011 | 0,012 | 21 | 0,9 | 1 | 41 | 0,1 | 0,1 |
| 2 | 0,14 | 0,25 | 22 | 0,3 | 1 | 42 | 0,1 | nn |
| 3 | 1,5 | 1,8 | 23 | 0,2 | 0,2 | 43 | nn | nn |
| 4 | 1,0 | 1,1 | 24 | 1 | 1 | 44 | 0,1 | 0,1 |
| 5 | 0,4 | 0,13 | 25 | 1 | 2 | 45 | 0,1 | 0,1 |
| 6 | 0,16 | 0,12 | 26 | 0,9 | 1 | 46 | 0,07 | 0,09 |
| 7 | 0,012 | 0,011 | 27 | 0,2 | 0,1 | 47 | 0,8 | 0,3 |
| 8 | 0,012 | 0,077 | 28 | 0,06 | 0,1 | 48 | 0,09 | 0,03 |
| 9 | 0,055 | 0,09 | 29 | 0,08 | 0,09 | 49 | 0,1 | 0,3 |
| 10 | 0,2 | 0,86 | 30 | 0,09 | 0,08 | 50 | 8 | 3 |
| 11 | 0,011 | 0,012 | 31 | 0,09 | 0,1 | 51 | 0,1 | 0,1 |
| 12 | 0,13 | 0,26 | 32 | 0,01 | 0,01 | 52 | 3 | 2 |
| 13 | 0,1 | 0,3 | 33 | 0,1 | 0,8 | 53 | 0,1 | 0,2 |
| 14 | 0,011 | 0,011 | 34 | 3,9 | 2,8 | 54 | 0,1 | nn |
| 15 | 0,011 | < 0,01 | 35 | 0,1 | 0,09 | 55 | 0,1 | 0,09 |
| 16 | 0,1 | 0,3 | 36 | 0,08 | 0,08 | 56 | 0,9 | 1,9 |
| 17 | 0,1 | 0,7 | 37 | 0,4 | 0,5 | 57 | 0,09 | 0,1 |
| 18 | 0,01 | 0,02 | 38 | 0,09 | 0,1 | 58 | 0,5 | nn |
| 19 | 3 | 3 | 39 | 0,09 | 0,1 | Ref. 1* | 0,7 | 0,8 |
| 20 | 9 | 11 | 40 | 0,1 | 0,1 | | | |

| | | | | | | | | |
|---|---|---|---|---|---|---|---|---|
| nn = nicht gemessen * Ref. 1 Tabelle 1: Als Vergleichsverbindung (Referenzsubstanz) dient die in Beispiel 10 der WO 1998/34947 beschriebene Verbindung (8S,11R,13S,14S,17S)-17-Hydroxy-11-[4-(hydroxymethyl)-phenyl]-13-methyl-17-pentafluorethyl-1,2,6,7, 8,11,12,13,14,15,16,17-dodecahydro-cyclopenta[a]phenanthren-3-on. | | | | | | | | |

### Beispiel 60

### Bestimmung der metabolischen Stabilität in humanen und Ratten-Lebermikrosomen

Es wurden isolierte humane Lebermikrosomen (HLM) zur Beurteilung der metabolischen Stabilität von Verbindungen der allgemeinen Formel I eingesetzt.

Die Inkubationen wurden mit 2.4 ml HLM-lösung (0.5 mg/ml Proteingehalt), 30 µl der Testverbindung (finale Konzentration 1 µM) und 0.6 ml des Cofaktorgemisches (=NADPH-generierendem System aus 3 IU Glucose-6-phosphatdehydrogenase, 14.6 mg Glucose-6-phosphat, 1.2 mg NADP) bei 37°C in 100 mM Phosphatpuffer bei pH 7.4 durchgeführt. Es wurden zu 6 Zeitpunkten (2 - 60 min) Proben entnommen, mit gleichem Volumen Methanol gefällt und der Wiederfund der eingesetzten Testsubstanzen im Überstand mittels LC-MS/MS-Analytik ermittelt. Aus der daraus ermittelten Halbwertzeit des Substanzabbaus wurde die sogenannte intrinsische Clearance der Substanz im Lebermikrosomenansatz berechnet. Mit Hilfe dieser wurde unter Zuhilfenahme verschiedener physiologischer Kenngrößen (humaner Leberblutfluss: 1.3 L*kg/h; spezifisches Lebergewicht (pro kg Körpergewicht): 21g/kg; mikrosomaler Proteingehalt: 40mg/g Leber) nach dem well-stirred Modell eine (metabolische) *in vivo* Clearance in Bezug auf Phase I Reaktionen vorhersagt. Weiterhin wurde unter den Annahmen, dass (i) die Absorption der Prüfsubstanz 100% beträgt, und (ii) der First pass vollständig vom Lebermikrosomenmetabolismus abgebildet wird, eine maximale orale Bioverfügbarkeit (Fmax) errechnet.

Die getesteten Verbindungen weisen eine überraschend hohe metabolische Stabilität (kleine auf Basis der in vitro Daten vorhergesagte "Clearance" -Rate) und eine gute vorhergesagte maximale orale Bioverfügbarkeit Fₘₐₓ auf (Tabelle 2).

Zusätzlich weisen einige Verbindungen eine für diese Wirkstoffklasse ungewöhnlich gute Löslichkeit in wässrigem Medium unter physiologischen Bedingungen auf (Tabelle 2).

**Tabelle 2**

| Bsp. | vorhergesagte Clearance [l/h/kg] | | vorhergesagte Fₘₐₓ [%] (maximale orale Bioverfügbarkeit) | | Löslichkeit bei pH 7,4 [mg/l] |
|---|---|---|---|---|---|
| | Human | Ratte | Human | Ratte | |
| Ref. 1* | 0,7 | 0,8 | 42 | 80 | 17 |
| 1 | 0,90 | 1,9 | 31 | 54 | 6 |
| 2 | 0,41 | 1,3 | 69 | 69 | 11 |
| 5 | 0,00 | 0,5 | 100 | 88 | 203 |
| 6 | 0,21 | 0,7 | 84 | 83 | 9 |
| 7 | 0,38 | 0,9 | 71 | 79 | 6 |
| 8 | 0,18 | 0,3 | 87 | 92 | 7 |
| 9 | 0,43 | 1,0 | 68 | 76 | 8 |
| 10 | 0,36 | 1,0 | 73 | 76 | 8 |
| 13 | 0,63 | 0,1 | 52 | 97 | >179 |
| 14 | 0,9 | 1,1 | 31 | 75 | 9 |
| 16 | 1,02 | 2,0 | 23 | 52 | 10 |
| 17 | 0,89 | 0,4 | 33 | 91 | >188 |
| 18 | 0,84 | 1,8 | 36 | 57 | 6 |
| 21 | 0,7 | 0,5 | 48 | 88 | 7 |
| 22 | 0,8 | 1,0 | 41 | 77 | 6 |
| 23 | 0,4 | 0,2 | 67 | 95 | 8 |
| 27 | 0,98 | 0,9 | 26 | 79 | 10 |
| 28 | 0,35 | 0,1 | 74 | 97 | 120 |
| 29 | 0,08 | 0,0 | 94 | 100 | 102 |
| 36 | 0,36 | 0,8 | 72 | 82 | 5 |

| | | | | | |
|---|---|---|---|---|---|
| * Ref. 1 Tabelle 2: Als Vergleichsverbindung (Referenzsubstanz) dient die in Beispiel 10 der WO 1998/34947 beschriebene Verbindung (8S,11R,13S,14S,17S)-17-Hydroxy-11-[4-(hydroxymethyl)-phenyl]-13-methyl-17-pentafluorethyl-1,2,6,7,8,11,12,13,14,15,16,17-dodecahydro-cyclopenta[a]phenanthren-3-on. | | | | | |

Diese Verbindung weist eine gute vorhergesagte maximale Bioverfügbarkeit in der Ratte, nicht aber im Menschen auf.

Besonders bevorzugt sind daher solche Verbindungen, die eine vorhergesagte maximale orale Bioverfügbarkeit bei unterschiedlichen Spezies (Ratte, Mensch) aufweisen, die jeweils größer 50% ist.

Ganz besonders bevorzugt sind solche Verbindungen, die eine vorhergesagte maximale orale Bioverfügbarkeit bei unterschiedlichen Spezies (Ratte, Mensch) aufweisen, die jeweils größer 70% ist. Beispielsweise genannt seien Verbindungen der Beispiele 5, 6, 7, 8, 10, 28, 29 und 36.

Ebenfalls besonders bevorzugt sind Verbindungen mit einer verbesserten Löslichkeit. Beispielsweise genannt seien Verbindungen der Beispiele 5, 13, 17, 28 und 29.

### Beispiel 61

### Abortivtest an weiblichen Raten

Die Wirkung von Progesteron und des Progesteron Rezeptors sind für eine erfolgreiche Schwangerschaft bzw. Trächtigkeit bei Säugetieren grundlegende Voraussetzung. Die Progesteron-antagonistische Wirkung der erfindungsgemäßen Verbindungen wurde an trächtigen Ratten (6 Ratten pro Gruppe) an Tag 5 bis 7 post coitum unter herkömmlichen Haltungs- und Fütterungsbedingungen getestet.

Nach erfolgreicher Anpaarung, wurden die trächtigen Tiere (Vorhandensein von Spermien im Vaginalabstrich an Tag 1 der Schwangerschaft = d1 p.c.) randomisiert und auf die Behandlungs- und Kontrollgruppe aufgeteilt. Die Tiere erhielten dann subkutan oder oral je 0,15; 0,5; 1,5 oder 5 mg/kg der Testverbindung oder 1,0 ml/kg Vehikel (Benzylbenzoat/Rhizinusöl: 1+4 [v/v]) täglich von Tag 5 bis Tag 7 (d5 - d7 p.c.).

Die Autopsie wurde an Tag 9 (d9 p.c.) durchgeführt. Als Kenngröße der Progesteronrezeptor-antagonistischen Wirkung wurde der Uterus auf das Vorhandensein von Nidationsstellen untersucht. Dabei wurde das völlige Fehlen, aber auch das Vorhandensein pathologischer, hämorrhagischer oder sonst abnormer Nidationsstellen an Tag 9 (d9 p.c.) als Abort gewertet. Die Ergebnisse der Tests sind in Tabelle 3 dargestellt.

**Tabelle 3**

| Testverbindung | Tagesdosis [mg/kg] p.o. | Abortrate [%] |
|---|---|---|
| Vehikel | | 0 |
| Beispiel 17 | 0,5 | 0 |
| | 1,5 | 16,7 |
| | 5,0 | 100,0 |
| Beispiel 29 | 0,5 | 0 |
| | 1,5 | 16,7 |
| | 5,0 | 100,0 |

## Patentansprüche

1. 17-Hydroxy-17-pentafluorethyl-estra-4,9(10)-dien-11-benzyliden-Derivate der allgemeinen Formel (I) 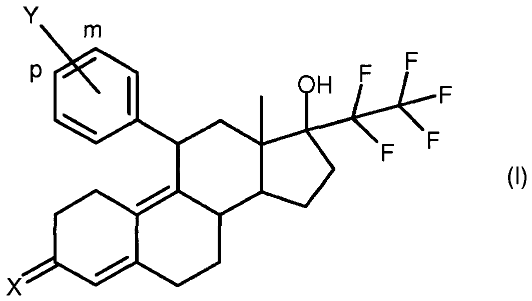 worin
Y für eine -CR¹=CHR² oder eine -CR¹=NR³ Gruppe steht, die jeweils in der meta (m) oder para (p) Position am Phenylring angeknüpft ist,
worin
R¹ Wasserstoff oder ein C₁-C₁₀-Alkylrest,
R² für Wasserstoff, einen C₁-C₁₀-Alkyl, C₂-C₁₀-Alkenyl, Aryl, C₇-C₂₀-Aralkyl, C₁-C₁₀-Acyl, CO₂R⁴, CN, CH=CH-CO₂R⁴, CH₂CH(CO₂R⁴)₂, CH₂CH(CN)₂ oder einen CH₂NHCONHR⁵ Rest steht und
R³ für einen C₁-C₁₀-Alkyl, O-Alkyl, O-(CH₂)ₙ-COOR⁴, O-CH₂-Aryl, Aryl, C₇-C₂₀-Aralkyl oder einen CH₂CO₂R⁴ -Rest steht, worin
R⁴ für Wasserstoff, oder eine C₁-C₁₀-Alkylgruppe und
R⁵ für eine C₁-C₁₀-Alkyl -, C₂-C₁₀-Alkenyl -, Aryl -, C₇-C₂₀-Aralkyl -, (CH₂)ₙCO₂R⁴ - Gruppe mit n = 1 oder 2 und R⁴ in der angegebenen Bedeutung oder für eine 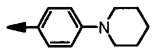 Gruppe steht und
X Sauerstoff, eine NOR⁶ oder eine NNHSO₂R⁶ Gruppe bedeutet,
worin
R⁶ für Wasserstoff, einen C₁-C₁₀-Alkyl, Aryl oder C₇-C₂₀-Aralkyl-Rest steht,
sowie ihre Salze, Solvate oder Solvate der Salze, einschließlich aller Kristallmodifikationen, die α-, β- oder γ-Cyclodextrinclathrate, sowie die mit Liposomen verkapselten Verbindungen
worin
Alkyl steht für Alkylgruppen und können ferner perfluoriert oder durch 1-5 Halogenatome, Hydroxygruppen, C₁-C₄-Alkoxygruppen, C₆-C₁₂-Arylgruppen (die wiederum durch 1-3 Halogenatome substituiert sein können) substituiert sein,
Alkenyl steht für Alkenylgruppen und können durch 1-5 Halogenatome, Hydroxygruppen, C₁-C₃-Alkoxygruppen oder C₆-C₁₂-Arylgruppen (die wiederum durch 1-3 Halogenatome substituiert sein können) substituiert sein,
Aryl steht für substituierte und unsubstituierte carbocyclische Reste, die einfach oder mehrfach mit Halogen, OH, O-Alkyl, S-Alkyl, S(O)Alkyl und SO₂Alkyl, CO₂H, CO₂-Alkyl, NH₂, NO₂, N₃, CN, C₁-C₁₀-Alkyl, C₁-C₁₀-Acyl, C₁-C₁₀-Acyloxy-Gruppen, substituiert sein können,
Aralkyl steht für Aralkylgruppen und können einfach oder mehrfach durch Halogen, OH, O-Alkyl, CO₂H, CO₂-Alkyl, NO₂, N₃, CN, C₁-C₂₀-Alkyl, C₁-C₂₀-Acyl, C₁-C₂₀-Acyloxy-Gruppen substituiert sein.

2. Verbindungen nach Anspruch 1 worin X für Sauerstoff steht.

3. Verbindungen nach Anspruch 1 worin die Gruppe Y in para Position an den Phenylring geknüpft ist.

4. Verbindungen nach einem der Ansprüch 1 - 3 vorliegend als Solvat.

5. Verbindungen nach Anspruch 1 oder 2, worin Y für eine -CR¹ =CHR² Gruppe mit R¹ in der Bedeutung von Wasserstoff und R² in der genannten Bedeutung steht.

6. Verbindungen nach Anspruch 1, nämlich
➢ (8S,11R,13S,14S,17S)-17-Hydroxy-13-methyl-17-pentafluorethyl-11-(4-vinyl-phenyl)-1,2,6,7,8,11,12,13,14,15,16,17-dodecahydrocyclopenta[a]phenanthren-3-on
➢ (8S,11R,13S,14S,17S)-17-Hydroxy-11-[4-((E)-3-hydroxy-propenyl)-phenyl]-13-methyl-17-pentafluorethyl-1,2,6,7,8,11,12,13,14,15,16,17-dodecahydrocyclopenta[*a*]phenanthren-3-on
➢ 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-malonsäure
➢ (8S,11R,13S,14S,17S)-17-Hydroxy-13-methyl-17-pentafluorethyl-11-[4-((E)-2-pyridin-2-yl-vinyl)-phenyl]-1,2,6,7,8,11,12,13,14,15,16,17-dodecahydrocyclopenta[*a*]phenanthren-3-on
➢ (8S,11R,13S,14S,17S)-17-Hydroxy-13-methyl-17-pentafluorethyl-11-[4-((E)-2-pyridin-3-yl-vinyl)-phenyl]-1,2,6,7,8,11,12,13,14,15,16,17-dodecahydrocyclopenta[*a*]phenanthren-3-on
➢ (8S,11R,13S,14S,17S)-17-Hydroxy-13-methyl-17-pentafluorethyl-11-[4-((E)-2-pyridin-4-yl-vinyl)-phenyl]-1,2,6,7,8,11,12,13,14,15,16,17-dodecahydrocyclopenta[*a*]phenanthren-3-on
➢ (8S,11R,138,14S,178)-17-Hydroxy-13-methyl-11-{4-[(E)-2-(2-methyl-thiazol-4-yl)-vinyl]-phenyl}-17-pentafluorethyl-1,2,6,7,8,11,12,13,14,15,16,17-dodecahydro-cyclopenta[a]phenanthren-3-on
➢ (8S,11R,138,148 178)-17-Hydroxy-13-methyl-11-{4-[(E)-2-(2-methyl-benzothiazol-5-yl)-vinyl]-phenyl}-17-pentafluorethyl-1,2,6,7,8,11,12,13,14,15,16,17-dodecahydro-cyclopenta[a]phenanthren-3-on
➢ (8S,11R,13S,14S,17S)-17-Hydroxy-11-(4-isopropenyl-phenyl)-13-methyl-17-pentafluorethyl-1,2,6,7,8,11,12,13,14,15,16,17-dodecahydrocyclopenta[a]phenanthren-3-on
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylsäureethylester
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-acrylsäure
➢ (E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-acrylnitril
➢ (2E,4E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-penta-2,4-diensäureethylester)
➢ (8S,11R,13S,14S,17S)-17-Hydroxy-13-methy)-11-[4-((E)-3-oxo-but-1-enyl)-phenyl]-17-pentafluorethyl-1,2,6,7,8,11,12,13,14,15,16,17-dodecahydrocyclopenta[*a*]phenanthren-3-on
➢ (2E,4E)-5-[4-((8S,11 R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-penta-2,4-diensäure
➢ (8S,11R,13S,14S,17S)-11-[((E/Z)-4-Buta-1,3-dienyl)-phenyl]-17-hydroxy-13-methyl-17-pentafluorethyl-1,2,6,7,8,11,12,13,14,15,16,17-dodecahydrocyclopenta[*a*]phenanthren-3-on
➢ (8S,11R,13S,14S,17S)-17-Hydroxy-13-methyl-17-pentafluorethyl-11-[3-((E)-2-pyridin-4-yl-vinyl)-phenyl]-1,2,6,7,8,11,12,13,14,15,16,17-dodecahydrocyclopenta[*a*]phenanthren-3-on
➢ 2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-malonitril
➢ 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure
➢ 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure
➢ 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäuremethylester
➢ 4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäuremethylester
➢ (8S,11R,13S,14S,17S)-17-Hydroxy-11-{4-[(E)-2-(4-methansulfonyl-phenyl)-vinyl]-phenyl}-13-methyl-17-pentafluorethyl-1,2,6,7,8,11,12,13,14,15,16,17-dodecahydro-cyclopenta[a]phenanthren-3-on
➢ (8S,11R,13S,14S,17S)-17-Hydroxy-13-methyl-11-{4-[(E)-2-(4-methylsulfanylphenyl)-vinyl]-phenyl}-17-pentafluorethyl-1,2,6,7,8,11,12,13,14,15,16,17-dodecahydro-cyclopenta[a]phenanthren-3-on
➢ 4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzaldehyd *O*-benzyl-oxim
➢ 4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzaldehyd *O*-ethyl-oxim
➢ 4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzaldehyd *O*-isobutyl-oxim
➢ 1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff
➢ 1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzyl]-3-isopropyl-harnstoff
➢ 1-*tert*-Butyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-3-phenyl-harnstoff
➢ 1-(4-Fluor-phenyl)-3-{(E)-3-[4-((8S,11R,13S,14S,17S)-17-hydroxy-13-methyl-3-oxo-17-pentafluorethyl-2,3,6,7,8,11,12,13, 14,15,16,17-dodecahydro-1*H-*cyclopenta[a]phenanthren-11-yl)-phenyl]-allyl}-harnstoff
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-3-p-tolyl-harnstoff
➢ 1-Benzyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff
➢ 1-tert-Butyl-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff
➢ 1-(3,5-Dimethyl-isoxazol-4-yl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-allyl}-harnstoff
➢ 3-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-propionsäureethylester
➢ (3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-ureido)-essigsäureethylester
➢ 1-(4-Chlor-phenyl)-3-{(E)-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-harnstoff
➢ 1-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-3-(4-methoxy-phenyl)-harnstoff
> 4-(3-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-ureido)-benzoesäureethylester
➢ 1-Allyl-3-{(E)-3-[4-((8S,11R,13S,14S,17S)-17-hydroxy-13-methyl-3-oxo-17-pentafluorethyl-2,3,6,7,8,11,12,13,4,15,16,17-dodecahydro-1*H-*cyclopenta[a]phenanthren-11-yl)-phenyl]-allyl}-harnstoff

7. Verfahren zur Herstellung der Verbindungen der allgemeinen Formel I, **dadurch**
**gekennzeichnet, dass** ein Epoxid der allgemeinen Formel II, 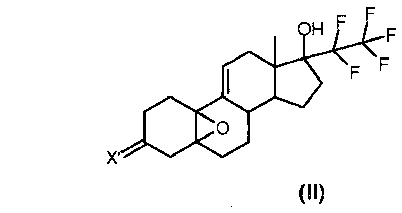 worin
X' ein Sauerstoffatom, zwei Alkoxygruppen OR⁷, eine C₂-C₁₀-Alkylen-α,ω-dioxygruppe, die geradkettig oder verzweigt sein kann und R⁷ eine C₁-C₄-Alkylgruppe bedeutet,
durch metallorganische Kupplungsreaktionen, vorzugsweise durch Kupfer katalysierte Grignardreaktionen, in eine Verbindung der allgemeinen Formel III, 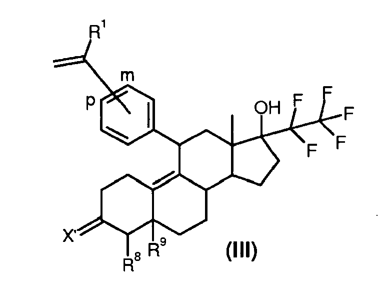 worin
R¹ und X' die genannten Bedeutungen haben und
R⁸ Wasserstoff,
R⁹ eine Hydroxylgruppe, oder
R⁸, R⁹ gemeinsam eine Bindung darstellen,
umsetzt und gegebenenfalls in R¹ vorhandene Funktionalitäten umwandelt und/oder weitere Folgereaktionen zu Verbindungen der allgemeinen Formel I' durchführt 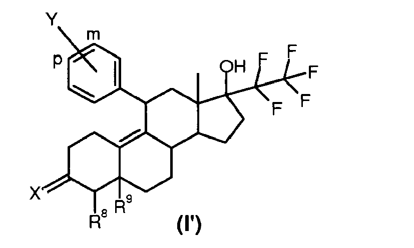 und anschließend aus der Gruppe X' eine Gruppe X mit X in der Bedeutung von Sauerstoff freisetzt und diese Carbonylgruppe (X = Sauerstoff) gegebenenfalls weiter funktionalisiert und zu einer Gruppe mit X in der Bedeutung von NOR⁶ oder NNHSO₂R⁶ umsetzt und/oder für den Fall das R⁸ für Wasserstoff und R⁹ für eine Hydroxylgruppe steht, durch Eliminierung von Wasser eine Doppelbindung erzeugt und R⁸ und R⁹ somit eine gemeinsam Bindung darstellen.

8. Verbindungen einem der Ansprüche 1-6, die eine Progesteronerezeptorantagonistische Wirkung in stabilen Transfektanten humaner Neuroblastoma-Zellen zeigen.

9. Verbindungen nach Anspruch 1, nämlich
(8S,11R,13S,14S,17S)-17-Hydroxy-11-[4-((E)-3-hydroxy-propenyl)-phenyl]-13-methyl-17-pentafluorethyl-1,2,6,7,8,11,12,13,14,15,16,17-dodecahydro-cyclopenta[a]phenanthren-3-on (Beispiel 2),
2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-allyl}-malonsäure (Beispiel 5),
(8S,11R,13S,14S,17S)-17-Hydroxy-13-methyl-17-pentafluorethyl-11-[4-((E)-2-pyridin-2-yl-vinyl)-phenyl]-1,2,6,7,8,11,12,13,14,15,16,17-dodecahydro-cyclopenta[a]phenanthren-3-on (Beispiel 6),
(8S,11R,13S,14S,17S)-17-Hydroxy-13-methyl-17-pentafluorethyl-11-[4-((E)-2-pyridin-3-yl-vinyl)-phenyl]-1,2,6,7,8,11,12,13,14,15,16,17-dodecahydro-cyclopenta[*a*]phenanthren-3-on (Beispiel 7),
(8S,11R,13S,14S,17S)-17-Hydroxy-13-methyl-17-pentafluorethyl-11-[4-((E)-2-pyridin-4-yl-vinyl)-phenyl]-1,2,6,7,8,11,12,13,14,15,16,17-dodecahydro-cyclopenta[a]phenanthren-3-on (Beispiel 8),
(8S,11R,13S,14S,17S)-17-Hydroxy-13-methyl-11-{4-[(E)-2-(2-methyl-thiazol-4-yl)-vinyl]-phenyl}-17-pentafluorethyl-1,2,6,7,8,11,12,13,14,15,16,17-dodecahydro-cyclopenta[a]phenanthren-3-on (Beispiel 9),
(8S,11R,13S,14S,17S)-17-Hydroxy-13-methyl-11-{4-[(E)-2-(2-methyl-benzothiazol-5-yl)-vinyl]-phenyl}-17-pentafluorethyl-1,2,6,7,8,11,12,13,14,15,16,17-dodecahydro-cyclopenta[a]phenanthren-3-on (Beispiel 10),
(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-acrylsäure (Beispiel 13),
(8S,11R,13S,14S,17S)-17-Hydroxy-13-methyl-17-pentafluorethyl-11-[3-((E)-2-pyridin-4-yl-vinyl)-phenyl]-1,2,6,7,8,11,12,13,14,15,16,17-dodecahydro-cyclopenta[*a*]phenanthren-3-on (Beispiel 23), 3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure (Beispiel 28),
4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure (Beispiel 29), oder
4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzaldehyd *O*-ethyl-oxim (Beispiel 36)
die eine vergleichbare maximale orale Bioverfügbarkeit bei unterschiedlichen Spezies (Ratte, Mensch) aufweisen, die jeweils größer 50% ist.

10. Verbindungen nach Anspruch 1, nämlich
2-{(E)-3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-allyl}-malonsäure (Beispiel 5),
(8S,11R,13S,14S,17S)-17-Hydroxy-13-methyl-17-pentafluorethyl-11-[4-((E)-2-pyridin-2-yl-vinyl)-phenyl]-1,2,6,7,8,11,12,13,14,15,16,17-dodecahydro-cyclopenta[*a*]phenanthren-3-on (Beispiel 6),
(8S,11R,13S,14S,17S)-17-Hydroxy-13-methyl-17-pentafluorethyl-11-[4-((E)-2-pyridin-3-yl-vinyl)-phenyl]-1,2,6,7,8,11,12,13,14,15,16,17-dodecahydro-cyclopenta[a]phenanthren-3-on (Beispiel 7),
(8S,11R,13S,14S,17S)-17-Hydroxy-13-methyl-17-pentafluorethyl-11-[4-((E)-2-pyridin-4-yl-vinyl)-phenyl]-1,2,6,7,8,11,12,13,14,15,16,17-dodecahydro-cyclopenta[*a*]phenanthren-3-on (Beispiel 8),
(8S,11R,13S,14S,17S)-17-Hydroxy-13-methyl-11-{4-[(E)-2-(2-methyl-benzothiazol-5-yl)-vinyl]-phenyl}-17-pentafluorethyl-1,2,6,7,8,11,12,13,14,15,16,17-dodecahydro-cyclopenta[a]phenanthren-3-on (Beispiel 10),
3-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure (Beispiel 28),
4-{(E)-2-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-vinyl}-benzoesäure (Beispiel 29), oder
4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzaldehyd *O*-ethyl-oxim (Beispiel 36)
die eine vorhergesagte maximale orale Bioverfügbarkeit bei unterschiedlichen Spezies (Ratte, Mensch) aufweisen, die jeweils größer 70%.

11. Pharmazeutische Präparate enthaltend mindestens eine Verbindung nach einem der Ansprüche 1-6 oder 8-10 oder deren Gemische sowie pharmazeutisch verträgliche Träger.

12. Verwendung der Verbindungen gemäß Anspruch 1-6 oder 8-10 zur Herstellung eines Arzneimittels.

13. Verwendung der Verbindungen gemäß Anspruch 1 zur Herstellung eines Arzneimittels zur Behandlung und/oder Prophylaxe von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

## Claims

1. 17-Hydroxy-17-pentafluoroethylestra-4,9(10)-diene 11-benzylidene derivatives of the general formula (I) 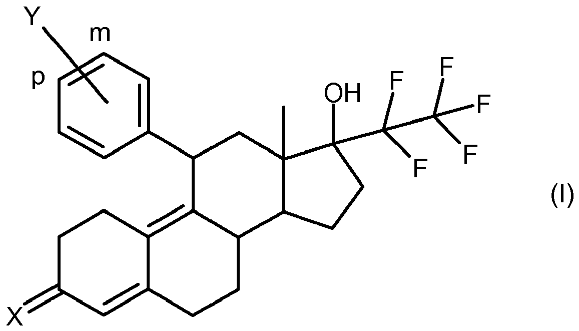 in which
Y is a -CR¹=CHR² or a -CR¹=NR³ group, each of which is joined to the phenyl ring in the meta (m) or para (p) position, in which
R¹ is hydrogen or a C ₁-C₁₀-alkyl radical,
R² is hydrogen, a C₁-C₁₀-alkyl, C₂-C₁₀-alkenyl, aryl, C₇-C₂₀-aralkyl, C₁-C₁₀-acyl, CO₂R⁴, CN, CH=CH-CO₂R⁴, CH₂CH(CO₂R⁴)₂, CH₂CH(CN)₂ or a CH₂NHCONHR⁵ radical and
R³ is a C₁-C₁₀-alkyl, O-alkyl, O-(CH₂)ₙ-COOR⁴, O-CH₂-aryl, aryl, C₇-C₂₀-aralkyl or a CH₂CO₂R⁴ radical in which
R⁴ is hydrogen, or a C₁-C₁₀-alkyl group and
R⁵ is a C₁-C₁₀-alkyl, C₂-C₁₀-alkenyl, aryl, C₇-C₂₀-aralkyl, (CH₂)ₙCO₂R⁴ group where n = 1 or 2 and R⁴ is defined as specified, or is a and 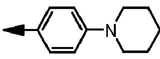 group
X is oxygen, an NOR⁶ or an NNHSO₂R⁶ group, in which
R⁶ is hydrogen, a C₁-C₁₀-alkyl, aryl or C₇-C₂₀-aralkyl radical,
and the salts, solvates or solvates of the salts thereof, including all crystal polymorphs, the α-, β- or γ-cyclodextrin clathrates, and the compounds encapsulated with liposomes, in which
Alkyl is alkyl groups and may also be perfluorinated or substituted by 1-5 halogen atoms, hydroxyl groups, C₁-C₄-alkoxy groups, C₆-C₁₂-aryl groups (which may in turn be substituted by 1-3 halogen atoms),
Alkenyl is alkenyl groups and may be substituted by 1-5 halogen atoms, hydroxyl groups, C₁-C₃-alkoxy groups or C₆-C₁₂-aryl groups (which may in turn be substituted by 1-3 halogen atoms),
Aryl is substituted and unsubstituted carbocyclic radicals which may be mono- or polysubstituted by halogen, OH, O-alkyl, S-alkyl, S(O)alkyl and SO₂alkyl, CO₂H, CO₂-alkyl, NH₂, NO₂, N₃, CN, C₁-C₁₀-alkyl, C₁-C₁₀-acyl, C₁-C₁₀-acyloxy groups,
Aralkyl is aralkyl groups and may be mono- or polysubstituted by halogen, OH, O-alkyl, CO₂H, CO₂-alkyl, NO₂, N₃, CN, C₁-C₂₀-alkyl, C₁-C₂₀-acyl, C₁-C₂₀-acyloxy groups.

2. Compounds according to Claim 1 in which X is oxygen.

3. Compounds according to Claim 1 in which the Y group is bonded to the phenyl ring in the para position.

4. Compounds according to any of Claims 1 - 3 present in solvate form.

5. Compounds according to Claim 1 or 2, in which Y is a -CR¹=CHR² group where R¹ is defined as hydrogen and R² is defined as specified.

6. Compounds according to Claim 1, specifically
➢ (8S,11R,13S,14S,17S)-17-hydroxy-13-methyl-17-pentafluoroethyl-11-(4-vinylphenyl)-1,2,6,7,8,11,12,13,14,15,16, 17-dodecahydrocyclopenta[*a*]phenanthren-3-one
➢ (8S,11R,13S,14S,17S)-17-hydroxy-11-[4-((E)-3-hydroxypropenyl)phenyl]-13-methyl-17-pentafluoroethyl-1,2,6,7,8,11,12, 13,14,15,16,17-dodecahydrocyclopenta[*a*]phenanthren-3-one
➢ 2-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}malonic acid
➢ (8S,11R,13S,14S,17S)-17-hydroxy-13-methyl-17-pentafluoroethyl-11-[4-((E)-2-pyridin-2-ylvinyl)phenyl]-1,2,6,7,8,11,12,13,14,15,16,17-dodecahydrocyclopenta[*a*]phenanthren-3-one
➢ (8S,11R,13S,14S,17S)-17-hydroxy-13-methyl-17-pentafluoroethyl-11-[4-((E)-2-pyridin-3-ylvinyl)phenyl]-1,2,6,7,8,11,12,13,14,15,16,17-dodecahydrocyclopenta[*a*]phenanthren-3-one
➢ (8S,11R,13S,14S,17S)-17-hydroxy-13-methyl-17-pentafluoroethyl-11-[4-((E)-2-pyridin-4-ylvinyl)phenyl]-1,2,6,7,8,11,12,13,14,15,16,17-dodecahydrocyclopenta[*a*]phenanthren-3-one
➢ (8S,11R,13S,14S,17S)-17-hydroxy-13-methyl-11-{4-[(E)-2-(2-methylthiazol-4-yl)vinyl]phenyl}-17-pentafluoroethyl-1,2,6,7,8,11,12,13,14,15,16,17-dodecahydrocyclopenta[a]phenanthren-3-one
➢ (8S,11R,13S,14S,17S)-17-hydroxy-13-methyl-11-{4-[(E)-2-(2-methylbenzothiazol-5-yl)vinyl]phenyl}-17-pentafluoroethyl-1,2,6,7,8,11,12,13,14,15,16,17-dodecahydrocyclopenta[a]phenanthren-3-one
➢ (8S,11R,13S,14S,17S)-17-hydroxy-11-(4-isopropenylphenyl)-13-methyl-17-pentafluoroethyl-1,2,6,7,8,11,12,13,14,15,16,17-dodecahydrocyclopenta[*a*]phenanthren-3-one
➢ (E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]acrylic acid ethyl ester
➢ (E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]acrylic acid
➢ (E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]acrylonitrile
➢ (2E,4E)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]penta-2,4-dienoic acid ethyl ester
➢ (8S,11R,13S,14S,17S)-17-hydroxy-13-methyl-11-[4-((E)-3-oxobut-1-enyl)phenyl]-17-pentafluoroethyl-1,2,6,7,8,11,12,13,14,15, 16,17-dodecahydrocyclopenta[a]phenanthren-3-one
➢ (2E,4E)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]penta-2,4-dienoic acid
➢ (8S,11R,13S,14S,17S)-11-[((E/Z)-4-buta-1,3-dienyl)phenyl]-17-hydroxy-13-methyl-17-pentafluoroethyl-1,2,6,7,8,11,12,13,14,15, 16,17-dodecahydrocyclopenta[a]phenanthren-3-one
➢ (8S,11R,13S,14S,17S)-17-hydroxy-13-methyl-17-pentafluoroethyl-11-[3-((E)-2-pyridin-4-ylvinyl)phenyl]-1,2,6,7,8,11,12,13,14,15,16,17-dodecahydrocyclopenta[*a*]phenanthren-3-one
➢ 2-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}malonitrile
➢ 3-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid
➢ 4-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid
➢ 3-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid methyl ester
➢ 4-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid methyl ester
➢ (8S,11R,13S,14S,17S)-17-hydroxy-11-{4-[(E)-2-(4-methanesulphonylphenyl)vinyl]phenyl}-13-methyl-17-pentafluoroethyl-1,2,6,7,8,11,12,13,14,15,16,17-dodecahydrocyclopenta[a]phenanthren-3-one
➢ (8S,11R,13S,14S,17S)-17-hydroxy-13-methyl-11-{4-[(E)-2-(4-methylsulphanylphenyl)vinyl]phenyl}-17-pentafluoroethyl-1,2,6,7,8,11,12,13,14,15,16,17-dodecahydrocyclopenta[a]phenanthren-3-one
➢ 4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzaldehyde *O*-benzyl oxime
➢ 4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzaldehyde *O*-methyl oxime
➢ 4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzaldehyde *O*-isobutyl oxime
➢ 1-ethyl-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea
➢ 1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]-3-isopropylurea
➢ 1-*tert*-butyl-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea
➢ 1-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}-3-phenylurea
➢ 1-(4-fluorophenyl)-3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopcnta[a]phenanthren-11-yl)phenyl]allyl}urea
➢ 1-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}-3-p-tolylurea
➢ 1-benzyl-3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}urea
➢ 1-tert-butyl-3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}urea
➢ 1-(3,5-dimethylisoxazol-4-yl)-3-{(E)-3-[4-((8S,11R,13S,14S, 17S)-17-hydroxy-13-methyl-3-oxo-17-pentafluoroethyl-2,3,6,7,8,11,12,13,14,15,16,17-dodecahydro-1*H*-cyclopcnta[a]phenanthren-11-yl)phenyl]allyl}urea
➢ 3-(3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}ureido)propionic acid ethyl ester
➢ (3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}ureido)acetic acid ethyl ester
➢ 1-(4-chlorophenyl)-3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopcnta[a]phenanthren-11-yl)phenyl]allyl}urea
➢ 1-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}-3-(4-methoxyphenyl)urea
➢ 4-(3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}ureido)benzoic acid ethyl ester
➢ 1-allyl-3-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a]phenanthren-11-yl)phenyl]allyl}urea

7. Process for preparing the compounds of the general formula I, **characterized in that** an epoxide of the general formula II 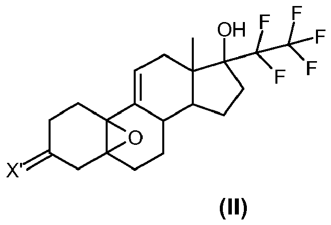 in which
X' is an oxygen atom, two alkoxy groups OR⁷, a C₂-C₁₀-alkylene-α,ω-dioxy group which may be straight-chain or branched and R⁷ is a C₁-C₄-alkyl group,
by organometallic coupling reactions, preferably by copper-catalysed Grignard reactions, is converted to a compound of the general formula III 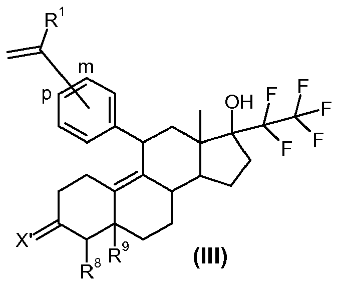 in which
R¹ and X' are each defined as specified and
R⁸ is hydrogen,
R⁹ is a hydroxyl group, or
R⁸, R⁹ together are a bond,
and any functionalities present in R¹ are converted and/or further conversion reactions are conducted to give compounds of the general formula I' 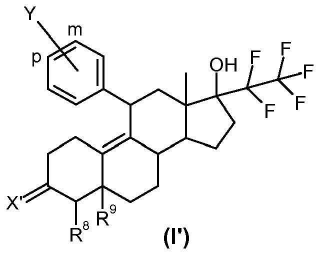 and then an X group where X is defined as oxygen is released from the X' group and this carbonyl group (X = oxygen) is optionally functionalized further and converted to a group where X is defined as NOR⁶ or NNHSO₂R⁶ and/or, in the case that R⁸ is hydrogen and R⁹ is a hydroxyl group, a double bond is obtained by elimination of water, and so R⁸ and R⁹ together are a bond.

8. Compounds according to any of Claims 1-6 which exhibit progesterone receptor-antagonistic action in stable transfectants of human neuroblastoma cells.

9. Compounds according to Claim 1, specifically
(8S,11R,13S,14S,17S)-17-hydroxy-11-[4-((E)-3-hydroxypropenyl)phenyl]-13-methyl-17-pentafluoroethyl-1,2,6,7,8,11,12,13,14,15,16,17-dodecahydrocyclopenta[*a*]phenanthren-3-one (Example 2),
2-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}malonic acid (Example 5),
(8S,11R,13S,14S,17S)-17-hydroxy-13-methyl-17-pentafluoroethyl-11-[4-((E)-2-pyridin-2-ylvinyl)phenyl]-1,2,6,7,8,11,12,13,14,15,16,17-dodecahydrocyclopenta[*a*]phenanthren-3-one (Example 6),
(8S,11R,13S,14S,17S)-17-hydroxy-13-methyl-17-pentafluoroethyl-11-[4-((E)-2-pyridin-3-ylvinyl)phenyl]-1,2,6,7,8,11,12,13,14,15,16,17-dodecahydrocyclopenta[a]phenanthren-3-one (Example 7),
(8S,11R,13S,14S,17S)-17-hydroxy-13-methyl-17-pentafluoroethyl-11-[4-((E)-2-pyridin-4-ylvinyl)phenyl]-1,2,6,7,8,11,12,13,14,15,16,17-dodecahydrocyclopenta[*a*]phenanthren-3-one (Example 8),
(8S,11R,13S,14S,17S)-17-hydroxy-13-methyl-11-{4-[(E)-2-(2-methylthiazol-4-yl)vinyl]phenyl}-17-pentafluoroethyl-1,2,6,7,8,11,12,13,14,15,16,17-dodecahydrocyclopenta[*a*]phenanthren-3-one (Example 9),
(8S,11R,13S,14S,17S)-17-hydroxy-13-methyl-11-{4-[(E)-2-(2-methylbenzothiazol-5-yl)vinyl]phenyl}-17-pentafluoroethyl-1,2,6,7,8,11,12,13,14,15,16,17-dodecahydrocyclopenta[*a*]phenanthren-3-one (Example 10),
(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]acrylic acid (Example 13),
(8S,11R,13S,14S,17S)-17-hydroxy-13-methyl-17-pentafluoroethyl-11-[3-((E)-2-pyridin-4-ylvinyl)phenyl]-1,2,6,7,8,11,12,13,14,15,16,17-dodecahydrocyclopenta[*a*]phenanthren-3-one (Example 23),
3-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid (Example 28),
4-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid (Example 29), or
4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)benzaldehyde *O*-ethyl oxime (Example 36),
which have a comparable maximum oral bioavailability in different species (rats, humans), which is in each case greater than 50%.

10. Compounds according to Claim 1, specifically
2-{(E)-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]allyl}malonic acid (Example 5),
(8S,11R,13S,14S,17S)-17-hydroxy-13-methyl-17-pentafluoroethyl-11-[4-((E)-2-pyridin-2-ylvinyl)phenyl]-1,2,6,7,8,11,12,13,14,15,16,17-dodecahydrocyclopenta[*a*]phenanthren-3-one (Example 6),
(8S,11R,13S,14S,17S)-17-hydroxy-13-methyl-17-pentafluoroethyl-11-[4-((E)-2-pyridin-3-ylvinyl)phenyl]-1,2,6,7,8,11,12,13,14,15,16,17-dodecahydrocyclopenta[*a*]phenanthren-3-one (Example 7),
(8S,11R,13S,14S,17S)-17-hydroxy-13-methyl-17-pentafluoroethyl-11-[4-((E)-2-pyridin-4-ylvinyl)phenyl]-1,2,6,7,8,11,12,13,14,15,16,17-dodecahydrocyclopenta[*a*]phenanthren-3-one (Example 8),
(8S,11R,13S,14S,17S)-17-hydroxy-13-methyl-11-{4-[(E)-2-(2-methylbenzothiazol-5-yl)vinyl]phenyl}-17-pentafluoroethyl-1,2,6,7,8,11,12,13,14,15,16,17-dodecahydrocyclopenta[*a*]phenanthren-3-one (Example 10),
3-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid (Example 28),
4-{(E)-2-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]vinyl}benzoic acid (Example 29), or
4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)benzaldehyde *O*-ethyl oxime (Example 36),
which have a predicted maximum oral bioavailability in different species (rats, humans), which is in each case greater than 70%.

11. Pharmaceutical formulations comprising at least one compound according to any of Claims 1-6 or 8-10 or mixtures thereof, and pharmaceutically suitable carriers.

12. Use of the compounds according to Claim 1-6 or 8-10 for production of a medicament.

13. Use of the compounds according to Claim 1 for production of a medicament for treatment and/or prophylaxis of fibroids of the uterus (myomas, uterine leiomyomas), endometriosis, heavy menstrual bleeds, meningiomas, hormone-dependent breast cancers and complaints associated with the menopause, or for fertility control and emergency contraception.

## Revendications

1. Dérivés de 17-hydroxy-17-pentafluoroéthyl-estra-4,9(10)-diène-11-benzylidène de formule générale (I) 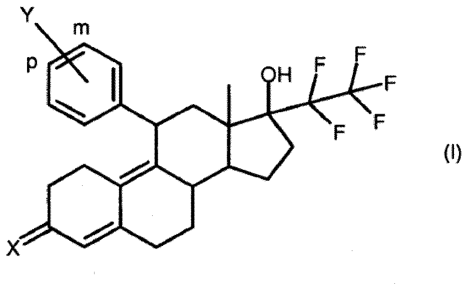 dans laquelle
Y représente un groupe -CR¹=CHR² ou -CR¹=NR³, qui est à chaque fois relié au cycle phényle en position méta (m) ou para (p),
R¹ représentant l'hydrogène ou un radical alkyle en C₁-C₁₀,
R² représentant l' hydrogène, un alkyle en C₁-C₁₀, alcényle en C₂-C₁₀, aryle, aralkyle en C₇-C₂₀, acyle en C₁-C₁₀, CO₂R⁴, CN, CH=CH-CO₂R⁴, CH₂CH(CO₂R⁴)₂, CH₂CH(CN)₂ ou un radical CH₂NHCONHR⁵, et
R³ représentant un alkyle en C₁-C₁₀, O-alkyle, O-(CH₂)ₙ-COOR⁴, O-CH₂-aryle, aryle, aralkyle en C₇-C₂₀ ou un radical CH₂CO₂R⁴,
R⁴ représentant l'hydrogène ou un groupe alkyle en C₁-C₁₀, et
R⁵ représentant un groupe alkyle en C₁-C₁₀, alcényle en C₂-C₁₀, aryle, aralkyle en C₇-C₂₀, (CH₂)ₙCO₂R⁴ avec n = 1 ou 2, et R⁴ ayant la signification indiquée, ou un groupe 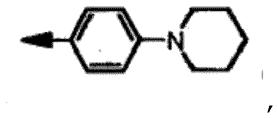 et
X signifie l'oxygène, un groupe NOR⁶ ou NNHSO₂R⁶,
R⁶ représentant l'hydrogène, un radical alkyle en C₁-C₁₀, aryle ou aralkyle en C₇-C₂₀,
ainsi que leurs sels, solvates ou solvates des sels, y compris toutes les formes cristallines, les clathrates d'α-, de β- ou de γ-cyclodextrine, ainsi que les composés encapsulés avec des liposomes,
« alkyle » désignant des groupes alkyle et pouvant être perfluorés ou substitués par 1 à 5 atomes d'halogène, groupes hydroxy, groupes alcoxy en C₁-C₄, groupes aryle en C₆-C₁₂ (qui peuvent eux-mêmes être substitués par 1 à 3 atomes d'halogène),
« alcényle » désignant des groupes alcényle et pouvant être substitués par 1 à 5 atomes d'halogène, groupes hydroxy, groupes alcoxy en C₁-C₃ ou groupes aryle en C₆-C₁₂ (qui peuvent eux-mêmes être substitués par 1 à 3 atomes d'halogène),
« aryle » désignant des radicaux carbocycliques substitués et non substitués, qui peuvent être substitués une ou plusieurs fois avec des groupes halogène, OH, O-alkyle, S-alkyle, S(O)alkyle et SO₂-alkyle, CO₂H, CO₂-alkyle, NH₂, NO₂, N₃, CN, alkyle en C₁-C₁₀, acyle en C₁-C₁₀, acyloxy en C₁-C₁₀,
« aralkyle » désignant des groupes aralkyle et pouvant être substitués une ou plusieurs fois par des groupes halogène, OH, O-alkyle, CO₂H, CO₂-alkyle, NO₂, N₃, CN, alkyle en C₁-C₂₀, acyle en C₁-C₂₀, acyloxy en C₁-C₂₀.

2. Composés selon la revendication 1, dans lesquels X représente l'oxygène.

3. Composés selon la revendication 1, dans lesquels le groupe Y est relié au cycle phényle en position para.

4. Composés selon l'une quelconque des revendications 1 à 3, présents sous la forme de solvates.

5. Composés selon la revendication 1 ou 2, dans lesquels Y représente un groupe -CR¹=CHR² avec R¹ signifiant hydrogène et R² ayant la signification indiquée.

6. Composés selon la revendication 1, à savoir :
- la (8S,11R,13S,14S,17S)-17-hydroxy-13-méthyl-17-pentafluoroéthyl-11-(4-vinyl-phényl)-1,2,6,7,8,11,12,13,14,15,16,17-dodécahydro-cyclopenta[a]phénanthrén-3-one
- la (8S,11R,13S,14S,17S)-17-hydroxy-11-[4-((E)-3-hydroxy-propényl)-phényl]-13-méthyl-17-pentafluoroéthyl-1,2,6,7,8,11,12,13,14,15,16,17-dodécahydro-cyclopenta[a]phénanthrén-3-one
- l'acide 2-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-malonique
- la (8S,11R,13S,14S,17S)-17-hydroxy-13-méthyl-17-pentafluoroéthyl-11-[4-((E)-2-pyridin-2-yl-vinyl)-phényl]-1,2,6,7,8,11,12,13,14,15,16,17-dodécahydro-cyclopenta[a]phénanthrén-3-one
- la (8S,11R,13S,14S,17S)-17-hydroxy-13-méthyl-17-pentafluoroéthyl-11-[4-((E)-2-pyridin-3-yl-vinyl)-phényl]-1,2,6,7,8,11,12,13,14,15,16,17-dodécahydro-cyclopenta[a]phénanthrén-3-one
- la (8S,11R,13S,14S,17S)-17-hydroxy-13-méthyl-17-pentafluoroéthyl-11-[4-((E)-2-pyridin-4-yl-vinyl)-phényl]-1,2,6,7,8,11,12,13,14,15,16,17-dodécahydro-cyclopenta[a]phénanthrén-3-one
- la (8S,11R,13S,14S,17S)-17-hydroxy-13-méthyl-11-{4-[(E)-2-(2-méthyl-thiazol-4-yl)-vinyl]-phényl}-17-pentafluoroéthyl-1,2,6,7,8,11,12,13,14,15,16,17-dodécahydro-cyclopenta[a]phénanthrén-3-one
- la (8S,11R,13S,14S,17S)-17-hydroxy-13-méthyl-11-{4-[(E)-2-(2-méthyl-benzothiazol-5-yl)-vinyl]-phényl}-17-pentafluoroéthyl-1,2,6,7,8,11,12,13,14,15,16,17-dodécahydro-cyclopenta[a]phénanthrén-3-one
- la (8S,11R,13S,14S,17S)-17-hydroxy-11-(4-isopropényl-phényl)-13-méthyl-17-pentafluoroéthyl-1,2,6,7,8,11,12,13,14,15,16,17-dodécahydro-cyclopenta[a]phénanthrén-3-one
- l'ester éthylique de l'acide (E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyctopenta[a]phénanthrén-11-yl)-phényl]-acrylique
- l'acide (E)-3-[4-((8S,11R,135,145,17S)-17-hydroxy-13-méthyl-3-oxo-17-pentafluoroéthyl-2,3,6,7,8,11,12,13,14,15,16,17-dodécahydro-1H-cyctopenta[a]phénanthrén-11-yl)-phényl]-acrylique
- le (E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-acrylonitrile
- l'ester éthylique de l'acide (2E,4E)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-penta-2,4-diénique)
- la (8S,11R,13S,14S,17S)-17-hydroxy-13-méthyl-11-[4-((E)-3-oxo-but-1-ényl)-phényl]-17-pentafluoroéthyl-1,2,6,7,8,11,12,13,14,15,16,17-dodécahydro-cyclopenta[a]phénanthrén-3-one
- l'acide (2E,4E)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-penta-2,4-diénique
- la (8S,11R,13S,14S,17S)-11-[((E/Z)-4-buta-1,3-diényl)-phényl]-17-hydroxy-13-méthyl-17-pentafluoroéthyl-1,2,6,7,8,11,12,13,14,15,16,17-dodécahydro-cyclopenta[a]phénanthrén-3-one
- la (8S,11R,13S,14S,17S)-17-hydroxy-13-méthyl-17-pentafluoroéthyl-11-[3-((E)-2-pyridin-4-yl-vinyl)-phényl]-1,2,6,7,8,11,12,13,14,15,16,17-dodécahydro-cyclopenta[a]phénanthrén-3-one
- le 2-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-malonitrile
- l'acide 3-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque
- l'acide 4-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque
- l'ester méthylique de l'acide 3-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque
- l'ester méthylique de l'acide 4-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque
- la (8S,11R,13S,14S,17S)-17-hydroxy-11-{4-[(E)-2-(4-méthanesulfonyl-phényl)-vinyl]-phényl}-13-méthyl-17-pentafluoroéthyl-1,2,6,7,8,11,12,13,14,15,16,17-dodécahydro-cyclopenta[a]phénanthrén-3-one
- la (8S,11R,13S,14S,17S)-17-hydroxy-13-méthyl-11-{4-[(E)-2-(4-méthylsulfanyl-phényl)-vinyl]-phényl}-17-pentafluoroéthyl-1,2,6,7,8,11,12,13,14,15,16,17-dodécahydro-cyclopenta[a]phénanthrén-3-one
- l'O-benzyl-oxime de 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzaldéhyde
- l'O-éthyl-oxime de 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzaldéhyde
- l'O-isobutyl-oxime de 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzaldéhyde
- la 1-éthyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée
- la 1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-3-isopropyl-urée
- la 1-tert-butyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée
- la 1-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-3-phényl-urée
- la 1-(4-fluoro-phényl)-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée
- la 1-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-3-p-tolyl-urée
- la 1-benzyl-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée
- la 1-tert-butyl-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée
- la 1-(3,5-diméthyl-isoxazol-4-yl)-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée
- l'ester éthylique de l'acide 3-(3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-uréido)-propionique
- l'ester éthylique de l'acide (3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-uréido)-acétique
- la 1-(4-chloro-phényl)-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée
- la 1-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-3-(4-méthoxy-phényl)-urée
- l'ester éthylique de l'acide 4-(3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-uréido)-benzoïque
- la 1-allyl-3-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-urée.

7. Procédé de fabrication des composés de formule générale I, **caractérisé en ce qu'**un époxyde de formule générale II 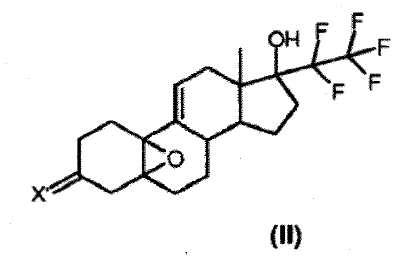 dans laquelle
X' représente un atome d'oxygène, deux groupes alcoxy OR⁷, un groupe alkylène en C₂-C₁₀-α,ω-dioxy, qui peut être linéaire ou ramifié, R⁷ signifiant un groupe alkyle en C₁-C₄,
est transformé par des réactions de couplage métallo-organiques, de préférence par des réactions de Grignard catalysées par du cuivre, en un composé de formule générale III 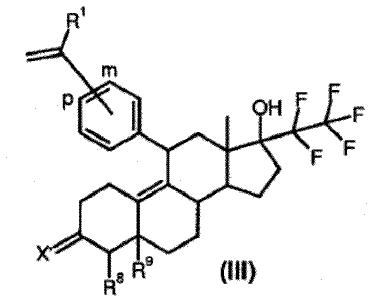 dans laquelle
R¹ et X' ont les significations indiquées et
R⁸ représente l'hydrogène,
R⁹ représente un groupe hydroxyle ou
R⁸, R⁹ représentent ensemble une liaison,
et les fonctionnalités présentes en R¹ sont éventuellement transformées et/ou des réactions ultérieures sont réalisées pour former des composés de formule générale I' 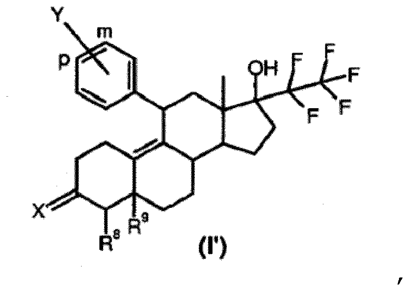 puis un groupe X avec X signifiant l'oxygène est libéré à partir du groupe X' et ce groupe carbonyle (X = oxygène) est éventuellement davantage fonctionnalisé et transformé en un groupe où X signifie NOR⁶ ou NNHSO₂R⁶ et/ou, lorsque R⁸ représente l'hydrogène et R⁹ représente un groupe hydroxyle, une double liaison est formée par élimination d'eau et R⁸ et R⁹ représentent ainsi ensemble une liaison.

8. Composés selon l'une quelconque des revendications 1 à 6, qui présentent un effet antagoniste sur les récepteurs de progestérone dans des transfectants stables de cellules de neuroblastomes humains.

9. Composés selon la revendication 1, à savoir :
la (8S,11R,13S,14S,17S)-17-hydroxy-11-[4-((E)-3-hydroxy-propényl)-phényl]-13-méthyl-17-pentafluoroéthyl-1,2,6,7,8,11,12,13,14,15,16,17-dodécahydro-cyclopenta[a]phénanthrén-3-one (Exemple 2), l'acide 2-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-malonique (Exemple 5),
la (8S,11R,13S,14S,17S)-17-hydroxy-13-méthyl-17-pentafluoroéthyl-11-[4-((E)-2-pyridin-2-yl-vinyl)-phényl]-1,2,6,7,8,11,12,13,14,15,16,17-dodécahydro-cyclopenta[a]phénanthrén-3-one (Exemple 6),
la (8S,11R,13S,14S,17S)-17-hydroxy-13-méthyl-17-pentafluoroéthyl-11-[4-((E)-2-pyridin-3-yl-vinyl)-phényl]-1,2,6,7,8,11,12,13,14,15,16,17-dodécahydro-cyclopenta[a]phénanthrén-3-one (Exemple 7),
la (8S,11R,13S,14S,17S)-17-hydroxy-13-méthyl-17-pentafluoroéthyl-11-[4-((E)-2-pyridin-4-yl-vinyl)-phényl]-1,2,6,7,8,11,12,13,14,15,16,17-dodécahydro-cyclopenta[a]phénanthrén-3-one (Exemple 8),
la (8S,11R,13S,14S,17S)-17-hydroxy-13-méthyl-11-{4-[(E)-2-(2-méthyl-thiazol-4-yl)-vinyl]-phényl}-17-pentafluoroéthyl-1,2,6,7,8,11,12,13,14,15,16,17-dodécahydro-cyclopenta[a]phénanthrén-3-one (Exemple 9),
la (8S,11R,13S,14S,17S)-17-hydroxy-13-méthyl-11-{4-[(E)-2-(2-méthyl-benzothiazol-5-yl)-vinyl]-phényl}-17-pentafluoroéthyl-1,2,6,7,8,11,12,13,14,15,16,17-dodécahydro-cyclopenta[a]phénanthrén-3-one (Exemple 10),
l'acide (E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-acrylique (Exemple 13),
la (8S,11R,13S,14S,17S)-17-hydroxy-13-méthyl-17-pentafluoroéthyl-11-[3-((E)-2-pyridin-4-yl-vinyl)-phényl]-1,2,6,7,8,11,12,13,14,15,16,17-dodécahydro-cyclopenta[a]phénanthrén-3-one (Exemple 23),
l'acide 3-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque (Exemple 28),
l'acide 4-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque (Exemple 29) ou
l'O-éthyl-oxime de 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzaldéhyde (Exemple 36),
qui présentent une biodisponibilité orale maximale comparable chez différentes espèces (rat, homme) qui est à chaque fois supérieure à 50 %.

10. Composés selon la revendication 1, à savoir :
l'acide 2-{(E)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-allyl}-malonique (Exemple 5),
la (8S,11R,13S,14S,17S)-17-hydroxy-13-méthyl-17-pentafluoroéthyl-11-[4-((E)-2-pyridin-2-yl-vinyl)-phényl]-1,2,6,7,8,11,12,13,14,15,16,17-dodécahydro-cyclopenta[a]phénanthrén-3-one (Exemple 6),
la (8S,11R,13S,14S,17S)-17-hydroxy-13-méthyl-17-pentafluoroéthyl-11-[4-((E)-2-pyridin-3-yl-vinyl)-phényl]-1,2,6,7,8,11,12,13,14,15,16,17-dodécahydro-cyclopenta[a]phénanthrén-3-one (Exemple 7),
la (8S,11R,13S,14S,17S)-17-hydroxy-13-méthyl-17-pentafluoroéthyl-11-[4-((E)-2-pyridin-4-yl-vinyl)-phényl]-1,2,6,7,8,11,12,13,14,15,16,17-dodécahydro-cyclopenta[a]phénanthrén-3-one (Exemple 8),
la (8S,11R,13S,14S,17S)-17-hydroxy-13-méthyl-11-{4-[(E)-2-(2-méthyl-benzothiazol-5-yl)-vinyl]-phényl}-17-pentafluoroéthyl-1,2,6,7,8,11,12,13,14,15,16,17-dodécahydro-cyclopenta[a]phénanthrén-3-one (Exemple 10),
l'acide 3-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque (Exemple 28),
l'acide 4-{(E)-2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-vinyl}-benzoïque (Exemple 29) ou
l'O-éthyl-oxime de 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzaldéhyde (Exemple 36),
qui présentent une biodisponibilité orale maximale anticipée chez différentes espèces (rat, homme) qui est à chaque fois supérieure à 70 %.

11. Préparations pharmaceutiques contenant au moins un composé selon l'une quelconque des revendications 1 à 6 ou 8 à 10 ou leurs mélanges, et un véhicule pharmaceutiquement compatible.

12. Utilisation des composés selon les revendications 1 à 6 ou 8 à 10 pour la fabrication d'un médicament.

13. Utilisation des composés selon la revendication 1 pour la fabrication d'un médicament pour le traitement et/ou la prophylaxie de fibromes utérins (myomes, léiomyomes utérins), de l'endométriose, de l'hyperménorrhée, des méningiomes, des cancers du sein hormono-dépendants et des troubles associés à la ménopause ou pour la contraception et la contraception d'urgence.
